(19) Europäisches Patentamt
European Patent Office
Office européen des brevets

(11) EP 0 606 661 B1

(12) EUROPÄISCHE PATENTSCHRIFT

(45) Veröffentlichungstag und Bekanntmachung des Hinweises auf die Patenterteilung:
**12.03.1997 Patentblatt 1997/11**

(51) Int Cl.[6]: **C07C 215/44**, C07C 219/24, C07C 233/18, C07C 217/60, A61K 31/135

(21) Anmeldenummer: **93121161.9**

(22) Anmeldetag: **31.12.1993**

(54) **Octahydrophenanthrenderivate**

Octahydrophenanthrene derivatives and their use as NMDA-Antagonists

Dérivés octahydrophénanthrène et leur usage courure antagonistes de NMDA

(84) Benannte Vertragsstaaten:
**AT BE CH DE DK ES FR GB GR IE IT LI LU MC NL PT SE**

(30) Priorität: **15.01.1993 CH 123/93**

(43) Veröffentlichungstag der Anmeldung:
**20.07.1994 Patentblatt 1994/29**

(73) Patentinhaber: **F. HOFFMANN-LA ROCHE AG
4002 Basel (CH)**

(72) Erfinder:
- **Godel, Thierry
CH-4153 Reinach (CH)**
- **Gutknecht, Eva-Maria
D-79426 Buggingen-Seefelden (DE)**

(74) Vertreter: **Bertschinger, Christoph, Dr. et al
F.Hoffmann-La Roche AG
Patent Department (PLP),124
Grenzacherstrasse
4070 Basel (CH)**

(56) Entgegenhaltungen:
**EP-A- 0 388 977**

- **TETRAHEDRON LETT. (TELEAY); 69; (23); PP.1889-92 DOYLE T W; CONWAY 'N-Chlorosulfonyl isocyanate-olefin adduct: cyclization via oxygen in the ambident anion'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

EP 0 606 661 B1

## Beschreibung

Die vorliegende Erfindung betrifft tricyclische Octahydrophenanthrenderivate, und zwar Verbindungen der allgemeinen Formel

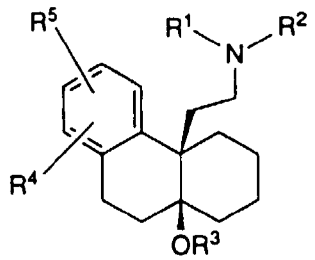

I

worin

| | |
|---|---|
| $R^1$ und $R^2$ | unabhängig voneinander Wasserstoff oder gegebenenfalls durch Aryl oder $C_{3-6}$-Cycloalkyl substituiertes niederes Alkyl; |
| $R^4$ und $R^5$ | entweder beide Wasserstoff oder beide Halogen, oder das eine Wasserstoff und das andere Halogen, Hydroxy, niederes Alkoxy, Aryloxy oder Amino und |
| $R^3$ | Wasserstoff oder - falls keine primäre(n) oder sekundäre(n) Aminogruppe(n) vorhanden ist (sind) - Alkanoyl bedeuten, |

sowie pharmazeutisch verwendbare Salze von Verbindungen der Formel I mit Säuren.

Diese Verbindungen sind neu, mit Ausnahme von rac-cis-4b-(2-Amino-ethyl)-4b,5,6,7,8,8a,9,10-octahydrophenanthren-8a-ol, und es hat sich gezeigt, dass sie wertvolle pharmakodynamische Eigenschaften als nicht-kompetitive NMDA-Antagonisten besitzen, so dass sie als Neuroprotectiva verwendet werden können, insbesondere zur Behandlung oder Verhütung von Ischämie, Hypoglykämie, Hypoxie, cerebralen Gefässpasmen, Spastizität, Trauma, Hemorrhagie, Infektionen (viral, bakteriell, amöbisch, prional), epileptischen Anfällen, autoimmunen Erkrankungen, Entzugssymptomen, Alzheimerscher Krankheit, Parkinsonscher Krankheit, amyotropher Lateralsclerose, Morbus Huntington, Vergiftungen, Olivoponto-cerebellarer Atrophie, Rückenmarkverletzung, Schizophrenie, Depressionen, Angstzuständen, Abhängigkeit, Schmerzen, Autismus und mentaler Retardierung.

Gegenstand der vorliegenden Erfindung sind die eingangs definierten Verbindungen und pharmazeutisch annehmbaren Säureadditionssalze als therapeutische Wirkstoffe, Arzneimittel, enthaltend eine solche Verbindung oder ein Salz davon, die Herstellung solcher Arzneimittel, die Verwendung der eingangs definierten Verbindungen und Salze als Neuroprotectiva, insbesondere bei der Behandlung oder Verhütung von Ischämie, Hypoglykämie, Hypoxie, cerebralen Gefässpasmen, Spastizität, Trauma, Hemorrhagie, Infektionen (viral, bakteriell, amöbisch, prional), epileptischen Anfällen, autoimmunen Erkrankungen, Entzugssymptomen, Alzheimerscher Krankheit, Parkinsonscher Krankheit, amyotropher Lateralsclerose, Morbus Huntington,Vergiftungen, Olivoponto-cerebellarer Atrophie, Rückenmarkverletzung, Schizophrenie, Depressionen, Angstzuständen, Abhängigkeit, Schmerzen, Autismus und mentaler Retardierung, die Verwendung der eingangs definierten Verbindungen und Salze zur Herstellung von Arzneimitteln, weiterhin die neuen unter den eingangs definierten Verbindungen und Salzen als solche sowie Verfahren und Zwischenprodukte für die Herstellung dieser neuen Verbindungen und Salze.

Der Ausdruck "nieder" bezeichnet Verbindungen oder Reste mit höchstens 7, vorzugsweise höchstens 4 Kohlenstoffatomen.

Der Ausdruck "Alkyl" bezeichnet gradkettige oder verzweigte gesättigte Kohlenwasserstoffreste wie Methyl, Ethyl, Propyl und dergleichen.

Der Ausdruck "Alkoxy" bezeichnet über ein Sauerstoffatom gebundene Alkylgruppen im Sinne der vorstehenden Definition, wie Methoxy und dergleichen.

Der Ausdruck "Cycloalkyl" bezeichnet cyclische gesättigte Kohlenwasserstoffreste, wie beispielsweise Cyclopropyl oder Cyclobutyl.

In der EP(a) 388 977 wird eine tricyklische Verbindung offenbart, die an einem der Ringkohlenstoffatome eine direkt verbundene substituierte Aminogruppe aufweist und zur Behandlung cerebrovasculärer Störungen oder als Analgetikum oder Anestetikum dient.

Der Ausdruck "Aryl" bezeichnet über ein Kohlenstoffatom gebundene sechsgliedrige, aromatische, gegebenenfalls substituierte cyclische Reste, wie beispielsweise einen gegebenenfalls substituierten Phenylrest, wobei als Substituenten niedere Alkylgruppen in Betracht kommen können, und schliesst auch Heteroarylreste ein, d.h. beispielsweise

ein bis drei Stickstoffatome enthaltende Heterocyclen, welche unsubstituiert oder durch niederes Alkyl, Nitro, Halogen oder Amino substituiert sein können, wie beispielsweise eine Pyridyl-Gruppe.

Der Ausdruck "Arylmethoxy" bezeichnet durch gegebenenfalls substituiertes Phenyl substituierte Methoxygruppen, wie beispielsweise Benzyloxy.

Der Ausdruck "Alkanoyl" bezeichnet über eine Carbonylgruppe gebundene Alkylgruppen im Sinne der vorstehenden Definition, und umfasst insbesondere niedere Alkanoylgruppen wie Acetyl oder Propionyl und dergleichen.

Der Ausdruck "Heteroaryloxy" bezeichnet über ein Sauerstoffatom gebundene Heteroarylreste.

Der Ausdruck "Arylmethylamino" bezeichnet eine Aminogruppe, welche durch Methyl substituiert ist, wobei die Methylgruppe ihrerseits durch Aryl substituiert ist, wie beispielsweise Benzylamino.

Der Ausdruck "Halogen" bedeutet Fluor, Chlor, Brom und Iod.

Der Ausdruck "pharmazeutisch annehmbare Säureadditionssalze" umfasst Salze mit anorganischen und organischen Säuren, wie Salzsäure, Bromwasserstoffsäure, Salpetersäure, Schwefelsäure, Phosphorsäure, Zitronensäure, Ameisensäure, Fumarsäure, Maleinsäure, Essigsäure, Bernsteinsäure, Weinsäure, Methansulfonsäure, p-Toluolsulfonsäure und dergleichen. Solche Salze können im Hinblick auf den Stand der Technik und unter Berücksichtigung der Natur der in ein Salz zu überführenden Verbindung durch jeden Fachmann ohne weiteres hergestellt werden.

Falls in Formel I keines der Symbole $R^1$ bis $R^5$ ein Asymmetriezentrum aufweist, können die erfindungsgemässen Verbindungen als Enantiomere vorliegen, andernfalls sind verschiedene Diastereomere möglich. Die Erfindung umfasst sämtliche möglichen Stereoisomeren sowie auch Gemische davon, insbesondere Racemate.

In Formel I können zweckmässigerweise $R^1$ und $R^2$ unabhängig voneinander Wasserstoff oder gegebenenfalls durch $C_{3-6}$-Cycloalkyl substituiertes niederes Alkyl, $R^3$ Wasserstoff oder Alkanoyl, $R^4$ und $R^5$ jeweils Halogen oder das eine Wasserstoff und das andere Halogen, Heteroaryloxy, niederes Alkoxy oder Hydroxy bedeuten.

Bevorzugt sind Verbindungen der Formel I worin $R^1$ und $R^2$ niederes Alkyl, $R^3$ Wasserstoff, und $R^4$ und $R^5$ jeweils Wasserstoff, oder das eine Wasserstoff und das andere Halogen oder Hydroxy bedeuten.

Besonders bevorzugte Verbindungen der Formel I sind

rac-cis-4b-(2-Dimethylamino-ethyl)-4b,5,6,7,8,8a,9,10-octahydro-phenanthren-8a-ol;
rac-cis-3-Chloro-4b-(2-dimethylamino-ethyl)-4b,5,6,7,8,8a,9,10-octahydrophenanthren-8a-ol;
(-)-cis-3-Chlor-4b-(2-dimethylamino-ethyl)-4b,5,6,7,8,8a,9,10-octahydrophenanthren-8a-ol;
(+)-cis-3-Chloro-4b-(2-dimethylamino-ethyl)-4b,5,6,7,8,8a,9,10-octahydrophenanthren-8a-ol und
rac-cis-4b-(2-Dimethylamino-ethyl)-4b,5,6,7,8,8a,9,10-octahydro-phenanthren-3,8a-diol.

Weitere bevorzugte Verbindungen der Formel I sind

rac-cis-4b-(2-Amino-ethyl)-3-fluoro-4b,5,6,7,8,8a,9,10-octahydro-phenanthren-8a-ol;
rac-cis-4b-(2-Amino-ethyl)-3-chloro-4b,5,6,7,8,8a,9,10-octahydro-phenanthren-8a-ol;
rac-cis-4b-(2-Amino-ethyl)-3-bromo-4b,5,6,7,8,8a,9,10-octahydro-phenanthren-8a-ol;
rac-cis-4b-(2-Amino-ethyl)-4b,5,6,7,8,8a,9,10-octahydro-phenanthren-3,8a-diol;
(+)-cis-4b-(2-Amino-ethyl)-4b,5,6,7,8,8a,9,10-octahydro-phenanthren-3,8a-diol;
(-)-cis-4b-(2-Amino-ethyl)-4b,5,6,7,8,8a,9,10-octahydro-phenanthren-3,8a-diol;
rac-cis-4b-(2-Dimethylamino-ethyl)-3-fluoro-4b,5,6,7,8,8a,9,10-octahydro-phenanthren-8a-ol;
rac-cis-4b-(2-Dimethylamino-ethyl)-3-methoxy-4b,5,6,7,8,8a,9,10-octahydrophenanthren-8a-ol;
Essigsäure rac-cis-4b-(2-dimethylamino-ethyl)-3-fluoro-4b,5,6,7,8,8a,9,10-octahydro-phenanthren-8a-yl ester;
Essigsäure rac-cis-3-chloro-4b-(2-dimethylamino-ethyl)-4b,5,6,7,8,8a,9,10-octahydro-phenanthren-8a-yl ester;
rac-cis-4b-(2-Amino-ethyl)-3-chloro-1-fluoro-4b,5,6,7,8,8a,9,10-octahydro-phenanthren-8a-ol.

Die neuen Octahydrophenanthrenderivate der eingangs definierten Formel I und deren Salze können erfindungsgemäss dadurch hergestellt werden, dass man

a) zur Herstellung einer Verbindung der Formel I, worin $R^1$, $R^2$ und $R^3$ je Wasserstoff und $R^4$ und $R^5$ beide Halogen oder das eine Wasserstoff und das andere Halogen, niederes Alkoxy oder Aryloxy bedeuten,
eine Verbindung der allgemeinen Formel

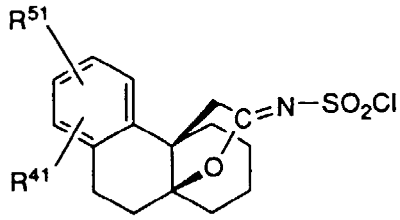

II

worin $R^{41}$ und $R^{51}$ beide Halogen oder das eine Wasserstoff und das andere Halogen, niederes Alkoxy oder Aryloxy bedeuten, reduziert, oder

b) zur Herstellung einer Verbindung der Formel I, worin eines von $R^1$ und $R^2$ Wasserstoff und das andere gegebenenfalls durch Aryl oder $C_{3-6}$-Cycloalkyl substituiertes niederes Alkyl, oder $R^1$ und $R^2$ je gegebenenfalls durch Aryl oder $C_{3-6}$-Cycloalkyl substituiertes niederes Alkyl, $R^3$ Wasserstoff und $R^4$ und $R^5$ beide Wasserstoff oder Halogen, oder das eine Wasserstoff und das andere Halogen, niederes Alkoxy oder Aryloxy bedeuten, eine Verbindung der allgemeinen Formel

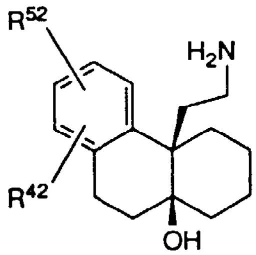

Ia

worin $R^{42}$ und $R^{52}$ beide Wasserstoff oder Halogen oder das eine Wasserstoff und das andere Halogen, niederes Alkoxy oder Aryloxy bedeuten, an der primären Aminogruppe entsprechend mono- oder disubstituiert; oder

c) zur Herstellung einer Verbindung der Formel I, worin $R^1$ und $R^2$ gegebenenfalls durch Aryl oder $C_{3-6}$-Cycloalkyl substituiertes niederes Alkyl, $R^3$ Alkanoyl und $R^4$ und $R^5$ beide Wasserstoff oder Halogen oder das eine Wasserstoff und das andere Halogen, niederes Alkoxy oder Aryloxy bedeuten, eine Verbindung der allgemeinen Formel

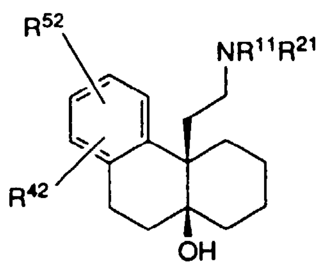

Ib

worin $R^{11}$ und $R^{21}$ gegebenenfalls durch Aryl oder $C_{3-6}$-Cycloalkyl substituiertes niederes Alkyl bedeuten und $R^{42}$ und $R^{52}$ obige Bedeutung besitzen, mit einer eine Alkanoyl-Gruppe liefernden Verbindung behandelt, oder

d) zur Herstellung einer Verbindung der Formel I, worin eines von $R^4$ und $R^5$ Wasserstoff und das andere Hydroxy bedeutet, eine Verbindung der allgemeinen Formel

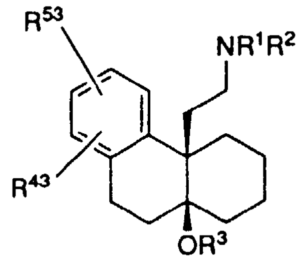

III

worin $R^1$, $R^2$ und $R^3$ obige Bedeutung besitzen, und eines von $R^{43}$ und $R^{53}$ Wasserstoff und das andere Arylmethoxy bedeutet,
in Gegenwart eines Katalysators hydriert, oder

e) zur Herstellung einer Verbindung der Formel I, worin $R^1$ und $R^2$ obige Bedeutung besitzen, $R^3$ Wasserstoff und eines von $R^4$ und $R^5$ Wasserstoff und das andere Amino bedeuten,
eine Verbindung der allgemeinen Formel

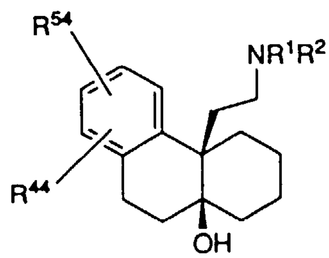

IV

worin $R^1$ und $R^2$ obige Bedeutung besitzen und eines von $R^{44}$ und $R^{54}$ Wasserstoff und das andere Arylmethylamino bedeutet,
in Gegenwart eines Katalysators hydriert, oder

f) zur Herstellung einer Verbindung der Formel I, worin $R^1$, $R^2$ und $R^3$ obige Bedeutung besitzen und eines von $R^4$ und $R^5$ Wasserstoff und das andere Heteroaryloxy bedeutet,
eine Verbindung der allgemeinen Formel

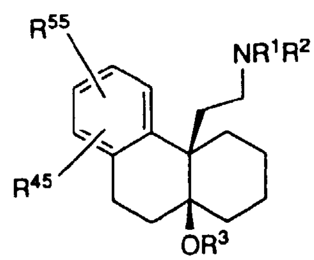

Ic

worin $R^1$, $R^2$ und $R^3$ obige Bedeutung besitzen und eines von $R^{45}$ und $R^{55}$ Wasserstoff und das andere Hydroxy bedeutet,
mit einer Verbindung der Formel X-Z, worin Z eine Abgangsgruppe und X Heteroaryl bedeuten, behandelt,

g) erwünschtenfalls ein erhaltenes Diastereomerengemisch und/oder Racemat auftrennt, und/oder

h) erwünschtenfalls eine erhaltene Verbindung der Formel I in ein pharmazeutisch annehmbares Salz überführt.

Gemäss Verfahrensvariante a) können Verbindungen der Formel I hergestellt werden, worin $R^1$, $R^2$ und $R^3$ Wasserstoff und $R^4$ und $R^5$ beide Halogen oder das eine Wasserstoff und das andere Halogen, niederes Alkoxy oder Aryloxy bedeuten. Dies erfolgt durch Reduktion einer Verbindung der Formel II, worin $R^{41}$ und $R^{51}$ beide Halogen oder

das eine Wasserstoff und das andere Halogen, niederes Alkoxy oder Aryloxy bedeuten, beispielsweise mit Lithiumaluminiumhydrid oder Lithiumborhydrid oder ähnlichen Reduktionsmitteln in Gegenwart inerter Lösungsmittel wie beispielsweise Dioxan oder THF oder ähnlichen inerten Lösungsmitteln; anschliessend erfolgt Umsetzung mit wässriger Alkalihydroxid-Lösung, wie beispielsweise NaOH-Lösung. Die Reduktion wird vorzugsweise in einem Temperaturbereich von Raumtemperatur bis zur Rückflusstemperatur des Reaktionsgemisches durchgeführt, wobei man zweckmässigerweise bei der Rückflusstemperatur arbeitet.

Gemäss Verfahrensvariante b) können Verbindungen der Formel I hergestellt werden, worin eines von $R^1$ und $R^2$ Wasserstoff und das andere gegebenenfalls durch Aryl oder $C_{3-6}$-Cycloalkyl substituiertes niederes Alkyl, oder $R^1$ und $R^2$ je gegebenenfalls durch Aryl oder $C_{3-6}$-Cycloalkyl substituiertes niederes Alkyl, $R^3$ Wasserstoff und $R^4$ und $R^5$ beide Wasserstoff oder Halogen oder das eine Wasserstoff und das andere Halogen, niederes Alkoxy oder Aryloxy bedeuten.

Dies kann dadurch bewerkstelligt werden, dass man eine Verbindung der Formel Ia, worin $R^{42}$ und $R^{52}$ beide Wasserstoff oder Halogen oder das eine Wasserstoff und das andere Halogen, niederes Alkoxy oder Aryloxy bedeuten, an der primären Aminogruppe entsprechend mono- oder disubstituiert. Dies kann z. B. so erfolgen, dass man die Verbindungen der Formel Ia mit einer Verbindung acyliert, welche eine gegebenenfalls durch Aryl oder $C_{3-6}$-Cycloalkyl substituierte Alkanoylgruppe oder eine Formylgruppe oder eine über eine Carbonylgruppe gebundene Arylgruppe oder eine über eine Carbonylgruppe gebundene $C_{3-6}$-Cycloalkylgruppe ergibt, und diese anschliessend reduziert, worauf man die so erhaltene Verbindung gegebenenfalls erneut mit einer Verbindung acyliert, welche eine gegebenenfalls durch Aryl oder $C_{3-6}$-Cycloalkyl substituierte Alkanoylgruppe oder eine Formylgruppe oder eine über eine Carbonylgruppe gebundene Arylgruppe oder eine über eine Carbonylgruppe gebundene $C_{3-6}$-Cycloalkylgruppe ergibt, und diese anschliessend reduziert. Die in der Verbindung der Formel Ia vorhandene Hydroxygruppe wird dabei ebenfalls acyliert, bei der nachfolgenden Reduktion jedoch wieder freigesetzt.

Zur Acylierung eignen sich bevorzugt die reaktiven Derivate der entsprechenden Carbonsäuren, beispielsweise Carbonsäurechloride, welche man vorzugsweise mittels Thionylchlorid in Gegenwart einer geringen Menge an N,N-Dimethylformamid in Toluol herstellt. Die Acylierung erfolgt in Gegenwart einer Base. Als Basen eignen sich beispielsweise Amine wie Triethylamin, Pyridin und dergleichen. Die Reaktion wird vorzugsweise in einem Temperaturbereich von etwa Raumtemperatur bis zur Rückflusstemperatur des Reaktionsgemisches durchgeführt, wobei man zweckmässigerweise bei Raumtemperatur arbeitet.

Die Formylgruppe kann beispielsweise durch Umsetzung einer Verbindung der Formel Ia mit einem Gemisch aus Ameisensäureethylester und Ameisensäure eingeführt werden; das erwähnte Gemisch dient gleichzeitig als Lösungsmittel. Die Reaktion wird vorzugsweise bei der Rückflusstemperatur des Reaktionsgemisches durchgeführt.

Als Reduktionsmittel eignet sich beispielsweise Lithiumaluminiumhydrid. Die Reduktion wird in inerten organischen Lösungsmitteln, beispielsweise THF oder ähnlichen Lösungsmitteln durchgeführt. Die Reaktionstemperatur liegt vorzugsweise in einem Temperaturbereich von Raumtemperatur bis zur Rückflusstemperatur, wobei vorzugsweise bei der Rückflusstemperatur des Reaktionsgemisches gearbeitet wird.

Man kann auch eine Verbindung der Formel Ia in einem Schritt dimethylieren, beispielsweise durch Behandeln mit einer Mischung aus wässriger Ameisensäurelösung und wässriger Formaldehyd-Lösung; diese Mischung dient gleichzeitig als Lösungsmittel. Bevorzugt wird bei der Rückflusstemperatur der Reaktionsmischung gearbeitet.

Gemäss Verfahrensvariate c) können Verbindungen der Formel I hergestellt werden, worin $R^1$ und $R^2$ gegebenenfalls durch Aryl oder $C_{3-6}$-Cycloalkyl substituiertes niederes Alkyl, $R^3$ Alkanoyl und $R^4$ und $R^5$ beide Wasserstoff oder Halogen oder das eine Wasserstoff und das andere Halogen, niederes Alkoxy oder Aryloxy bedeuten. Dies kann dadurch bewerkstelligt werden, dass man beispielsweise ein in an sich bekannter Weise hergestelltes reaktives Derivat einer entsprechenden Carbonsäure mit einer Verbindung der Formel Ib, worin $R^{11}$ und $R^{21}$ gegebenenfalls durch Aryl oder $C_{3-6}$-Cycloalkyl substituiertes niederes Alkyl bedeuten und $R^{42}$ und $R^{52}$ obige Bedeutung besitzen, umsetzt. Als reaktive Carbonsäurederivate verwendet man beispielsweise die entsprechenden Carbonsäurechloride, welche man aus den entsprechenden Carbonsäuren zweckmässigerweise mittels Thionylchlorid in Gegenwart einer geringen Menge an N,N-Dimethylformamid in Toluol herstellt. Es können auch die entsprechenden Imidazolide durch Behandeln der Carbonsäure mit 1,1'-Carbonyl-diimidazol in einem inerten Lösungsmittel, wie beispielsweise N,N-Dimethylformamid hergestellt werden. Die Umsetzung des reaktionsfähigen Carbonsäurederivats mit einer Verbindung der Formel Ib erfolgt in Gegenwart einer Base. Als Basen eignen sich beispielsweise Amine wie Triethylamin, Pyridin und dergleichen. Die Reaktion wird vorzugsweise in einem Temperaturbereich von etwa Raumtemperatur bis zur Rückflusstemperatur des Reaktionsgemisches durchgeführt, wobei man zweckmässigerweise bei Raumtemperatur arbeitet.

Gemäss Verfahrensvariante d) können Verbindungen der Formel I hergestellt werden, worin $R^1$, $R^2$ und $R^3$ obige Bedeutung besitzen und eines von $R^4$ und $R^5$ Wasserstoff und das andere Hydroxy bedeutet.

Bei dieser Reaktion wird eine Verbindung der Formel III, worin $R^1$, $R^2$ und $R^3$ obige Bedeutung besitzen und eines von $R^{43}$ und $R^{53}$ Wasserstoff und das andere Arylmethoxy bedeutet, in Gegenwart eines Katalysators wie beispielsweise eines Palladium-Katalysators hydriert. Als Lösungsmittel eignen sich bevorzugt niedere Alkohole wie beispielsweise Methanol oder Ethanol. Es wird bevorzugt bei Raumtemperatur gearbeitet.

Gemäss Verfahrensvariante e) können Verbindungen der Formel I hergestellt werden, worin $R^1$ und $R^2$ obige

Bedeutung besitzen und $R^3$ Wasserstoff und eines von $R^4$ und $R^5$ Wasserstoff und das andere Amino bedeuten. Hierzu wird eine Verbindung der Formel IV, worin $R^1$ und $R^2$ obige Bedeutung besitzen und eines von $R^{44}$ und $R^{54}$ Wasserstoff und das andere Arylmethylamino bedeutet, in Gegenwart eines Katalysators, beispielsweise eines Palladium-Katalysators, hydriert. Als Lösungsmittel eignen sich bevorzugt niedere Alkohole wie beispielsweise Methanol oder Ethanol. Es wird bevorzugt bei Raumtemperatur und einem Druck von etwa 50 bar gearbeitet.

Gemäss Verfahrensvariante f) können Verbindungen der Formel I hergestellt werden, worin $R^1$, $R^2$ und $R^3$ obige Bedeutung besitzen und eines von $R^4$ und $R^5$ Wasserstoff und das andere Heteroaryloxy bedeutet. Diese Verfahrensvariante wird vorzugsweise durch Behandeln einer Verbindung der Formel Ic, worin $R^1$, $R^2$ und $R^3$ obige Bedeutung besitzen und eines von $R^{45}$ und $R^{55}$ Wasserstoff und das andere Hydroxy bedeutet, mit einem reaktionsfähigen Heteroarylderivat, beispielsweise einem Heteroarylhalogenid wie 2-Brom-pyridin oder dergleichen durchgeführt. Als Lösungsmittel eignen sich beispielsweise Pyridin oder ähnliche unter den Reaktionsbedingungen inerte Lösungsmitteln. Es wird bevorzugt bei der Rückflusstemperatur der Reaktionsmischung gearbeitet.

Die Auftrennung von Diastereomerengemischen und/oder die Aufspaltung von Racematen gemäss Verfahrensvariante g) kann nach allgemein üblichen Methoden erfolgen, wobei es zweckmässig sein kann, dies nicht erst bei Verbindungen der Formel I durchzuführen, sondern bereits auf einer früheren Synthesestufe, z.B. auf der Stufe der Verbindungen der Formel III oder IV.

Gemäss Verfahrensvariante h) können Verbindungen der Formel I in pharmazeutisch annehmbare Säureadditionssalze übergeführt werden. Es kommen dabei sowohl Salze mit anorganischen als auch Salze mit organischen Säuren in Betracht. Beispiele derartiger Salze sind Hydrochloride, Hydrobromide, Nitrate, Sulfate, Phosphate, Citrate, Formiate, Fumarate, Maleate, Acetate, Succinate, Tartrate, Methansulfonate, p-Toluolsulfonate und dergleichen. Diese Salze können nach an sich bekannten und jedem Fachmann geläufigen Methoden hergestellt werden.

Die als Ausgangsstoffe verwendeten Verbindungen der Formel II können beispielsweise gemäss dem nachfolgenden Reaktionsschema und der daran anschliessenden Erläuterung der verschiedenen Reaktionen hergestellt werden.

Reaktionsschema I

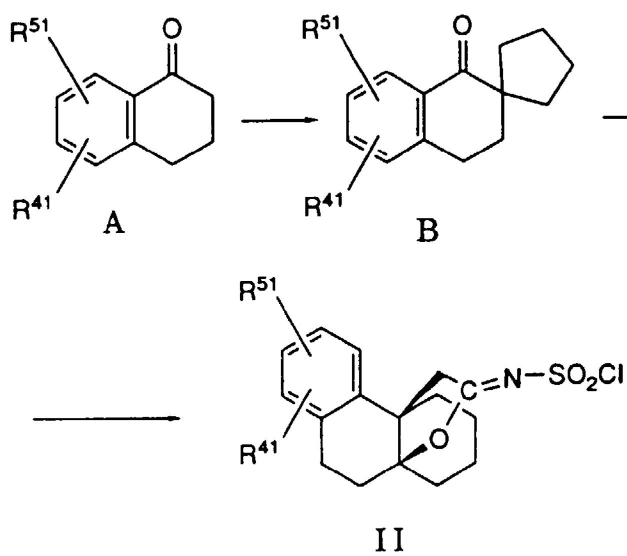

A B C II

$R^{41}$ und $R^{51}$ bedeuten beide jeweils Halogen oder das eine Wasserstoff und das andere Halogen, niederes Alkoxy oder Aryloxy.

Die Tetralone der allgemeinen Formel A sind bekannt oder können analog zu den bekannten Synthesewegen hergestellt werden. Neue Tetralone wurden folgendermassen hergestellt:

1) 8-Chloro-1,2,3,4-tetrahydro-naphthalen-1-on:

a) Orthometallierung von 1,2,3,4-Tetrahydro-naphthalen-1-ol mit Butyllithium und N,N,N',N'-Tetramethylethylendiamin (TMEDA) in Hexan gemäss der in Synthesis 1981, 59 beschriebenen Methode;

b) Quenchen mit Hexachlorethan nach V. Snieckus, Chem. Rev. 1990, 90, 879;

c) Jones-Oxidation.

2) 7-Chloro-5-fluoro-1,2,3,4-tetrahydro-naphthalen-1-on:

a) Bromierung von 4-Chloro-2-fluorotoluol mit N-Bromsuccinimid in Tetrachlorkohlenstoff in Anwesenheit katalytischer Mengen Dibenzoylperoxid;

b) Oxidation zum Aldehyd gemäss der in Tetr. Letters 1990, 31, 4825 beschriebenen Methode;

c) Wittig-Reaktion mit 2-(1,3-Dioxan-2-yl)-ethyl-triphenyl-phosphoniumbromid und Kaliumtertbutylat in DMSO/THF;

d) Hydrierung des Alkens mit Pd-C-Katalysator(10%ig) in THF bei RT unter Normaldruck;

e) Ketalspaltung zum Aldehyd mit Cr(III)chlorid in 4N wässriger Salzsäure gemäss der in Synthesis, 1979, 132 beschriebenen Methode;

f) Oxidation mit Kaliumpermanganat und Natriumdihydrogenphosphat in tert-Butanol gemäss der in Tetr. Letters 1986, 27, 4537 beschriebenen Methode;

g) Cyclisierung mit Polyphosphorsäure bei ca. 100°C.

3) 5,7-Dichloro-1,2,3,4-tetrahydro-naphthalen-1-on:

a) Wittig Reaktion von 2,4-Dichlorobenzaldehyd mit 2-(1,3-Dioxan-2-yl)-ethyl-triphenyl-phosphoniumbromid und Kalium-tert-butylat in DMSO/THF;

b) Hydrierung des Alkens mit Pd-C-Katalysator(10%ig) in THF bei Raumtemperatur unter Normaldruck;

c) Ketalspaltung zum Aldehyd mit Cr(III)chlorid in 4N wässriger Salzsäure gemäss der in Synthesis, 1979, 132 beschriebenen Methode;

d) Oxidation mit Kaliumpermanganat und Natriumdihydrogenphosphat in tert-Butanol gemäss der in Tetr. Letters 1986, 27, 4537 beschriebenen Methode;

f) Cyclisierung mit Polyphosphorsäure bei ca. 100°C.

Die Verbindung entsprechend der allgemeinen Formel II, worin aber $R^{41}$ und $R^{51}$ jeweils Wasserstoff bedeutet, ist bekannt, vgl. Tetr. Letters 1969, 1889. Analog hierzu lassen sich Verbindungen der allgemeinen Formel II herstellen, worin $R^{41}$ und $R^{51}$ die oben angegebene Bedeutung haben:

Durch Umsetzung eines Tetralons der Formel A mit einer Base wie beispielsweise Kalium-tert-butanolat in einem geeigneten organischen Lösungsmittel, vorzugsweise THF, und anschliessende Umsetzung mit 1,4-Dibrombutan erhält man eine Verbindung der Formel B. Diese Umsetzung erfolgt vorzugsweise unter Schutzgasatmosphäre bei tiefen Temperaturen, bevorzugt in einem Temperaturbereich von etwa -50°C bis etwa - 80°C.

Die Verbindungen der Formel B werden mit (Triphenylphosphonio)methanid unter Schutzgasatmosphäre behandelt, wobei Verbindungen der Formel C entstehen. (Triphenylphosphonio)methanid wird bevorzugt in situ aus einem Methyltriphenylphosphoniumhalogenid/Alkaliamid-Gemisch in einem inerten Lösungsmitel wie beispielsweise THF bei Raumtemperatur erzeugt.

Durch Umsetzung einer Verbindung der Formel C mit Chlorsulfonylisocyanat in einem geeigneten Lösungsmittel, beispielsweise einem Ether, unter Schutzgasatmosphäre und bei tiefer Temperatur, bevorzugt bei etwa -50°C bis etwa - 80°C, erhält man die gewünschte Verbindung der Formel II.

Zur Herstellung von Verbindungen der Formel III geht man analog vor, allerdings ausgehend von Analoga von Verbindungen der Formel A, worin eines von $R^{41}$ und $R^{51}$ Wasserstoff und das andere Arylmethoxy bedeutet; anschliessend geht man analog zu Verfahrensvariante a) und gegebenenfalls b) und allenfalls c) vor.

Die Synthese einer Verbindung der Formel IV beginnt abweichend von den oben beschriebenen Methoden nach folgendem Schema:

## Reaktionsschema II

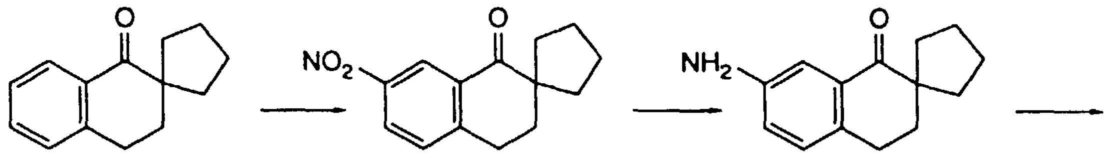

B'    D    E

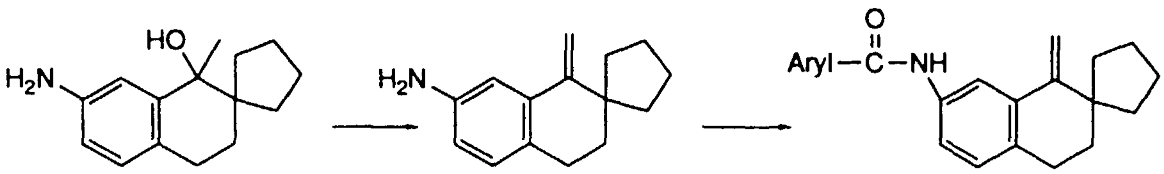

F    G    H

a) Zuerst wird eine Verbindung der Formel B' mit einem Nitrierungsmittel, beispielsweise mit einem Schwefelsäure/Salpetersäure-Gemisch behandelt. Als Lösungsmittel dient zweckmässigerweise Schwefelsäure, und die Reaktion erfolgt vorzugsweise bei der Rückflusstemperatur des Reaktionsgemisches.

b) Die so erhaltene Nitro-spiro-Verbindung der Formel D wird in einem inerten Lösungsmittel wie beispielsweise THF bei der Rückflusstemperatur des Reaktionsgemisches in Gegenwart eines geeigneten Katalysators, beispielsweise eines Pd/C-Katalysators, hydriert.

c) Die Amino-Verbindung der Formel E wird in einem inerten Lösungsmittel, vorzugsweise einem Ether, mit Methyllithium, behandelt. Vorzugsweise wird bei der Rückflusstemperatur gearbeitet.

d) Die Verbindung der Formel F wird mit geeigneten Mitteln, beispielsweise mit Jod, zur entsprechenden Methylen-Verbindung der Formel G dehydratisiert.

e) Durch Umsetzung der Verbindung der Formel G mit einem Arylcarbonsäurederivat, beispielsweise Benzoylchlorid, in Gegenwart einer Base wie beispielsweise Triethylamin in einem inerten Lösungsmittel wie THF oder ähnlichen Lösungsmitteln, erhält man eine Verbindung der Formel H. Zur entsprechenden Verbindung IV gelangt man durch Umsetzung mit Chlorsulfonylisocyanat und anschliessende Reduktion mit Lithiumaluminiumhydrid, worauf gegebenenfalls analog zu Verfahrensvariante b) vorgegangen wird.

Die als Zwischenprodukte verwendeten Verbindungen der Formel II sind neu und ebenfalls Gegenstand der vorliegenden Erfindung, und dasselbe gilt für Analoga von Verbindungen der Formel II, worin aber eines von $R^{41}$ und $R^{51}$ Wasserstoff und das andere Arylmethoxy oder Arylcarbonylamino bedeutet. Weiterhin sind auch die Zwischenprodukte der Formeln III und IV neu und ebenfalls Gegenstand der vorliegenden Erfindung.

Wie bereits eingangs erwähnt besitzen die Verbindungen der Formel I sowie rac-cis-4b-(2-Amino-ethyl)- 4b,5,6,7,8,8a,9,10-octahydro-phenanthren-8a-ol eine pharmakologische Aktivität als nicht-kompetitive NMDA-Antagonisten. Auf Grund dieser Aktivität können die Verbindungen der Formel I oder rac-cis-4b-(2-Amino-ethyl)- 4b,5,6,7,8,8a,9,10-octahydro-phenanthren-8a-ol und deren pharmazeutisch verwendbaren Säureadditionssalze als Neuroprotectiva verwendet werden, insbesondere zur Behandlung oder Verhütung von Ischämie, Hypoglykämie, Hypoxie, cerebralen Gefässpasmen, Spastizität, Trauma, Hemorrhagie, Infektionen (viral, bakteriell, amöbisch, prional), epileptischen Anfällen, autoimmunen Erkrankungen, Entzugssymptomen, Alzheimerscher Krankheit, Parkinsonscher Krankheit, amyotropher Lateralsclerose, Morbus Huntington,Vergiftungen, Olivoponto-cerebellarer Atrophie, Rückenmarkverletzung, Schizophrenie, Depressionen, Angstzuständen, Abhängigkeit, Schmerzen, Autismus und mentaler Retardierung.

Die pharmakologische Aktivität einiger erfindungsgemässer Verbindungen sowie von rac-cis-4b-(2-Amino-ethyl)-4b,5,6,7,8,8a,9,10-octahydro-phenanthren-8a-ol wurde in vitro nach der in Europ. J. Pharmacol. 135, 261 (1987) beschriebenen Methode festgestellt. Nach dieser Methode (nämlich dem 3H-MK801 (Dizocilpin)-Bindungstest) ermittelt man die Hemmung der Bindung von Dizocilpin an den spezifischen Bindungsstellen im Ratten-Cortex durch die jeweiligen Testsubstanzen. Man bezeichnet als $IC_{50}$ ("50% inhibiting concentration") diejenige Konzentration der jeweiligen Testsubstanz, welche eine 50-proz. Hemmung der spezifischen Bindung von Dizocilpin an die spezifischen Bindungstellen im Ratten-Cortex bewirkt.

Die so ermittelte Aktivität einiger erfindungsgemässer Verbindungen wird aus den in der folgenden Tabelle aufgeführten $IC_{50}$-Werten ersichtlich; ausserdem erhält die Tabelle Angaben über deren Toxizität (niedrigste letale Dosis = LLD an der Maus, p.o.).

| **Testsubstanz** | **$[^3H]$-MK801 Binding, $IC_{50}$ [nM/l]** | **Toxizität LLD (mg/kg)** |
|---|---|---|
| rac-cis-4b-(2-Amino-ethyl)-3-chloro-4b,5,6,7,8,8a, 9,10-octahydro-phenanthren-8a-ol hydrochlorid | 84.7 | 500 |
| rac-cis-4b-(2-Amino-ethyl)-3-chloro-1-fluoro-4b, 5,6,7,8,8a,9,10-octahydro-phenanthren-8a-ol hydrochlorid | 107 | 1000 |
| rac-cis-4b-(2-Amino-ethyl)-4b,5,6,7,8,8a, 9,10-octahydro-phenanthren-3,8a-diol hydrochlorid | 73.4 | 1000 |
| rac-cis-4b-(2-Dimethylamino-ethyl)-4b,5,6,7,8,8a, 9,10-octahydro-phenanthren-8a-ol hydrochlorid | 453 | - |
| (-)-cis-3-Chloro-4b-(2-dimethylamino-ethyl)-4b, 5,6,7,8,8a,9,10-octahydro-phenanthren-8a-ol hydrochlorid | 254 | 375 |
| rac-cis-4b-(2-Amino-ethyl)-4b,5,6,7,8,8a, 9,10-octahydro-phenanthren-8a-ol hydrochlorid | 93 | 250 |

Die Verbindungen der Formel I sowie rac-cis-4b-(2-Amino-ethyl)-4b,5,6,7,8,8a,9,10-octahydro-phenanthren-8a-ol und deren pharmazeutisch verwendbare Salze können als Heilmittel, z.B. in Form pharmazeutischer Präparate, Verwendung finden. Die pharmazeutischen Präparate können enteral, wie oral, z.B. in Form von Tabletten, Lacktabletten, Dragees, Hart- und Weichgelatinekapseln, Lösungen, Emulsionen oder Suspensionen, nasal, z.B. in Form Nasensprays, oder rektal, z.B. in Form von Suppositorien, verabreicht werden. Die Verabreichung kann auch parenteral, wie subcutan, intramuskulär oder intravenös, z.B. in Form von Injektionslösungen, erfolgen. Zur Herstellung von Tabletten, Lacktabletten, Dragees und Hartgelatinekapseln können die Verbindungen sowie ihre pharmazeutisch verwendbaren Salze mit pharmazeutisch inerten, anorganischen oder organischen Excipientien verarbeitet werden. Als solche Excipientien kann man z.B. für Tabletten, Dragees und Hartgelatinekapseln, Laktose, Maisstärke oder Derivate davon, Talk, Stearinsäure oder deren Salze etc. verwenden. Für Weichgelatinekapseln eignen sich als Excipientien z. B. vegetabile Öle, Wachse, Fette, halbfeste und flüssige Polyole etc. Zur Herstellung von Lösungen und Syrupen eignen sich als Excipientien z.B. Wasser, Polyole, Saccharose, Invertzucker, Glukose etc. Für Injektionslösungen eignen sich als Excipientien z.B. Wasser, Alkohole, Polyole, Glycerin, vegetabile Öle, etc. Für Suppositorien eignen sich als Excipientien z.B. natürliche oder gehärtete Öle, Wachse, Fette, halbflüssige oder flüssige Polyole etc. Die pharmazeutischen Präparate können daneben noch Konservierungsmittel, Lösungsvermittler, viskositätserhöhende Stoffe, Stabilisierungsmittel, Netzmittel, Emulgiermittel, Süssmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Erfindungsgemäss kann man Verbindungen der allgemeinen Formel I sowie rac-cis-4b-(2-Amino-ethyl)-4b, 5,6,7,8,8a,9,10-octahydro-phenanthren-8a-ol und deren pharmazeutisch annehmbare Säureadditionssalze als Neuroprotectiva verwenden, insbesondere bei der Behandlung oder Verhütung von Ischämie, Hypoglykämie, Hypoxie, cerebralen Gefässpasmen, Spastizität, Trauma, Hemorrhagie, Infektionen (viral, bakteriell, amöbisch, prional), epileptischen Anfällen, autoimmunen Erkrankungen, Entzugssymptomen, Alzheimerscher Krankheit, Parkinsonscher Krankheit, amyotropher Lateralsclerose, Morbus Huntington, Vergiftungen, Olivoponto-cerebellarer Atrophie, Rückenmarkverletzung, Schizophrenie, Depressionen, Angstzuständen, Abhängigkeit, Schmerzen, Autismus und mentaler Retardierung. Die Dosierung kann innerhalb weiter Grenzen variieren und ist natürlich in jedem einzelnen Fall den individuellen Gegebenheiten anzupassen. Bei oraler Verabreichung liegt die tägliche Dosis in einem Bereich von etwa 50 - 500 mg , wobei aber die obere Grenze auch überschritten werden kann, wenn sich dies als angezeigt erweisen sollte.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

**Beispiel 1**

**1.1.** Zu einer Lösung von 32.0 g 7-Fluoro-1,2,3,4-tetrahydro-naphthalen-1-on (0.19 Mol) in 320 ml THF unter Argonbegasung gab man portionsweise bei -78°C 43.7 g Kalium tert-Butylat (0.39 Mol) zu. Man rührte 2 St bei -78°C nach und tropfte dann eine Lösung von 40 ml 1,4-Dibrombutan (73 g, 0.338 Mol) in 40 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Toluol. Das Produkt wurde durch eine 20 cm Vigreux Säule destilliert. Ausbeute: 29.4 g (76%) 7'-Fluoro-3'4'-dihydro-spiro[cyclopentan-1,2'(1'H)-naphthalen]-1'-on als farblose Flüssigkeit; Sdp. 98-102°C/0.11 Torr.

**1.2.** 123 g Methyltriphenylphosphoniumbromid/Natriumamid-Gemisch (0.29 Mol) wurden während $^3/_4$ St in 500 ml THF bei RT unter Argon gerührt. Zu der gelben Suspension tropfte man eine Lösung von 29.4 g 7'-Fluoro-3'4'-dihydro-spiro[cyclopentan-1,2'(1'H)-naphthalen]-1'-on (0.13 Mol) in 50 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Toluol. Ausbeute: 26.3 g (90%) 7'-Fluoro-1'-methylen-3'4'-dihydro-spiro[cyclopentan-1,2'(1'H)-naphthalen] als farblose Flüssigkeit.

MS: m/e (% Basis peak) = 216 ($C_{15}H_{17}F^+$, 20), 175 (100), 159 (24), 147 (16), 133 (15), 109(4).

**1.3.** Zu einer Lösung von 26.3 g 7'-Fluoro-1'-methylen-3'4'-dihydrospiro[cyclopentan-1,2'(1'H)-naphthalen] (0.12 Mol) in 250 ml Ether bei -78°C unter Argonbegasung tropfte man eine Lösung von 12.9 ml Chlorsulfonylisocyanat (0.15 Mol) in 40 ml Ether zu. Man rührte 15 min bei -78°C, liess zur RT erwärmen und rührte noch 15 St. Das entstandene Kristallisat wurde abgenutscht, mit Ether gewaschen und i.Vak. getrocknet. Ausbeute: 27.1 g (62%) rac-cis-8-Fluoro-1,2,4,5-tetrahydro-3a,9b-butano-naphtho[2,1-b]furan-2-yliden-sulfamoylchlorid [(E) oder (Z) oder (E/Z)-Gemisch] als gelbliche Kristalle; Schmp. 161-162°C.

**1.4.** 14,4 g (0,38 Mol) Lithiumaluminiumhydrid wurden in 300 ml Dioxan unter Argon suspendiert. Man tropfte eine Lösung aus 27.1 g rac-cis-8-Fluoro-1,2,4,5-tetrahydro-3a,9b-butano-naphtho[2,1-b]furan-2-yliden-sulfamoylchlorid [ (E) oder (Z) oder (E/Z)-Gemisch] (0.076 Mol) in 300 ml THF zu und kochte 17 St unter Rückfluss. Vorsichtig tropfte man nacheinander bei RT 15 ml Wasser, 15 ml einer 15 prozentigen wässrigen NaOH Lösung, und 45 ml Wasser zu, so dass ein völlig weisser Niederschlag entstand. Nach Filtrieren und Einengen wurde der Rückstand über Kieselgel mit Methanol/Ammoniak 9:1 chromatographiert. Das Produkt wurde in Acetonitril umkristallisiert. Ausbeute: 14.4 g (67%) rac-cis-4b-(2-Amino-ethyl)-3-fluoro-4,5,6,7,8,8a,9,10-octahydro-phenanthren-8a-ol als weisse Kristalle; Schmp. 148-149°C.

**1.5.** rac-cis-4b-(2-Amino-ethyl)-3-fluoro-4b,5,6,7,8,8a,9,10-octahydrophenanthren-8a-ol (0.81 g, 0.003 Mol) wurde in 10 ml Methanol gelöst. Man tropfte eine 4.89 N ethanolische HCl-Lösung (0.63 ml, 0.003 Mol) zu und engte ein. Der Rückstand kristallisierte aus Ether. Ausbeute: 0.68 g (74%) rac-cis-4b-(2-Amino-ethyl)-3-fluoro-4b,5,6,7,8,8a,9,10-octahydro-phenanthren-8a-ol hydrochlorid als weisse Kristalle; Schmp. 233-235°C.

**Beispiel 2**

**2.1.** Zu einer Lösung von 31.7 g 7-Chloro-1,2,3,4-tetrahydro-naphthalen-1-on (0.17 Mol) in 500 ml THF unter Argonbegasung gab man portionsweise bei -78°C 39.4 g Kalium tert-Butylat (0.35 Mol) zu. Man rührte 2 St bei -78°C nach und tropfte dann eine Lösung von 35.8 ml 1,4-Dibrombutan (65.4 g, 0.3 Mol) in 100 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Toluol 1:1. Ausbeute: 29.8 g (86%) 7'-Chloro-3'4'-dihydro-spiro-[cyclopentan-1,2'(1'H)-naphthalen]-1'-on als farblose Flüssigkeit.

MS: m/e (% Basis peak) = 234 ($C_{14}H_{15}ClO^+$, 22), 193 (100), 165 (12), 152 (19), 124 (19),89(18).

**2.2.** 116 g Methyltriphenylphosphoniumbromid/Natriumamid-Gemisch (0.494 Mol) wurden während $^3/_4$ St in 1 l THF bei RT unter Argon gerührt. Zu der gelben Suspension tropfte man eine Lösung von 29.8 g 7'-Chloro-3'4'-dihydrospiro[cyclopentan-1,2'(1'H)-naphthalen]-1'-on (0.127 Mol) in 100 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Toluol. Ausbeute: 26.1 g (88%) 7'-Chloro-1'-methylen-3'4'-dihydro-spiro[cyclopentan-1,2'(1'H)-naphthalen] als farblose Flüssigkeit.

MS: m/e (% Basis peak) = 232 ($C_{15}H_{17}Cl^+$, 21), 217 (5), 191 (100), 175 (12), 153 (8), 141(8),115(10).

**2.3.** Zu einer Lösung von 26.1 g 7'-Chloro-1'-methylen-3'4'-dihydrospiro[cyclopentan-1,2'(1'H)-naphthalen] (0.11 Mol) in 260 ml Ether bei -78°C unter Argonbegasung tropfte man eine Lösung von 11.9 ml Chlorsulfonylisocyanat (0.13 Mol) in 30 ml Ether zu. Man rührte 15 min bei -78°C, liess zur RT erwärmen und rührte noch 15 St. Das entstandene Kristallisat wurde abgenutscht, mit Ether gewaschen und i. Vak. getrocknet. Ausbeute: 26.2 g (63%) rac-cis-8-Chloro-1,2,4,5-tetrahydro-3a,9b-butano-naphtho[2,1-b]furan-2-yliden-sulfamoylchlorid [(E) oder (Z) oder (E/Z)-Gemisch] als gelbliche Kristalle; Schmp. 192-194°C.

**2.4.** 13.3 g (0,35 Mol) Lithiumaluminiumhydrid wurden in 350 ml Dioxan unter Argon suspendiert. Man tropfte eine Lösung aus 26.2 g rac-cis-8-Chloro-1,2,4,5-tetrahydro-3a,9b-butano-naphtho[2,1-b]furan-2-yliden-sulfamoylchlorid [(E) oder (Z) oder (E/Z)-Gemisch] (0.070 Mol) in 800 ml THF zu und kochte 16 St unter Rückfluss. Vorsichtig tropfte man nacheinander bei -40°C 70 ml Essigester, 14 ml Wasser, 14 ml einer 15 prozentigen wässrigen NaOH Lösung, und 42 ml Wasser zu, so dass ein völlig weisser Niederschlag entstand. Nach Filtrieren und Einengen wurde der Rückstand über Kieselgel mit Methanol/Ammoniak 99:1 chromatographiert. Das Produkt wurde in Ether suspendiert, abgenutscht, mit Ether gewaschen und i. Vak. getrocknet. Ausbeute: 6.9 g (35%) rac-cis-4b-(2-Amino-ethyl)-3-chloro-4b,5,6,7,8,8a,9,10-octahydro-phenanthren-8a-ol als weisse Kristalle; Schmp. 143-145°C.

**2.5.** rac-cis-4b-(2-Amino-ethyl)-3-chloro-4b,5,6,7,8,8a,9,10-octahydrophenanthren-8a-ol (1.54 g, 0.0055 Mol) wurde in 50 ml Methanol gelöst. Man tropfte eine 4.89 N ethanolische HCl-Lösung (1.12 ml, 0.0055 Mol) zu und engte ein. Der Rückstand kristallisierte aus Ether. Ausbeute: 1.56 g (90%) rac-cis-4b-(2-Amino-ethyl)-3-chloro-4b,5,6,7,8,8a,9,10-octahydro-phenanthren-8a-ol hydrochlorid als weisse Kristalle; Schmp. 280-282°C.

**Beispiel 3**

**3.1.** Zu einer Lösung von 14.2 g 7-Bromo-1,2,3,4-tetrahydro-naphthalen-1-on (0.063 Mol) in 250 ml THF unter Argonbegasung gab man portionsweise bei -78°C 14.2 g Kalium tert-Butylat (0.126 Mol) zu. Man rührte 2 St bei -78°C nach und tropfte dann eine Lösung von 12.7 ml 1,4-Dibrombutan (23.1 g, 0.107 Mol) in 250 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Toluol. Ausbeute: 9.81 g (56%) 7'-Bromo-3'4'-dihydro-spiro[cyclopentan-1,2'(1'H)-naphthalen]-1'-on als gelbliches Öl.

MS: m/e (% Basis peak) = 278, 280 ($C_{14}H_{15}BrO^+$, 21), 237, 239 (100), 196, 198 (17), 168, 170 (20),128, 130( 18), 89 (34).

**3.2.** 38.1 g Methyltriphenylphosphoniumbromid/Natriumamid Gemisch (0.091 Mol) wurden während $^3/_4$ St in 380 ml THF bei RT unter Argon gerührt. Zu der gelben Suspension tropfte man eine Lösung von 11.7 g 7'-Bromo-3'4'-dihydro-spiro[cyclopentan-1,2'(1'H)-naphthalen]-1'-on (0.042 Mol) in 140 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab und engte ein. Der Rückstand wurde über Kieselgel mit Petrolether chromatographiert. Ausbeute: 8.42 g (77%) 7'-Bromo-1'-methylen-3'4'-dihydro-spiro[cyclopentan-1,2'(1'H)-naphthalen] als gelbliches Öl.

MS: m/e (% Basis peak) = 276, 278 ($C_{15}H_{17}Br^+$, 19), 235, 237 (100), 219, 221 (8), 156 (20), 141 (23), 128 (27), 115 (26).

**3.3.** Zu einer Lösung von 8.42 g 7'-Bromo-1'-methylen-3'4'-dihydro-spiro-[cyclopentan-1,2'(1'H)-naphthalen] (0.029 Mol) in 85 ml Ether bei -78°C unter Argonbegasung tropfte man eine Lösung von 3.02 ml Chlorsulfonylisocyanat (0.034 Mol) in 30 ml Ether zu. Man rührte 15 min bei -78°C, liess zu RT erwärmen und rührte noch 16 St nach. Das entstandene Kristallisat wurde abgenutscht, mit Ether gewaschen und i. Vak. getrocknet. Ausbeute: 5.08 g (42%) rac-cis-8-Bromo-1,2,4,5-tetrahydro-3a,9b-butano-naphtho[2,1-b]furan-2-yliden-sulfamoylchlorid [(E) oder (Z) oder (E/Z)-Gemisch] als gelbliche Kristalle; Schmp. 195-197°C.

**3.4.** 1.19 g ( 0,054 Mol) Lithiumborhydrid wurden in 95 ml Dioxan unter Argon suspendiert. Man tropfte eine Lösung aus 5.08 g rac-cis-8-Bromo-1,2,4,5-tetrahydro-3a,9b-butano-naphtho[2,1-b]furan-2-yliden-sulfamoylchlorid [(E) oder (Z) oder (E/Z)-Gemisch] (0.012 Mol) in 250 ml THF zu und kochte 16 St unter Rückfluss. Vorsichtig tropfte man nacheinander bei RT 1.2 ml Wasser, 1.2 ml einer 15 prozentigen wässrigen NaOH Lösung, und 3.6 ml Wasser zu, so dass ein völlig weisser Niederschlag entstand. Nach Filtrieren und Einengen wurde der Rückstand über Kieselgel mit Isopropylalkohol/Ammoniak 19:1 chromatographiert. Das Produkt kristallisierte aus Acetonitril. Ausbeute: 1.29 g (33%) rac-cis-4b-(2-Amino-ethyl)-3-bromo-4b-5,6,7,8,8a,9,10-octahydrophenanthren-8a-ol als weisse Kristalle; Schmp. 135-136°C.

**3.5.** rac-cis-4b-(2-Amino-ethyl)-3-bromo-4b,5,6,7,8,8a,9,10-octahydrophenanthren-8a-ol (0.57 g, 0.0017Mol) wurde in 20 ml Methanol gelöst. Man tropfte eine 4.89 N ethanolische HCl-Lösung (0.36 ml, 0.0017 Mol) zu und engte ein. Der Rückstand kristallisierte aus Ethanol/Ether. Ausbeute: 0.23 g (37%) rac-cis-4b-(2-Amino-ethyl)-3-bromo-4b,5,6,7,8,8a,9,10-octahydro-phenanthren-8a-ol hydrochlorid als weisse Kristalle; Schmp. 283-286°C.

**Beispiel 4**

**4.1.** Zu einer Lösung von 12.3 g 7-Iodo-1,2,3,4-tetrahydro-naphthalen-1-on (0.045 Mol) in 200 ml THF unter Argonbegasung gab man portionsweise bei -78°C 10.2 g Kalium tert-Butylat (0.090 Mol) zu. Man rührte 2 St bei -78°C

nach und tropfte dann eine Lösung von 9.25 ml 1,4-Dibrombutan (16.9 g, 0.078 Mol) in 25 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Toluol. Ausbeute: 8.67 g (59%) 7'-Iodo-3'4'-dihydro-spiro[cyclopentan-1,2'(1'H)-naphthalen]-1'-on als farblose Flüssigkeit.

MS: m/e (% Basis peak) = 326 ($C_{14}H_{15}IO^+$, 30), 285 (100), 243 (14), 21(12), 89 (14).

**4.2.** 24.4 g Methyltriphenylphosphoniumbromid/Natriumamid-Gemisch (0.058 Mol) wurden während $^3/_4$ St in 200 ml THF bei RT unter Argon gerührt. Zu der gelben Suspension tropfte man eine Lösung von 7.19 g 7'-Iodo-3'4'-dihydro-spiro[cyclopentan-1,2'(1'H)-naphthalen]-1'-on (0.027 Mol) in 50 ml THF zu und rührte dann 18 St nach. Man fügte Wasser zu, schüttelte mit Ether aus, wusch die organische Phase mit einer gesättigten wässrigen

Kochsalzlösung, trocknete über $MgSO_4$, filtrierte ab und engte ein. Der Rückstand wurde über Kieselgel mit Toluol chromatographiert. Ausbeute: 8.16 g (95%) 7'-Iodo-1'-methylen-3'4'-dihydro-spiro[cyclopentan-1,2'(1'H)-naphthalen] als gelbliches Öl.

MS: m/e (% Basis peak) = 324 ($C_{15}H_{17}I^+$, 25), 283 (100), 156 (11), 141 (15), 128 (16), 115 (15).

**4.3.** Zu einer Lösung von 8.16 g 7'-Iodo-1'-methylen-3'4'-dihydrospiro[cyclopentan-1,2'(1'H)-naphthalen] (0.025 Mol) in 80 ml Ether bei -78°C unter Argonbegasung tropfte man eine Lösung von 2.6 ml Chlorsulfonylisocyanat (0.030 Mol) in 20 ml Ether zu. Man rührte 15 min bei -78°C, liess zur RT erwärmen und rührte noch 16 St nach. Das entstandene Kristallisat wurde abgenutscht, mit Ether gewaschen und i. Vak. getrocknet. Ausbeute: 6.64 g (57%) rac-cis-1,2,4,5-Tetrahydro-8-iodo-3a,9b-butano-naphtho[2,1-b]furan-2-yliden-sulfamoylchlorid [(E) oder (Z) oder (E/Z)-Gemisch] als gelbliche Kristalle; Schmp.190-192°C.

**4.4.** 1.53 g ( 0,07 Mol) Lithiumborhydrid wurden in 100 ml Dioxan unter Argon suspendiert. Man tropfte eine Lösung aus 6.38 g rac-cis-1,2,4,5-Tetrahydro-8-iodo-3a,9b-butano-naphtho[2,1-b]furan-2-yliden-sulfamoylchlorid [(E) oder (Z) oder (E/Z)-Gemisch] (0.014 Mol) in 100 ml THF zu und kochte 2 St unter Rückfluss. Vorsichtig tropfte man nacheinander bei RT 1.5 ml Wasser, 1.5 ml einer 15 prozentigen wässrigen NaOH Lösung, und 4.5 ml Wasser zu, so dass ein völlig weisser Niederschlag entstand. Nach Filtrieren und Einengen wurde der Rückstand über Kieselgel mit Methanol/Ammoniak 99:1 chromatographiert. Das Produkt wurde in Ether suspendiert, abgenutscht, mit Ether gewaschen und i. Vak. getrocknet. Ausbeute: 1.43 g (28%) rac-cis-4b-(2-Amino-ethyl)-3-iodo-4b,5,6,7,8,8a,9,10-octahydro-phenanthren-8a-ol als weisse Kristalle; Schmp. 144-146°C.

**4.5.** rac-cis-4b-(2-Amino-ethyl)-3-iodo-4b,5,6,7,8,8a,9,10-octahydro-phenanthren-8a-ol (1.39 g, 0.0037 Mol) wurde in 40 ml Methanol gelöst. Man tropfte eine 4.89 N ethanolische HCl-Lösung (0.78 ml, 0.0037 Mol) zu und engte ein. Der Rückstand kristallisierte aus Ethanol/Ether. Ausbeute: 1.26 g (83%) rac-cis-4b-(2-Amino-ethyl)-3-iodo-4b, 5,6,7,8,8a,9,10-octahydro-phenanthren-8a-ol hydrochlorid als weisse Kristalle; Schmp. 271-273°C.

**Beispiel 5**

**5.1.** Zu einer Lösung von 99.5 g 7-Benzyloxy-1,2,3,4-tetrahydro-naphthalen-1-on ( 0.39 Mol) in 1 l THF unter Argonbegasung gab man portionsweise bei -78°C 88.5 g Kalium tert-Butylat (0.79 Mol) zu. Man rührte 2 St bei -78°C nach und tropfte dann eine Lösung von 80 ml 1,4-Dibrombutan (146 g, 0.67 Mol) in 100 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Essigester 9:1. Nach kräftigem Rühren in Hexan erhielt man eine Suspension, die abgenutscht und i. Vak. getrocknet wurde. Ausbeute: 48.5 g (77%) 7'-Benzyloxy-3'4'-dihydro-spiro [cyclopentan-1,2'(1'H)-naphthalen]-1'-on als weisse Kristalle; Schmp.61-63°C.

**5.2.** 84.9 g Methyltriphenylphosphoniumbromid/Natriumamid-Gemisch (0.2 Mol) wurden während $^3/_4$ St in 840 ml THF bei RT unter Argon gerührt. Zu der gelben Suspension tropfte man eine Lösung von 28.6 g 7'-Benzyloxy-3'4'-dihydro-spiro[cyclopentan-1,2'(1'H)-naphthalen]-1'-on (0.093 Mol) in 100 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab und engte ein. Das Produkt wurde mittels eines Kugelrohr-Apparates destilliert. Ausbeute: 28.7 g (100%) 7'-Benzyloxy-1'-methylen-3'4'-dihydro-spiro[cyclopentan-1,2'(1'H)-naphthalen] als farblose Flüssigkeit; Sdp. 230-250°C/0.04 Torr.

**5.3.** Zu einer Lösung von 25.7 g 7'-Benzyloxy-1'-methylen-3'4'-dihydro-spiro-[cyclopentan-1,2'(1'H)-naphthalen] (0.084 Mol) in 300 ml Ether bei -78°C unter Argonbegasung tropfte man eine Lösung von 8.5 ml Chlorsulfonylisocyanat (0.097 Mol) in 50 ml Ether zu. Man rührte 15 min bei -78°C und liess innerhalb 1 St zur RT erwärmen. Das entstandene Kristallisat wurde abgenutscht, mit Ether gewaschen und i. Vak. getrocknet. Ausbeute: 26.6 g (70%) rac-cis-8-Benzyloxy-1,2,4,5-tetrahydro-3a,9b-butano-naphtho[2,1-b]furan-2-ylidensulfamoylchlorid [(E) oder (Z) oder (E/Z)-Gemisch] als gelbliche Kristalle; Schmp.191-192°C.

**5.4.** 22,4 g ( 0,59 Mol) Lithiumaluminiumhydrid wurden in 800 ml Dioxan unter Argon suspendiert. Man tropfte eine Lösung aus 52.7 g rac-cis-8-Benzyloxy-1,2,4,5-tetrahydro-3a,9b-butano-naphtho[2,1-b]furan-2-ylidensulfamoylchlorid

[(E) oder (Z) oder (E/Z)-Gemisch] (0.12 Mol) in 2.2 l THF zu und kochte 24 St unter Rückfluss. Vorsichtig tropfte man nacheinander bei RT 22.4 ml Wasser, 22.4 ml einer 15 prozentigen wässrigen NaOH Lösung, und 67.2 ml Wasser zu, so dass ein völlig weisser Niederschlag entstand. Nach Filtrieren und Einengen wurde der Rückstand über Kieselgel mit Methanol chromatographiert. Das Produkt wurde aus Acetonitril umkristallisiert und i. Vak. getrocknet. Ausbeute: 18.7 g (45%) rac-cis-4b-(2-Amino-ethyl)-3-benzyloxy-4b,5,6,7,8,8a,9,10-octahydro-phenanthren-8a-ol als weisse Kristalle; Schmp. 162-163°C.

**5.5.** 29 g rac-cis-4b-(2-Aminoethyl)-3-benzyloxy-4b,5,6,7,8,8a,9,10-octahydrophenanthren-8a-ol (0.0825 Mol) wurden in 550 ml Ameisensäureethylester und 35 ml Ameisensäure gelöst. Man kochte das Gemisch 130 St unter Rückfluss, engte ein und chromatographierte über Kieselgel mit Methanol/Ammoniak 19:1. Das Produkt kristallisierte aus THF/Acetonitril. Ausbeute: 17.6 g (56%) rac-cis-N-[2-(6-Benzyloxy-10a-hydroxy-1,2,3,4,4a,9,10,10a-octahydro-phenanthren-4a-yl)-ethyl]-formamid als weisse Kristalle; Schmp. 137-138°C.

**5.6.** 4,8 g (0.12 Mol) Lithiumaluminiumhydrid wurden in 600 ml THF unter Argon suspendiert. Man tropfte eine Lösung aus 17.6 g rac-cis-N-[2-(6-Benzyloxy-10a-hydroxy-1,2,3,4,4a,9,10,10a-octahydro-phenanthren-4a-yl)-ethyl]-formamid (0.046 Mol) in 200 ml THF zu und kochte 5 St. unter Rückfluss. Vorsichtig tropfte man ein Gemisch von 20 ml Wasser und 100 ml THF zu, so dass ein völlig weisser Niederschlag entstand. Nach Filtrieren und Einengen wurde der Rückstand über Kieselgel mit $CH_2Cl_2$/Methanol/Ammoniak 90:9:1 chromatographiert. Das Produkt kristallisierte aus heissem Hexan. Ausbeute: 13.1 g (77%) rac-cis-3-Benzyloxy-4b-(2-methylamino-ethyl)-4b,5,6,7,8,8a,9,10-octahydro-phenanthren-8a-ol als weisse Kristalle; Schmp. 107-109°C.

**5.7.** Man löste 2.04 g rac-cis-3-Benzyloxy-4b-(2-methylamino-ethyl)-4b,5,6,7,8,8a,9,10-octahydro-phenanthren-8a-ol (0.005 Mol) in 150 ml Ethanol. Nach Zugabe von 0.55 g 10 prozentigem Pd-C Katalysator wurde das Gemisch während 1 St bei RT unter 1 Atm hydriert. Man filtrierte und engte ein. Das Produkt kristallisierte aus Methanol. Ausbeute: 1.04 g (68%) rac-cis-4b-(2-Methylamino-ethyl)-4b,5,6,7,8,8a,9,10-octahydro-phenanthren-3,8a-diol als weisse Kristalle; Schmp. 200-201°C.

**5.8.** rac-cis-4b-(2-Methylamino-ethyl)-4b,5,6,7,8,8a,9,10-octahydro-phenanthren-3,8a-diol (1 g, 0.0036 Mol) wurde in 25 ml Methanol gelöst. Man tropfte eine 1 N ethanolische HCl-Lösung (3.65 ml, 0.0036 Mol) zu und engte ein. Der Rückstand kristallisierte aus Ethanol. Ausbeute: 0.58 g (51%) rac-cis-4b-(2-Methylamino-ethyl)-4b,5,6,7,8,8a,9,10-octahydro-phenanthren-3,8a-diol hydrochlorid als weisse Kristalle; Schmp. 215-216°C.

**Beispiel 6**

**6.1.** Zu einer Lösung von 30 g 7-Methoxy-1,2,3,4-tetrahydro-naphthalen-1-on (0.17 Mol) in 350 ml THF unter Argonbegasung gab man portionsweise bei -78°C 38.2 g Kalium tert-Butylat (0.34 Mol) zu. Man rührte 2 St bei -78°C nach und tropfte dann eine Lösung von 34.2 ml 1,4-Dibrombutan (62.4 g, 0.289 Mol) in 150 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Ether 3:2. Das Produkt wurde durch eine 20 cm Vigreux Säule destilliert. Ausbeute: 32 g (82%) 3'4'-Dihydro-7'-methoxy-spiro-[cyclopentan-1,2'(1'H)-naphthalen]-1'-on als farblose Flüssigkeit; Sdp.140°C/5Torr.

**6.2.** 100 g Methyltriphenylphosphoniumbromid/Natriumamid-Gemisch (0.24 Mol) wurden während $^3/_4$ St in 500 ml THF bei RT unter Argon gerührt. Zu der gelben Suspension tropfte man eine Lösung von 46 g 3'4'-Dihydro-7'-methoxy-spiro-[cyclopentan-1,2'(1'H)-naphthalen]-1'-on (0.20 Mol) in 100 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab und engte ein. Der Rückstand wurde in 300 ml heissem Hexan suspendiert, auf 0°C abgekühlt und abfiltriert (der Filterkuchen besteht aus Triphenylphosphinoxid). Das Produkt wurde über Kieselgel mit Hexan/Ether 3:2 chromatographiert und durch eine 20 cm Vigreux Säule destilliert. Ausbeute: 33 g (72%) 7'-Methoxy-1'-methylen-3'4'-dihydro-spiro[cyclopentan-1,2'(1'H)-naphthalen] als farblose Flüssigkeit; Sdp. 155°C/9 Torr.

**6.3.** Zu einer Lösung von 33 g 7'-Methoxy-1'-methylen-3'4'-dihydrospiro[cyclopentan-1,2'(1'H)-naphthalen] (0.145 Mol) in 300 ml Ether bei -78°C unter Argonbegasung tropfte man 15.2 ml Chlorsulfonylisocyanat (0.173 Mol) zu. Man rührte 15 min bei -78°C und liess innerhalb 1 St zur RT erwärmen.

Das entstandene Kristallisat wurde abgenutscht, mit Ether gewaschen und i. Vak. getrocknet. Ausbeute: 24 g (45%) rac-cis-1,2,4,5-Tetrahydro-8-methoxy-3a,9b-butano-naphtho[2,1-b]furan-2-yliden-sulfamoylchlorid [(E) oder (Z) oder (E/Z)-Gemisch] als gelbliche Kristalle; Schmp. 152-153°C.

**6.4.** 2.57 g (0,068 Mol) Lithiumaluminiumhydrid wurden in 80 ml Dioxan unter Argon suspendiert. Bei -45°C tropfte man eine Lösung aus 5 g rac-cis-1,2,4,5-Tetrahydro-8-methoxy-3a,9b-butano-naphtho[2,1-b]furan-2-ylidensulfamoylchlorid [(E) oder (Z) oder (E/Z)-Gemisch] (0.014 Mol) in 80 ml THF zu und kochte $1^1/_2$ St unter Rückfluss. Vorsichtig tropfte man nacheinander bei RT 2.6 ml Wasser, 3.0 ml einer 15 prozentigen wässrigen NaOH Lösung, und 2.6 ml Wasser zu, so dass ein völlig weisser Niederschlag entstand. Nach Filtrieren und Einengen wurde das Produkt aus Hexan/Ether kristallisiert. Ausbeute: 2.3 g (62%) rac-cis-4b-(2-Amino-ethyl)-3-methoxy-4b,5,6,7,8,8a,9,10-octahydro-phenanthren-8a-ol als weisse Kristalle; Schmp. 103-106°C.

**Beispiel 7**

**7.1.** Zu einer Lösung von 26.8 g 5-Benzyloxy-1,2,3,4-tetrahydro-naphthalen-1-on (0.106 Mol) in 270 ml THF unter Argonbegasung gab man portionsweise bei -78°C 20.3 g Kalium tert-Butylat (0.181 Mol) zu. Man rührte 2 St bei -78°C nach und tropfte dann eine Lösung von 25.2 ml 1,4-Dibrombutan (45.9 g, 0.212 Mol) in 60 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Essigester 9:1. Ausbeute: 11.8 g (36%) 5'-Benzyloxy-3'4'-dihydrospiro-[cyclopentan-1,2'(1'H)-naphthalen]-1'-on als festes Material ; Schmp. 67-70°C.

**7.2.** 61.6 g Methyltriphenylphosphoniumbromid/Natriumamid-Gemisch (0.148 Mol) wurden während $^3/_4$ St in 600 ml THF bei RT unter Argon gerührt. Zu der gelben Suspension tropfte man eine Lösung von 20.5 g 5'-Benzyloxy-3'4'-dihydro-spiro[cyclopentan-1,2'(1'H)-naphthalen]-1'-on (0.0672 Mol) in 200 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Ether 3:1. Ausbeute: 19.8 g (97%) 5'-Benzyloxy-1'-methylen-3'4'-dihydro-spiro-[cyclopentan-1,2'(1'H)-naphthalen] als gelbliches Öl.
MS: m/e (% basis peak) = 304 ($C_{22}H_{24}O^+$, 8,5), 263 (17), 212 (2), 171 (1), 115 (2), 91 (100).

**7.3.** Zu einer Lösung von 1.80 g 5'-Benzyloxy-1'-methylen-3'4'-dihydrospiro[cyclopentan-1,2'(1'H)-naphthalen] (0.0059 Mol) in 20 ml Ether bei -78°C unter Argonbegasung tropfte man eine Lösung von 0.62 ml Chlorsulfonylisocyanat (0.0071 Mol) in 10 ml Ether zu. Man rührte 15 min bei -78°C und liess innerhalb 1 St zur RT erwärmen. Das entstandene Kristallisat wurde abgenutscht, mit Ether gewaschen und i. Vak. getrocknet. Ausbeute: 1.2 g (46%) rac-cis-6-Benzyloxy-1,2,4,5-tetrahydro-3a,9b-butano-naphtho[2,1-b]furan-2-yliden-sulfamoylchlorid [(E) oder (Z) oder (E/Z)-Gemisch] als weissliche Kristalle; Schmp. 167-168°C.

**7.4.** 7.0 g (0,185 Mol) Lithiumaluminiumhydrid wurden in 185 ml Dioxan unter Argon suspendiert. Man tropfte eine Lösung aus 16.5 g rac-cis-6-Benzyloxy-1,2,4,5-tetrahydro-3a,9b-butano-naphtho[2,1-b]furan-2-ylidensulfamoylchlorid [(E) oder (Z) oder (E/Z)-Gemisch] (0.037 Mol) in 340 ml THF zu und kochte 19 St unter Rückfluss. Vorsichtig tropfte man nacheinander bei ~40°C 50 ml Essigester, 7 ml Wasser, 14 ml einer 15 prozentigen wässrigen NaOH Lösung, und 7 ml Wasser zu, so dass ein völlig weisser Niederschlag entstand. Nach Filtrieren und Einengen wurde der Rückstand über Kieselgel mit Methanol/Ammoniak chromatographiert. Das Produkt wurde in Ether suspendiert, abgenutscht, mit Ether gewaschen und i. Vak. getrocknet. Ausbeute: 3.8 g (29%) rac-cis-4b-(2-Amino-ethyl)-1-benzyloxy-4b,5,6,7,8,8a,9,10-octahydro-phenanthren-8a-ol als weisse Kristalle; Schmp. 164-165°C.

**7.5.** Man löste 3.8 g rac-cis-4b-(2-Aminoethyl)-1-benzyloxy-4b,5,6,7,8,8a,9,10-octahydro-phenanthren-8a-ol (0.0109 Mol) in 100 ml Methanol. Nach Zugabe von 0.6 g 10 prozentigem Pd-C Katalysator wurde das Gemisch während 16 St bei RT unter 1 Atm hydriert. Man filtrierte und engte ein. Ausbeute: 2.9 g (90%) rac-cis-4b-(2-Amino-ethyl)-4b,5,6,7,8,8a,9,10-octahydro-phenanthren-1,8a-diol als weisser Schaum.

**7.6.** rac-cis-4b-(2-Amino-ethyl)-4b,5,6,7,8,8a,9,10-octahydro-phenanthren-1,8a-diol (2.9 g, 0.0097 Mol) wurde in 60 ml Ethanol gelöst. Man tropfte eine 5 N ethanolische HCl-Lösung (2.3 ml, 11.6 Mol) zu und engte ein. Nach kräftigem Rühren in Ether erhielt man eine Suspension, die abgenutscht und i. Vak. getrocknet wurde. Ausbeute: 2.85 g (88%) rac-cis-4b-(2-Amino-ethyl)-4b,5,6,7,8,8a,9,10-octahydro-phenanthren-1,8a-diol hydrochlorid als weisse Kristalle (88%) mit Schmp. 244-246°C.

**Beispiel 8**

**8.1.** Zu einer Lösung von 5.0 g 6-Benzyloxy-1,2,3,4-tetrahydro-naphthalen-1-on (0.0198 Mol) in 50 ml THF unter Argonbegasung gab man portionsweise bei -78°C 3.8 g Kalium tert-Butylat (0.0338 Mol) zu. Man rührte 2 St bei -78°C nach und tropfte dann eine Lösung von 4.7 ml 1,4-Dibrombutan (8.55 g, 0.0396 Mol) in 50 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Toluol. Das Produkt wurde aus Hexan/Ether umkristallisiert und i. Vak. getrocknet. Ausbeute: 2.0 g (33%) 6'-Benzyloxy-3'4'-dihydro-spiro-[cydopentan-1,2'(1'H)-naphthalen]-1'-on als weisse Kristalle ; Schmp. 79-81°C.

**8.2.** 5.9 g Methyltriphenylphosphoniumbromid/Natriumamid-Gemisch (0.0143 Mol) wurden während $^3/_4$ St in 60 ml THF bei RT unter Argon gerührt. Zu der gelben Suspension tropfte man eine Lösung von 2.0 g 6'-Benzyloxy-3'4'-dihydro-spiro-[cyclopentan-1,2'(1'H)-naphthalen]-1'-on (0.0065 Mol) in 20 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Ether 9:1. Ausbeute: 1.8 g (91%) 6'-Benzyloxy-1'-methylen-3'4'-dihydro-spiro[cyclopentan-1,2'(1'H)-naphthalen] als gelblicher Feststoff; Schmp. 64-65°C.

**8.3.** Zu einer Lösung von 1.8 g 6'-Benzyloxy-1'-methylen-3'4'-dihydrospiro[cyclopentan-1,2'(1'H)-naphthalen] (0.0059 Mol) in 60 ml Ether bei -78°C unter Argonbegasung tropfte man eine Lösung von 0.62 ml Chlorsulfonylisocyanat (0.0071 Mol) in 10 ml Ether zu. Man rührte 15 min bei -78°C und liess innerhalb 1 St zur RT erwärmen. Das Gemisch wurde eingeengt und der entstandene Schaum aus Ether umkristallisiert. Ausbeute: 1.3 g (49%) rac-cis-7-Benzyloxy-1,2,4,5-tetrahydro-3a,9b-butano-naphtho[2,1-b]furan-2-yliden-sulfamoylchlorid [(E) oder (Z) oder (E/Z)-Gemisch] als weisse Kristalle; Schmp. 155-157°C.

**8.4.** 8.6 g (0,227 Mol) Lithiumaluminiumhydrid wurden in 230 ml Dioxan unter Argon suspendiert. Man tropfte eine Lösung aus 20.3 g rac-cis-7-Benzyloxy-1,2,4,5-tetrahydro-3a,9b-butano-naphtho[2,1-b]furan-2-yliden-sulfamoylchlorid [(E) oder (Z) oder (E/Z)-Gemisch] (45.3 Mol) in 600 ml Dioxan zu und kochte 24 St unter Rückfluss. Vorsichtig tropfte man nacheinander bei -40°C 60 ml Essigester, 9 ml Wasser, 9 ml einer 15 prozentigen wässrigen NaOH Lösung, und 18 ml Wasser zu, so dass ein völlig weisser Niederschlag entstand. Nach Filtrieren und Einengen wurde der Rückstand über Kieselgel mit Methanol/Ammoniak 19:1 chromatographiert. Das Produkt wurde in Ether suspendiert, abgenutscht, mit Ether gewaschen und i. Vak. getrocknet. Ausbeute: 2.1 g (13%) rac-cis-4b-(2-Amino-ethyl)-2-benzyloxy-4b,5,6,7,8,8a,9,10-octahydro-phenanthren-8a-ol als weisse Kristalle; Schmp. 125-128°C.

**8.5.** Man löste 2.8 g rac-cis-4b-(2-Amino-ethyl)-2-benzyloxy-4b,5,6,7,8,8a,9,10-octahydro-phenanthren-8a-ol (0.008 Mol) in 80 ml Methanol. Nach Zugabe von 0.28 g 10 prozentigem Pd-C Katalysator wurde das Gemisch während 16 St bei RT unter 1 Atm hydriert. Man filtrierte und engte ein. Ausbeute: 2.0 g (96%) rac-cis-4b-(2-Amino-ethyl)-4b, 5,6,7,8,8a,9,10-octahydro-phenanthren-2,8a-diol als weisser Schaum.

**8.6.** rac-cis-4b-(2-Amino-ethyl)-4b,5,6,7,8,8a,9,10-octahydro-phenanthren-2,8a-diol (2.0 g, 0.067 Mol) wurde in 40 ml Methanol gelöst. Man tropfte eine 5 N ethanolische HCl-Lösung (1.6 ml, 0.008 Mol) zu und engte ein. Ausbeute: 2.3 g (97%) rac-cis-4b-(2-Amino-ethyl)-4b,5,6,7,8,8a,9,10-octahydro-phenanthren-2,8a-diol hydrochlorid als weisser Schaum.

MS: m/e (% Basis peak) = 261 ($C_{16}H_{23}NO_2^+$, 16), 217 (43), 199 (79), 187 (31), 173 (20), 157 (37), 133 (20), 107 (22), 45 (90), 30 (100).

**Beispiel 9**

**9.1.** Zu einer Lösung von 1.3 g 8-Benzyloxy-1,2,3,4-tetrahydro-naphthalen-1-on (0.005 Mol) in 15 ml THF unter Argonbegasung gab man portionsweise bei -78°C 0.95 g Kalium tert-Butylat (0.085 Mol) zu. Man rührte 2 St bei -78°C nach und tropfte dann eine Lösung von 1.2 ml 1,4-Dibrombutan (2.15 g, 0.010 Mol) in 6 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Essigester 9:1. Ausbeute: 0.5 g (33%) 8'-Benzyloxy-3'4'-dihydro-spiro[cyclopentan-1,2'(1'H)-naphthalen]-1'-on als gelbliche Flüssigkeit .

MS: m/e (% Basis peak) = 306 ($C_{21}H_{22}O_2^+$, 10), 288 (1), 265 (2), 224 (6), 197 (3), 159 (6),91(100).

**9.2.** 69 g Methyltriphenylphosphoniumbromid/Natriumamid-Gemisch (0.166 Mol) wurden während $^3/_4$ St in 700 ml THF bei RT unter Argon gerührt. Zu der gelben Suspension tropfte man eine Lösung von 20.3 g 8'-Benzyloxy-3'4'-dihydro-spiro[cyclopentan-1,2'(1'H)-naphthalen]-1'-on (0.0663 Mol) in 200 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan. Ausbeute: 11.2 g (55%) 8'-Benzyloxy-1'-methylen-3'4'-dihydro-spiro[cyclopentan-1,2'(1'H)-naphthalen] als gelbliche Flüssigkeit.

MS: m/e (% Basis peak) = 304 ($C_{22}H_{24}O^+$, 5.6), 263 (3.2), 222 (39), 213 (27), 200 (10), 131, (79), 91, (100).

**9.3.** Zu einer Lösung von 1.80 g 8'-Benzyloxy-1'-methylen-3'4'-dihydrospiro[cyclopentan-1,2'(1'H)-naphthalen] (0.0059 Mol) in 60 ml Ether bei -78°C unter Argonbegasung tropfte man eine Lösung von 0.62 ml Chlorsulfonylisocyanat (0.0071 Mol) in 6 ml Ether zu. Man rührte 15 min bei -78°C und liess innerhalb 1 St zur RT erwärmen. Das entstandene Kristallisat wurde abgenutscht, mit Ether gewaschen und i. Vak. getrocknet. Ausbeute: 1.2 g (46%) rac-cis-9-Benzyloxy-1,2,4,5-tetrahydro-3a,9b-butano-naphtho[2,1-b]furan-2-yliden-sulfamoylchlorid [(E) oder (Z) oder (E/Z)-Gemisch] als weissliche Kristalle; Schmp. 135-137°C.

**9.4.** 4.4 g (0,116 Mol) Lithiumaluminiumhydrid wurden in 115 ml Dioxan unter Argon suspendiert. Man tropfte eine Lösung aus 10.3 g rac-cis-9-Benzyloxy-1,2,4,5-tetrahydro-3a,9b-butano-naphtho[2,1-b]furan-2-yliden-sulfamoylchlorid [(E) oder (Z) oder (E/Z)-Gemisch] (0.0231 Mol) in 200 ml Dioxan/THF 1:1 zu und kochte 20 St unter Rückfluss. Vorsichtig tropfte man nacheinander bei -40°C 50 ml Essigester, 4 ml Wasser, 4 ml einer 15 prozentigen wässrigen NaOH Lösung, und 12 ml Wasser zu, so dass ein völlig weisser Niederschlag entstand. Nach Filtrieren und Einengen wurde der Rückstand über Kieselgel mit Methanol/Ammoniak 9:1 chromatographiert. Eine Probe wurde aus Ether/Hexan bei -20°C umkristallisiert. Ausbeute: 3.55 g (44%) rac-cis-4b(2-Amino-ethyl)-4-benzyloxy-4b,5,6,7,8,8a,9,10-octahydrophenanthren-8a-ol als weisser Schaum und weisse Kristalle; Schmp. 106-108°C.

**9.5.** Man löste 3.5 g rac-cis-4b-(2-Amino-ethyl)-4-benzyloxy-4b,5,6,7,8,8a,9,10-octahydro-phenanthren-8a-ol (0.00995 Mol) in 100 ml Methanol. Nach Zugabe von 0.35 g 10 prozentigem Pd-C Katalysator wurde das Gemisch während 70 St bei RT unter 1 Atm hydriert. Man filtrierte und engte ein. Ausbeute: 2.66 g (100%) rac-cis-4b-(2-Amino-ethyl)-4b,5,6,7,8,8a,9,10-octahydro-phenanthren-4,8a-diol als weisser Schaum.

MS: m/e (% Basis peak) = 261 ($C_{16}H_{23}NO_2^+$, 18), 244 (14), 226 (29), 199 (46), 187 (30), 173 (21), 157 (23), 145 (25), 133 (21), 30 (100).

**9.6.** rac-cis-4b-(2-Amino-ethyl)-4b,5,6,7,8,8a,9,10-octahydro-phenanthren-4,8a-diol (2.66 g, 0.0101 Mol) wurde in 60 ml Ethanol gelöst. Man tropfte eine 5 N ethanolische HCl-Lösung (2.5 ml, 0.0125 Mol) zu und engte ein. Der Rückstand kristallisierte aus Ethanol. Ausbeute: 2.62 g (87%) rac-cis-4b-(2-Amino-ethyl)-4b,5,6,7,8,8a,9,10-octahydro-phenanthren-4,8a-diol hydrochlorid als weisse Kristalle; Schmp. 248-249°C.

**Beispiel 10**

**10.1.** Zu einer Lösung von 75.6 g 5-Chloro-1,2,3,4-tetrahydro-naphthalen-1-on (0.419 Mol) in 760 ml THF unter Argonbegasung gab man portionsweise bei -78°C 93.8 g Kalium tert-Butylat (0.837 Mol) zu. Man rührte 2 St bei -78°C nach und tropfte dann eine Lösung von 84 ml 1,4-Dibrombutan (134 g, 0.711 Mol) in 350 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Toluol 1:1. Ausbeute: 54.4 g (55%) 5'-Chloro-3'4'-dihydrospiro [cyclopentan-1,2'(1'H)-naphthalen]-1'-on als farbloses Öl.

MS: m/e (% Basis peak) = 234 ($C_{14}H_{15}ClO^+$, 21), 193 (100), 165 (13), 152 (21), 124 (14), 89 (14).

**10.2.** 211 g Methyltriphenylphosphoniumbromid /Natriumamid-Gemisch (0.506 Mol) wurden während $^3/_4$ St in 1 l THF bei RT unter Argon gerührt. Zu der gelben Suspension tropfte man eine Lösung von 54.4 g 5'-Chloro-3'4'-dihydro-spiro[cydopentan-1,2'(1'H)-naphthalen]-1'-on (0.232 Mol) in 500 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Petrolether. Ausbeute: 40.5 g (75%) 5'-Chloro-1'-methylen-3'4'-dihydro-spiro[cyclopentan-1,2'(1'H)-naphthalen] als gelbliches Öl.

MS: m/e (% Basis peak) = 232 ($C_{15}H_{17}Cl^+$, 18), 191 (100), 175 (13), 155 (8), 141 (10), 128 (10), 115 (11).

**10.3.** Zu einer Lösung von 40.5 g 5'-Chloro-1'-methylen-3'4'-dihydrospiro[cyclopentan-1,2'(1'H)-naphthalen] (0.173 Mol) in 300 ml Ether bei -78°C unter Argonbegasung tropfte man eine Lösung von 18.2 ml Chlorsulfonylisocyanat (0.209 Mol) in 180 ml Ether zu. Man rührte 15 min bei -78°C und liess innerhalb 1 St zur RT erwärmen. Das entstandene Kristallisat wurde abgenutscht, mit Ether gewaschen und i. Vak. getrocknet. Ausbeute: 37.2 g (58%) rac-cis-6-Chloro-1,2,4,5-tetrahydro-3a,9b-butano-naphtho[2,1-b]furan-2-yliden-sulfamoylchlorid [(E) oder (Z) oder (E/Z)-Gemisch] als gelbliche Kristalle; Schmp.179-181°C.

**10.4.** 9,7 g (0,0447) Lithiumborhydrid wurden in 200 ml Dioxan unter Argon suspendiert. Man tropfte eine Lösung aus 37.2 g rac-cis-6-Chloro-1,2,4,5-tetrahydro-3a,9b-butano-naphtho[2,1-b]furan-2-yliden-sulfamoylchlorid [(E) oder (Z) oder (E/Z)-Gemisch] (0.099 Mol) in 550 ml THF zu und kochte 18 St unter Rückfluss. Vorsichtig tropfte man nacheinander bei ~40°C 10 ml Wasser, 10 ml einer 15 prozentigen wässrigen NaOH Lösung, und 30 ml Wasser zu, so dass ein völlig weisser Niederschlag entstand. Nach Filtrieren und Einengen wurde der Rückstand über Kieselgel mit Methanol/Ammoniak (19:1) chromatographiert. Das Produkt wurde aus heissem Acetonitril umkristallisiert. Ausbeute: 6.9 g (25%) rac-cis-4b-(2-Amino-ethyl)-1-chloro-4b,5,6,7,8,8a,9,10-octahydro-phenanthren-8a-ol als weisse Kristalle; Schmp. 161-162°C.

**10.5.** rac-cis-4b-(2-Amino-ethyl)-1-chloro-4b,5,6,7,8,8a,9,10-octahydrophenanthren-8a-ol (6.9 g, 0.0248 Mol) wurde in 200 ml THF gelöst. Man tropfte eine 5 N ethanolische HCl-Lösung (10 ml, 0.05 Mol) zu und nutschte die entstandene Suspension ab. Das Produkt wurde aus Ethanol/Ether umkristallisiert. Ausbeute: 6.7 g (85%) rac-cis-4b-(2-Amino-ethyl)-1-chloro-4b,5,6,7,8,8a,9,10-octahydro-phenanthren-8a-ol hydrochlorid als weisse Kristalle; Schmp. 269-271°C.

**Beispiel 11**

**11.1.** Zu einer Lösung von 40 g 6-Chloro-1,2,3,4-tetrahydro-naphthalen-1-on (0.221 Mol) in 400 ml THF unter Argonbegasung gab man portionsweise bei -78°C 42.2 g Kalium tert-Butylat (0.377 Mol) zu. Man rührte 2 St bei -78°C nach und tropfte dann eine Lösung von 52.4 ml 1,4-Dibrombutan (95.6 g, 0.443 Mol) in 260 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Essigester 9:1. Das Produkt kristallisierte aus Hexan bei -50°C. Ausbeute: 15 g (29%) 6'-Chloro-3'4'-dihydro-spiro[cyclopentan-1,2'(1'H)-naphthalen]-1'-on als weisse Kristalle;

Schmp.31-33°C.

**11.2.** 97.6 g Methyltriphenylphosphoniumbromid/Natriumamid-Gemisch (0.235 Mol) wurden während $^3/_4$ St in 1 l THF bei RT unter Argon gerührt. Zu der gelben Suspension tropfte man eine Lösung von 25 g 6'-Chloro-3'4'-dihydro-spiro[cyclopentan-1,2'(1'H)-naphthalen]-1'-on (0.107 Mol) in 250 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab und engte ein. Der Rückstand wurde in 1 l heissem Hexan suspendiert, auf 0°C abgekühlt und abfiltriert (der Filterkuchen besteht aus Triphenylphosphinoxid). Das Produkt wurde über Kieselgel mit Hexan chromatographiert. Ausbeute: 22.7 g (92%) 6'-Chloro-1'-methylen-3'4'-dihydro-spiro[cyclopentan-1,2'(1'H)-naphthalen] als gelbliche Flüssigkeit.

MS: m/e (% Basis peak) = 232 ($C_{15}H_{17}Cl^+$, 14), 191 (100), 175 (11), 153 (9), 141 (10), 128 (12), 115 (14).

**11.3** Zu einer Lösung von 22.6 g 6'-Chloro-1'-methylen-3'4'-dihydrospiro[cyclopentan-1,2'(1'H)-naphthalen] (0.0971 Mol) in 230 ml Ether bei -78°C unter Argonbegasung tropfte man eine Lösung von 10.2 ml Chlorsulfonylisocyanat (0.117 Mol) in 100 ml Ether zu. Man rührte 15 min bei -78°C und liess innerhalb 1 St zur RT erwärmen. Das entstandene Kristallisat wurde abgenutscht, mit Ether gewaschen und i. Vak. getrocknet. Ausbeute: 14.2 g (39%) rac-cis-7-Chloro-1,2,4,5-tetrahydro-3a,9b-butano-naphtho[2,1-b]furan-2-yliden-sulfamoylchlorid [(E) oder (Z) oder (E/Z)-Gemisch] als weisse Kristalle; Schmp.123-125°C.

**11.4.** 7,2 g (0,189 Mol) Lithiumaluminiumhydrid wurden in 190 ml Dioxan unter Argon suspendiert. Man tropfte eine Lösung aus 14.1 g rac-cis-7-Chloro-1,2,4,5-tetrahydro-3a,9b-butano-naphtho[2,1-b]furan-2-yliden-sulfamoylchlorid [(E) oder (Z) oder (E/Z)-Gemisch] (0.0376 Mol) in 280 ml THF zu und kochte 20 St unter Rückfluss. Vorsichtig tropfte man nacheinander bei ~40°C 7 ml Wasser, 14 ml einer 15 prozentigen wässrigen NaOH Lösung, und 7 ml Wasser zu, so dass ein völlig weisser Niederschlag entstand. Nach Filtrieren und Einengen wurde der Rückstand über Kieselgel mit Methanol/Ammoniak chromatographiert. Das Produkt kristallisierte aus heissem Acetonitril. Ausbeute: 3.6 g (34%) rac-cis-4b-(2-Amino-ethyl)-2-chloro-4b,5,6,7,8,8a,9,10-octahydro-phenanthren-8a-ol als weisse Kristalle; Schmp. 119-120°C.

**11.5** rac-cis-4b-(2-Amino-ethyl)-2-chloro-4b,5,6,7,8,8a,9,10-octahydrophenanthren-8a-ol (3.6 g, 0.0129 Mol) wurde in 70 ml Ethanol gelöst. Man tropfte eine 5 N ethanolische HCl-Lösung (10 ml, 0.05 Mol) zu und engte ein. Der Rückstand kristallisierte aus Isopropylalkohol/Ether. Ausbeute: 3.9 g (96%) rac-cis-4b-(2-Amino-ethyl)-2-chloro-4b, 5,6,7,8,8a,9,10-octahydro-phenanthren-8a-ol hydrochlorid als weisse Kristalle; Schmp. 248-250°C.

**Beispiel 12**

**12.1.** Zu einer Lösung von 17.2 g 8-Chloro-1,2,3,4-tetrahydro-naphthalen-1-on (0.0952 Mol) in 170 ml THF unter Argonbegasung gab man portionsweise bei -78°C 18.2 g Kalium tert-Butylat (0.162 Mol) zu. Man rührte 2 St bei -78°C nach und tropfte dann eine Lösung von 22.5 ml 1,4-Dibrombutan (41.1 g, 0.190 Mol) in 120 ml THF zu. Das Kühlbad wurde entfernt und man rührte 62 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Ether 4:1. Das Produkt kritallisierte aus Hexan bei -78°C. Ausbeute: 6.6 g (30%) 8'-Chloro-3'4'-dihydro-spiro[cyclopentan-1,2'(1'H)-naphthalen]-1'-on als weisse Kristalle; Schmp. 26-27°C.

**12.2.** 52.3 g Methyltriphenylphosphoniumbromid/Natriumamid Gemisch (0.126 Mol) wurden während $^3/_4$ St in 500 ml THF bei RT unter Argon gerührt.

Zu der gelben Suspension tropfte man eine Lösung von 13.4 g 8'-Chloro-3'4'-dihydro-spiro[cyclopentan-1,2'(1'H)-naphthalen]-1'-on (0.057 Mol) in 130 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab und engte ein. Der Rückstand wurde in 300 ml heissem Hexan suspendiert, auf 0°C abgekühlt und abfiltriert (der Filterkuchen besteht aus Triphenylphosphinoxid). Das Produkt wurde eingeengt und über Kieselgel mit Hexan chromatographiert. Ausbeute: 9.4 g (71%) 8'-Chloro-1'-methylen-3'4'-dihydro-spiro[cyclopentan-1,2'(1'H)-naphthalen] als farblose Flüssigkeit.

MS: m/e (% Basis peak) = 232 ($C_{15}H_{17}Cl^+$, 32), 217 (5), 191 (100), 175 (17), 164 (29), 141 (16), 129 (21), 115 (26).

**12.3.** Zu einer Lösung von 9.4 g 8'-Chloro-1'-methylen-3'4'-dihydrospiro[cyclopentan-1,2'(1'H)-naphthalen] (0.0404 Mol) in 100 ml Ether bei -78°C unter Argonbegasung tropfte man eine Lösung von 4.2 ml Chlorsulfonylisocyanat (0.0481 Mol) in 50 ml Ether zu. Man rührte 15 min bei -78°C, liess innerhalb 1 St zur RT erwärmen und engte ein. Ein Nebenprodukt kristallisierte aus Ether, das abgenutscht wurde. Aus der Mutterlauge kristallisierte dann das Produkt in Ether. Ausbeute: 2.80 g (19%) rac-cis-9-Chloro-1,2,4,5-tetrahydro-3a,9b-butano-naphtho[2,1-b]furan-2-yliden-sulfamoylchlorid [(E) oder (Z) oder (E/Z)-Gemisch] als weisse Kristalle; Schmp. 110-112°C.

**12.4.** 1,42 g (0,0374 Mol) Lithiumaluminiumhydrid wurden in 40 ml Dioxan unter Argon suspendiert. Man tropfte eine Lösung aus 2.8 g rac-cis-9-Chloro-1,2,4,5-tetrahydro-3a,9b-butano-naphtho[2,1-b]furan-2-yliden-sulfamoylchlorid [(E) oder (Z) oder (E/Z)-Gemisch] (0.0748 Mol) in 30 ml THF zu und kochte 22 St unter Rückfluss. Vorsichtig tropfte

man nacheinander bei ~40°C 1 ml Wasser, 2 ml einer 15 prozentigen wässrigen NaOH Lösung, und 4 ml Wasser zu, so dass ein völlig weisser Niederschlag entstand. Nach Filtrieren und Einengen wurde der Rückstand über Kieselgel mit Methanol/Ammoniak 9:1 chromatographiert. Das Produkt wurde in Ether suspendiert, abgenutscht, mit Ether gewaschen und i. Vak. getrocknet. Ausbeute: 1.40 g (67%) rac-cis-4b-(2-Amino-ethyl)-4-chloro-4b,5,6,7,8,8a,9,10-octahydro-phenanthren-8a-ol als weisse Kristalle; Schmp. 112-116°C.

**12.5.** rac-cis-4b-(2-Amino-ethyl)-4-chloro-4b,5,6,7,8,8a,9,10-octahydrophenanthren-8a-ol (1.35 g, 0.00482 Mol) wurde in 40 ml Ethanol gelöst. Man tropfte eine 5 N ethanolische HCl-Lösung (1.0 ml, 0.005 Mol) zu und engte ein. Der Rückstand kristallisierte aus Acetonitril. Ausbeute: 1.25 g (82%) rac-cis-4b-(2-Amino-ethyl)-4-chloro-4b,5,6,7,8,8a, 9,10-octahydro-phenanthren-8a-ol hydrochlorid als weisse Kristalle; Schmp. 248-250°C.

**Beispiel 13**

**13.1.** Zu einer Lösung von 35.2 g 7-Chloro-5-fluoro-1,2,3,4-tetrahydronaphthalen-1-on (0.177 Mol) in 350 ml THF unter Argonbegasung gab man portionsweise bei -78°C 33.8 g Kalium tert-Butylat (0.301 Mol) zu. Man rührte 2 St bei -78°C nach und tropfte dann eine Lösung von 41.9 ml 1,4-Dibrombutan (76.5 g, 0.354 Mol) in 200 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Toluol 7:3. Ausbeute: 18.4 g (42%) 7'-Chloro-5'-fluoro-3'4'-dihydro-spiro[cyclopentan-1,2'(1'H)-naphthalen]-1'-on als gelbliche Flüssigkeit .

MS: m/e (% Basis peak) = 252 ($C_{14}H_{14}ClFO^+$, 19), 211 (100), 183 (14), 170 (15), 142 (22), 107 (19), 81 (16).

**13.2.** 36.5 g Methyltriphenylphosphoniumbromid/Natriumamid Gemisch (0.0875 Mol) wurden während $^3/_4$ St in 400 ml THF bei RT unter Argon gerührt. Zu der gelben Suspension tropfte man eine Lösung von 18.4 g 7'-Chloro-5'-fluoro-3'4'-dihydro-spiro[cyclopentan-1,2'(1'H)-naphthalen]-1'-on (0.0729 Mol) in 180 ml THF zu und rührte dann $^3/_4$ St nach. Man fügte Wasser zu, schüttelte mit Ether aus, wusch die organische Phase mit einer gesättigten wässrigen Kochsalzlösung, trocknete über $MgSO_4$, filtrierte ab und engte ein. Der Rückstand wurde in 300 ml heissem Hexan suspendiert, auf 0°C abgekühlt und abfiltriert (der Filterkuchen besteht aus Triphenylphosphinoxid). Das Produkt wurde eingeengt und über Kieselgel mit Hexan chromatographiert. Ausbeute: 17.4 g (95%) 7'-Chloro-5'-fluoro-1'-methylen-3'4'-dihydro-spiro[cyclopentan-1,2'(1'H)-naphthalen] als farblose Flüssigkeit.

MS: m/e (% Basis peak) = 250 ($C_{15}H_{16}ClF^+$, 20), 209 (100), 193 (20), 174 (10), 159 (13), 146(16),133(15). **13.3.** Zu einer Lösung von 5.0 g 7'-Chloro-5'-fluoro-1'-methylen-3'4'-dihydro-spiro[cyclopentan-1,2'(1'H)-naphthalen] (0.0199 Mol) in 50 ml Ether bei -78°C unter Argonbegasung tropfte man eine Lösung von 1.9 ml Chlorsulfonylisocyanat (0.0219 Mol) in 20 ml Ether zu. Man rührte 15 min bei -78°C und liess innerhalb 1 St zur RT erwärmen. Das Gemisch wurde eingeengt und das Produkt kristallisierte aus Ether. Ausbeute: 2.85 g (37%) rac-cis-8-Chloro-6-fluoro-1,2,4,5-tetrahydro-3a,9b-butano-naphtho[2,1-b]furan-2-yliden-sulfamoylchlorid [(E) oder (Z) oder (E/Z)-Gemisch] als weissliche Kristalle; Schmp. 195-200°C.

**13.4.** 1,3 g ( 0,0347 Mol) Lithiumaluminiumhydrid wurden in 35 ml Dioxan unter Argon suspendiert. Man tropfte eine Lösung aus 2.7 g rac-cis-8-Chloro-6-fluoro-1,2,4,5-tetrahydro-3a,9b-butano-naphtho[2,1-b]furan-2-yliden-sulfamoylchlorid [(E) oder (Z) oder (E/Z)-Gemisch] (0.0069 Mol) in 100 ml THF zu und kochte 16 St unter Rückfluss. Vorsichtig tropfte man nacheinander bei -40°C 1.3 ml Wasser, 2.6 ml einer 15 prozentigen wässrigen NaOH Lösung, und 3.9 ml Wasser zu, so dass ein völlig weisser Niederschlag entstand. Nach Filtrieren und Einengen wurde der Rückstand über Kieselgel mit Methanol/Ammoniak 49:1 chromatographiert. Das Produkt kristallisierte aus Acetonitril. Ausbeute: 0.6 g (29%) rac-cis-4b-(2-Amino-ethyl)-3-chloro-1-fluoro-4b,5,6,7,8,8a,9,10-octahydro-phenanthren-8a-ol als weisse Kristalle; Schmp. 158-162°C.

**13.5.** rac-cis-4b-(2-Amino-ethyl)-3-chloro-1-fluoro-4b,5,6,7,8,8a,9,10-octahydrophenanthren-8a-ol (3.7 g, 0.0124 Mol) wurde in 90 ml Ethanol gelöst. Man tropfte eine 5 N ethanolische HCl-Lösung (2.95 ml, 0.0149 Mol) zu und engte ein. Der Rückstand kristallisierte aus Ethanol. Ausbeute: 3.83 g (92%) rac-cis-4b-(2-Amino-ethyl)-3-chloro-1-fluoro-4b,5,6,7,8,8a,9,10-octahydro-phenanthren-8a-ol hydrochlorid als weisse Kristalle; Schmp. 316-317°C.

**Beispiel 14**

**14.1.** Zu einer Lösung von 18 g 5,7-Dichloro-1,2,3,4-tetrahydro-naphthalen-1-on (0.0837 Mol) in 180 ml THF unter Argonbegasung gab man portionsweise bei -78°C 16 g Kalium tert-Butylat (0.142 Mol) zu. Man rührte 2 St bei -78°C nach und tropfte dann eine Lösung von 19.8 ml 1,4-Dibrombutan (36.1 g, 0.167 Mol) in 100 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Essigester 9:1. Ausbeute: 7.9 g (35%) 5',7'-Dichloro-3'4'-dihydro-spiro[cyclopentan-1,2'(1'H)-naphthalen]-1'-on als gelbliche Flüssigkeit.

MS: m/e (% Basis peak) = 268 ($C_{14}H_{14}Cl_2O^+$, 24), 227 (100), 186 (19), 158 (24), 123 (29), 81 (20).

**14.2.** 41.5 g Methyltriphenylphosphoniumbromid/Natriumamid-Gemisch (0.0997 Mol) wurden während $^{3}/_{4}$ St in 410 ml THF bei RT unter Argon gerührt. Zu der gelben Suspension tropfte man eine Lösung von 12.2 g 5',7'-Dichloro-3'4'-dihydro-spiro[cyclopentan-1,2'(1'H)-naphthalen]-1'-on (0.0453 Mol) in 60 ml THF zu und rührte dann 62 St nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan. Ausbeute: 9.75 g (81%) 5',7'-Dichloro-1'-methylen-3'4'-dihydro-spiro[cyclclopentan-1,2'(1'H)-naphthalen] als farblose Flüssigkeit.

MS: m/e (% Basis peak) = 266 ($C_{15}H_{16}Cl_2^+$,18), 225 (100), 209 (12), 190 (13), 175 (8), 163 (10), 152 (10)

**14.3.** Zu einer Lösung von 14.9 g 5',7'-Dichloro-1'-methylen-3'4'-dihydrospiro[cyclopentan-1,2'(1'H)-naphthalen] (0.0556 Mol) in 150 ml Ether bei -78°C unter Argonbegasung tropfte man eine Lösung von 5.84 ml Chlorsulfonylisocyanat (0.0669 Mol) in 20 ml Ether zu. Man rührte 15 min bei -78°C und liess innerhalb 1 St zur RT erwärmen. Das entstandene Kristallisat (Nebenprodukt) wurde abgenutscht und die Mutterlauge in Ether kristallisiert. Ausbeute: 4.82 g (21%) rac-cis-6,8-Dichloro-1,2,4,5-tetrahydro-3a,9b-butano-naphtho[2,1-b]furan-2-yliden-sulfamoylchlorid [(E) oder (Z) oder (E/Z)-Gemisch] als gelbliche Kristalle; Schmp. 180-184°C.

**14.4.** 2,06 g (0,0543 Mol) Lithiumaluminiumhydrid wurden in 50 ml Dioxan unter Argon suspendiert. Man tropfte eine Lösung aus 4.48 g rac-cis-6,8-Dichloro-1,2,4,5-tetrahydro-3a,9b-butano-naphtho[2,1-b]furan-2-yliden-sulfamoylchlorid [(E) oder (Z) oder (E/Z)-Gemisch] (0.011 Mol) in 50 ml THF zu und kochte 17 St unter Rückfluss. Vorsichtig tropfte man nacheinander bei ~40°C 2.1 ml Wasser, 2.1 ml einer 15 prozentigen wässrigen NaOH Lösung, und 6.3 ml Wasser zu, so dass ein völlig weisser Niederschlag entstand. Nach Filtrieren und Einengen wurde der Rückstand über Kieselgel mit Methanol/Ammoniak (99:1) chromatographiert. Das Produkt wurde in Ether suspendiert, abgenutscht, mit Ether gewaschen und i. Vak. getrocknet. Ausbeute: 1.08 g (31%) rac-cis-4b-(2-Amino-ethyl)-1,3-dichloro-4b,5,6,7,8,8a,9,10-octahydro-phenanthren-8a-ol als weisse Kristalle; Schmp. 169-171°C.

**14.5.** rac-cis-4b-(2-Amino-ethyl)-1,3-dichloro-4b,5,6,7,8,8a,9,10-octahydro-phenanthren-8a-ol (1.11 g, 0.00353 Mol) wurde in 30 ml Methanol gelöst. Man tropfte eine 4.89 N ethanolische HCl-Lösung (0.72 ml, 0.00353 Mol) zu und engte ein. Der Rückstand kristallisierte aus Methanol/Ether. Ausbeute: 1.09 g (88%) rac-cis-4b-(2-Amino-ethyl)-1,3-dichloro-4b,5,6,7,8,8a,9,10-octahydro-phenanthren-8a-ol hydrochlorid als weisse Kristalle; Schmp. 303-305°C.

**Beispiel 15**

**15.1.** Zu einer Lösung von 27.9 g 6,7-Dichloro-1,2,3,4-tetrahydro-naphthalen-1-on (0.13 Mol) in 280 ml THF unter Argonbegasung gab man portionsweise bei -78°C 24.7 g Kalium tert-Butylat (0.22 Mol) zu. Man rührte 2 St bei -78°C nach und tropfte dann eine Lösung von 30.7 ml 1,4-Dibrombutan (56 g, 0.26 Mol) in 150 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Toluol 3:2. Ausbeute: 17.8 g (51%) 6',7'-Dichloro-3'4'-dihydro-spiro[cyclopentan-1,2'(1'H)-naphthalen]-1'-on als gelbliche Flüssigkeit.

MS: m/e (% Basis peak) = 268 ($C_{14}H_{14}Cl_2O^+$, 18), 227 (100), 186 (15), 158 (12), 123 (11), 81 (11).

**15.2.** 5.0 g Methyltriphenylphosphoniumbromid/Natriumamid-Gemisch (0.012 Mol) wurden während $^{3}/_{4}$ St in 50 ml THF bei RT unter Argon gerührt. Zu der gelben Suspension tropfte man eine Lösung von 2.7 g 6',7'-Dichloro-3'4'-dihydro-spiro[cyclopentan-1,2'(1'H)-naphthalen]-1'-on (0.010 Mol) in 30 ml THF zu und rührte dann $1^{1}/_{2}$ St nach. Man fügte Wasser zu, schüttelte mit Ether aus, wusch die organische Phase mit einer gesättigten wässrigen Kochsalzlösung, trocknete über $MgSO_4$, filtrierte ab und engte ein. Der Rückstand wurde über Kieselgel mit Hexan/$CH_2Cl_2$ chromatographiert. Ausbeute: 2.4 g (90%) 6'7'-Dichloro-1'-methylen-3'4'-dihydro-spiro[cyclopentan-1,2'(1'H)-naphthalen] als gelbliche Flüssigkeit.

MS: m/e (% Basis peak) = 266 ($C_{15}H_{16}Cl_2^+$, 15), 225 (100), 209 (9), 190 (9), 175 (6), 162 (7), 152 (8), 139 (6).

**15.3** Zu einer Lösung von 2.3 g 6'7'-Dichloro-1'-methylen-3'4'-dihydro-spiro[cyclopentan-1,2'(1'H)-naphthalen] (0.0088 Mol) in 25 ml Ether bei -78°C unter Argonbegasung tropfte man eine Lösung von 0.92 ml Chlorsulfonylisocyanat (0.0206 Mol) in 10 ml Ether zu. Man rührte 15 min bei -78°C und liess innerhalb 1 St zur RT erwärmen. Das Gemisch wurde eingeengt und das Produkt kristallisierte aus Ether. Ausbeute: 1.65 g (46%) rac-cis-7,8-Dichloro-1,2,4,5-tetrahydro-3a,9b-butano-naphtho[2,1-b]furan-2-yliden-sulfamoylchlorid [(E) oder (Z) oder (E/Z)-Gemisch] als weissliche Kristalle; Schmp. 188-190°C.

**15.4.** 0,74 g (0,0196 Mol) Lithiumaluminiumhydrid wurden in 20 ml Dioxan unter Argon suspendiert. Man tropfte eine Lösung aus 1.6 g rac-cis-7,8-Dichloro-1,2,4,5-tetrahydro-3a,9b-butano-naphtho[2,1-b]furan-2-yliden-sulfamoylchlorid [(E) oder (Z) oder (E/Z)-Gemisch] (0.00391 Mol) in 16 ml THF zu und kochte 22 St unter Rückfluss. Vorsichtig tropfte man nacheinander bei ~40°C 0.8 ml Wasser, 1.6 ml einer 15 prozentigen wässrigen NaOH Lösung, und 1.6 ml Wasser zu, so dass ein völlig weisser Niederschlag entstand. Nach Filtrieren und Einengen wurde der Rückstand über Kieselgel mit Methanol/Ammoniak 49:1 chromatographiert. Das Produkt wurde in Ether suspendiert, abgenutscht, mit Ether gewaschen und i. Vak. getrocknet. Ausbeute: 0.64 g (52%) rac-cis-4b-(2-Amino-ethyl)-2,3-dichloro-4b,5,6,7,8,8a,9,10-octahydro-phenanthren-8a-ol als weisse Kristalle; Schmp. 189-191°C.

**15.5.** rac-cis-2b-(2-Amino-ethyl)-2,3-dichloro-4b,5,6,7,8,8a,9,10-octahydro-phenanthren-8a-ol (2.4 g, 0.00763 Mol) wurde in 75 ml Ethanol gelöst. Man tropfte eine 5 N ethanolische HCl-Lösung (2 ml, 0.010 Mol) zu und engte ein. Der Rückstand kristallisierte aus Ethanol/Ether. Ausbeute: 2.3 g (86%) rac-cis-4b-(2-Amino-ethyl)-2,3-dichloro-4b, 5,6,7,8,8a,9,10-octahydro-phenanthren-8a-ol hydrochlorid als weisse Kristalle; Schmp. 290-295°C.

**Beispiel 16**

**16.1.** Man löste 2.5 g rac-cis-4b-(2-Aminoethyl)-3-benzyloxy-4b,5,6,7,8,8a,9,10-octahydro-phenanthren-8a-ol (0.0071 Mol) in 50 ml THF und tropfte bei 25°C unter Argon eine Lösung von 5.07 ml Essigsäurechlorid (0.071 Mol) in 10 ml THF zu. Das Gemisch wurde 1 St. unter Rückfluss gekocht, dann auf Wasser gegossen und mit Ether ausgeschüttelt. Man trocknete die organische Phase mit $MgSO_4$, filtrierte ab, engte ein und chromatographierte den Rückstand über Kieselgel mit Essigester. Das Produkt wurde aus Ether umkristallisiert. Ausbeute: 1.56 g (56%) rac-cis-N-[2-(6-Benzyloxy-10a-hydroxy-1,2,3,4,4a,9,10,10a-octahydro-phenanthren-4a-yl)-ethyl]-acetamid als weisse Kristalle; Schmp. 178-179°C.

**16.2.** 0.29 g (0,0077 Mol) Lithiumaluminiumhydrid wurden in 30 ml THF unter Argon suspendiert. Man tropfte eine Lösung aus 1.5 g rac-cis-N-[2-(6-Benzyloxy-10a-hydroxy-1,2,3,4,4a,9,10,10a-octahydro-phenanthren-4a-yl)-ethyl]-acetamid (0.0038 Mol) in 30 ml THF zu und kochte 3 St. unter Rückfluss. Vorsichtig tropfte man bei 5°C 0.9 ml eines 2:1 Gemisches NaOH 28%/Wasser zu, so dass ein völlig weisser Niederschlag entstand. Nach Filtrieren und Einengen wurde der Rückstand über Kieselgel mit Acetonitril chromatographiert. Das Produkt kristallisierte aus Ether. Ausbeute: 0.98 g (68%) rac-cis-3-Benzyloxy-4b-(2-ethylamino-ethyl)-4b,5,6,7,8,8a,9,10-octahydro-phenanthren-8a-ol als weisse Kristalle; Schmp.108-110°C.

**16.3.** Man löste 1.63 g rac-cis-3-Benzyloxy-4b-(2-ethylamino-ethyl)-4b,5,6,7,8,8a,9,10-octahydro-phenanthren-8a-ol (0.0043 Mol) in 80 ml THF, fügte 0.64 ml (0.0046 Mol) Triethylamin zu, und tropfte bei 25°C unter Argon eine Lösung von 0.32 ml Essigsäurechlorid (0.0045 Mol) in 5 ml THF zu. Das Gemisch wurde $1^1/_2$ St. unter Rückfluss gekocht, dann auf Wasser gegossen und mit Ether ausgeschüttelt. Man trocknete die organische Phase mit $MgSO_4$, filtrierte ab, engte ein und chromatographierte den Rückstand über Kieselgel mit THF. Ausbeute: 2.09 g (100%) rac-cis-N-[2-(6-Benzyloxy-10a-hydroxy-1,2,3,4,4a,9,10,10a-octahydro-phenanthren-4a-yl)ethyl]-N-ethyl-acetamid als gelbliches Öl.

MS: m/e (% Basis peak) = 421 ($C_{27}H_{35}NO_3^+$, 0.6), 312 (2.4), 290 (6.6), 225 (5.6), 199 (3.6), 115 (85), 100 (29), 91 (100), 58 (56).

**16.4.** 0,73 g (0,019 Mol) Lithiumaluminiumhydrid wurden in 50 ml THF unter Argon suspendiert. Man tropfte eine Lösung aus 2.0 g rac-cis-N-[2-(6-Benzyloxy-10a-hydroxy-1,2,3,4,4a,9,10,10a-octahydro-phenanthren-4a-yl)ethyl]-N-ethyl-acetamid (0.0047 Mol) in 20 ml THF zu und kochte $1^1/_2$ St. unter Rückfluss. Vorsichtig tropfte man nacheinander bei RT 20 ml Essigester, 0.7 ml Wasser, 0.7 ml einer 15 prozentigen wässrigen NaOH Lösung, und 2.1 ml Wasser zu, so dass ein völlig weisser Niederschlag entstand. Nach Filtrieren und Einengen wurde der Rückstand über Kieselgel mit THF chromatographiert. Ausbeute: 1.56 g ( 81 %) rac-cis-3-Benzyloxy-4b-(2-diethylaminoethyl)-4b,5,6,7,8,8a, 9,10-octahydro-phenanthren-8a-ol als gelbliches Öl.

MS: m/e (% Basis peak) = 407 ($C_{27}H_{37}NO_2^+$, 0.42), 145 (33), 86 (77), 57 (100).

**16.5.** Man löste 1.51 g rac-cis-3-Benzyloxy-4b-(2-diethylamino-ethyl)-4b,5,6,7,8,8a,9,10-octahydro-phenanthren-8a-ol (0.0037 Mol) in 150 ml Ethanol. Nach Zugabe von 0.15 g 10 prozentigem Pd-C Katalysator wurde das Gemisch während 1 St bei RT unter 1 Atm hydriert. Man filtrierte, engte ein und chromatographierte über Kieselgel mit Methanol. Das Produkt kristallisierte aus Ether. Ausbeute: 0.91 g (78%) rac-cis-4b-(2-Diethylamino-ethyl)-4b,5,6,7,8,8a,9,10-octahydro-phenanthren-3,8a-diol als weisse Kristalle; Schmp. 197-199°C.

**16.6.** rac-cis-4b-(2-Diethylamino-ethyl)-4b,5,6,7,8,8a,9,10-octahydro-phenanthren-3,8a-diol (0.96 g, 0.003 Mol) wurde in 25 ml Methanol gelöst. Man tropfte eine 4.89 N ethanolische HCl-Lösung (0.61 ml, 0.003 Mol) zu und engte ein. Der Rückstand kristallisierte aus Acetonitril. Ausbeute: 0.98 g (92%) rac-cis-4b-(2-Diethylamino-ethyl)-4b, 5,6,7,8,8a,9,10-octahydro-phenanthren-3,8a-diol hydrochlorid als weisse Kristalle; Schmp. 155-157°C.

**Beispiel 17**

**17.1.** Man löste 1.22 g rac-cis-3-Benzyloxy-4b-(2-ethylamino-ethyl)-4b,5,6,7,8,8a,9,10-octahydro-phenanthren-8a-ol (0.003 Mol) in 100 ml Methanol. Nach Zugabe von 0.35 g 10 prozentigem Pd-C Katalysator wurde das Gemisch während 2 St bei RT unter 1 Atm hydriert. Man filtrierte, engte ein und chromatographierte über Kieselgel mit Ethanol. Nach Einengen erhielt man einen weissen Festkörper. Ausbeute: 0.80 g (86%) rac-cis-4b-(2-Ethylaminoethyl)4b, 5,6,7,8,8a,9,10-octahydro-phenanthren-3,8a-diol; Schmp. 189-191°C.

**17.2.** rac-cis-4b-(2-Ethylamino-ethyl)-4b,5,6,7,8,8a,9,10-octahydro-phenanthren-3,8a-diol (0.8 g, 0.0027 Mol) wurde in 40 ml Methanol gelöst. Man tropfte eine 4.89 N ethanolische HCl-Lösung (0.56 ml, 0.0027 Mol) zu und engte

ein. Der Rückstand kristallisierte aus Ethanol/Ether. Ausbeute: 0.76 g (84%) rac-cis-4b-(2-Ethylamino-ethyl)-4b, 5,6,7,8,8a,9,10-octahydro-phenanthren-3,8a-diol hydrochlorid als weisse Kristalle; Schmp. 219-221°C.

**Beispiel 18**

**18.1.** Man löste 2.5 g rac-cis-4b-(2-Aminoethyl)-3-benzyloxy-4b,5,6,7,8,8a,9,10-octahydro-phenanthren-8a-ol (0.0071 Mol) in 50 ml THF und tropfte bei 25°C unter Argon eine Lösung von 1.24 ml Propionsäurechlorid (0.014 Mol) in 5 ml THF zu. Das Gemisch wurde 1 St. unter Rückfluss gekocht. Man engte ein und chromatographierte den Rückstand über Kieselgel mit Essigester. Das Produkt wurde aus Ether umkristallisiert. Ausbeute: 1.75 g (60%) rac-cis-N-[2-(6-Benzyloxy-10a-hydroxy-1,2,3,4,4a,9,10,10a-octahydro-phenanthren-4a-yl)-ethyl]-propionamid als weisse Kristalle; Schmp. 154-155°C.

**18.2.** 0,18 g ( 0,0047 Mol) Lithiumaluminiumhydrid wurden in 30 ml THF unter Argon suspendiert. Man tropfte eine Lösung aus 0.5 g rac-cis-N-[2-(6-Benzyloxy-10a-hydroxy-1,2,3,4,4a,9,10,10a-octahydrophenanthren-4a-yl)-ethyl]-propionamid (0.0012 Mol) in 10 ml THF zu und kochte 2 St. unter Rückfluss. Vorsichtig tropfte man bei 25°C 0.9 ml eines 2:1 Gemisches NaOH 28%/Wasser zu, so dass ein völlig weisser Niederschlag entstand. Nach Filtrieren und Einengen wurde der Rückstand über Kieselgel mit Ethanol chromatographiert. Das erhaltene Festkörper wurde 15 St. in Hexan kräftig gerührt, abgenutscht und i. Vak. getrocknet. Ausbeute: 0.36 g (81%) rac-cis-3-Benzyloxy-4b-(2-propylamino-ethyl)-4b,5,6,7,8,8a,9,10-octahydro-phenanthren-8a-ol als weisse Kristalle; Schmp. 99-101°C.

**18.3.** Man löste 1.14 g rac-cis-3-Benzyloxy-4b-(2-propylamino-ethyl)-4b,5,6,7,8,8a,9,10-octahydro-phenanthren-8a-ol (0.0029 Mol) in 100 ml Methanol. Nach Zugabe von 0.25 g 10 prozentigem Pd-C Katalysator wurde das Gemisch während 1 St bei RT unter 1 Atm hydriert. Man filtrierte, engte ein und chromatographierte über Kieselgel mit Ethanol. Das Produkt kristallisierte aus Ether. Ausbeute: 0.66 g (75%) rac-cis-4b-(2-Propylamino-ethyl)-4b,5,6,7,8,8a,9,10-octahydro-phenanthren-3,8a-diol als weisse Kristalle; Schmp. 185-187°C.

**18.4.** rac-cis-4b-(2-Propylamino-ethyl)-4b,5,6,7,8,8a,9,10-octahydro-phenanthren-3,8a-diol (0.66 g, 0.0021 Mol) wurde in 30 ml Methanol gelöst. Man tropfte eine 4.89 N ethanolische HCl-Lösung (0.44 ml, 0.0021 Mol) zu und engte ein. Der Rückstand kristallisierte aus Acetonitril. Ausbeute: 0.58 g (78%) rac-cis-4b-(2-Propylamino-ethyl)-4b, 5,6,7,8,8a,9,10-octahydro-phenanthren-3,8a-diol hydrochlorid als weisse Kristalle; Schmp. 182-184°C.

**Beispiel 19**

**19.1.** Man löste 2.5 g rac-cis-4b-(2-Aminoethyl)-3-benzyloxy-4b,5,6,7,8,8a,9,10-octahydro-phenanthren-8a-ol (0.0071 Mol) in 50 ml THF und tropfte bei 25°C unter Argon eine Lösung von 1.3 ml Cyclopropancarbonsäurechlorid (0.0142 Mol) in 5 ml THF zu. Das Gemisch wurde 1 St. unter Rückfluss gekocht, dann auf Wasser gegossen und mit Ether ausgeschüttelt. Man trocknete die organische Phase mit $MgSO_4$, filtrierte ab, engte ein und chromatographierte den Rückstand über Kieselgel mit Essigester. Nach Einengen erhielt man einen weissen Festkörper. Ausbeute: 2.0 g (67%) rac-cis-N-[2-(6-Benzyloxy-10a-hydroxy-1,2,3,4,4a,9,10,10a-octahydro-phenanthren-4a-yl)-ethyl]-cyclopropancarboxamid; Schmp. 176-178°C.

**19.2.** 0.67 g ( 0.0178 Mol) Lithiumaluminiumhydrid wurden in 120 ml THF unter Argon suspendiert. Man tropfte eine Lösung aus 1.87 g rac-cis-N-[2-(6-Benzyloxy-10a-hydroxy-1,2,3,4,4a,9,10,10a-octahydro-phenanthren-4a-yl)-ethyl]-cyclopropancarboxamid (0.0044 Mol) in 30 ml THF zu und kochte $1^1/_2$ St. unter Rückfluss. Vorsichtig tropfte man bei RT 3.6 ml eines 2:1 Gemisches NaOH 28% /Wasser zu, so dass ein völlig weisser Niederschlag entstand. Nach Filtrieren und Einengen wurde der Rückstand über Kieselgel mit Ethanol chromatographiert. Das Produkt kristallisierte aus heissem Hexan. Ausbeute: 1.85 g (100%) rac-cis-3-Benzyloxy-4b-(2-cyclopropylmethylamino-ethyl)-4b,5,6,7,8,8a,9,10-octahydro-phenanthren-8a-ol als gelbliches Öl.

MS: m/e (% Basis peak) = 405 ($C_{27}H_{35}NO_2^+$, 6.2), 349 (1.3), 296 (6.1) 290 (6.6), 217 (17), 199 (13), 98 (22), 91 (100), 84 (65), 55 (38).

**19.3.** Man löste 1.73 g rac-cis-3-Benzyloxy-4b-(2-cyclopropylmethylaminoethyl)4b,5,6,7,8,8a,9,10-octahydro-phenanthren-8a-ol (0.0042 Mol) in 40 ml Methanol. Nach Zugabe von 0.46 g 10 prozentigem Pd-C Katalysator wurde das Gemisch während 1 St bei RT unter 1 Atm hydriert. Man filtrierte, engte ein und chromatographierte über Kieselgel mit Ethanol. Das Produkt kristallisierte aus Ether. Ausbeute: 0.59 g (44%) rac-cis-4b-(2-Cyclopropylmethylamino-ethyl)-4b,5,6,7,8,8a,9,10-octahydro-phenanthren-3,8a-diol als weisse Kristalle; Schmp. 167-169°C.

**19.4.** rac-cis-4b-(2-Cyclopropylmethylamino-ethyl)-4b,5,6,7,8,8a,9,10-octahydro-phenanthren-3,8a-diol (1 g, 0.003 Mol) wurde in 40 ml Methanol gelöst. Man tropfte eine 4.89 N ethanolische HCl-Lösung (0.65 ml, 0.003 Mol) zu und engte ein. Der Rückstand kristallisierte aus Ether. Ausbeute: 1.02 g (91%) rac-cis-4b-(2-Cyclopropylmethylamino-ethyl)-4b,5,6,7,8,8a,9,10-ooctahydro-phenanthren-3,8a-diol hydrochlorid als weisse Kristalle; Schmp. 207-209°C.

**Beispiel 20**

**20.1.** Man löste 3.0 g rac-cis-4b-(2-Aminoethyl)-3-benzyloxy-4b,5,6,7,8,8a,9,10-octahydro-phenanthren-8a-ol (0.0085 Mol) in 50 ml THF, fügte 1.3 ml (0.0094 Mol) Triethylamin zu, und tropfte bei 25°C unter Argon eine Lösung von 1.13 g Cyclobutancarbonsäurechlorid (0.0094 Mol) in 5 ml THF zu. Das Gemisch wurde 1 St. unter Rückfluss gekocht, dann auf Wasser gegossen und mit $CH_2Cl_2$ ausgeschüttelt. Man trocknete die organische Phase mit $MgSO_4$, filtrierte ab, engte ein und chromatographierte den Rückstand über Kieselgel mit Essigester. Man erhielt ein kristallines Material, das in Ether suspendiert, dann abgenutscht und i. Vak. getrocknet wurde. Ausbeute: 3.15 g (85%) rac-cis-N-[2-[6-Benzyloxy-10a-hydroxy-1,2,3,4,4a,9,10,10a-octahydro-phenanthren-4a-yl)-ethyl]-cyclobutancarboxamid als weisse Kristalle; Schmp. 111-113°C.

**20.2** 1,08 g (0,0286 Mol) Lithiumaluminiumhydrid wurden in 120 ml THF unter Argon suspendiert. Man tropfte eine Lösung aus 3.1 g rac-cis-N-[2-[6-Benzyloxy-10a-hydroxy-1,2,3,4,4a,9,10,10a-octahydro-phenanthren-4a-yl]-ethyl]-cyclobutancarboxamid (0.0071 Mol) in 20 ml THF zu und kochte $1^1/_2$ St. unter Rückfluss. Vorsichtig tropfte man bei 10°C 5.9 ml eines 2:1 Gemisches NaOH 48% Wasser zu, so dass ein völlig weisser Niederschlag entstand. Nach Filtrieren und Einengen wurde der Rückstand über Kieselgel mit Acetonitril chromatographiert. Das Produkt wurde im Kugelrohr destilliert. Ausbeute: 3.16 g (100%) rac-cis-3-Benzyloxy-4b-(2-cyclobutylmethylamino-ethyl)-4b,5,6,7,8,8a,9,10-octahydro-phenanthren-8a-ol als gelbliches Öl; Sdp. 250°C/0.04 Torr.

MS: m/e (% Basis peak) = 419 ($C_{28}H_{37}NO_2^+$, 7.4), 363 (1.9), 346 (6.3), 308 (6.5), 290 (9), 217 (23), 199 (15), 157 (5.5), 112 (24), 98 (56), 91 (100).

**20.3.** Man löste 3.1 g rac-cis-3-Benzyloxy-4b-(2-cyclobutylmethylamino-ethyl)-4b,5,6,7,8,8a,9,10-octahydro-phenanthren-8a-ol (0.0074 Mol) in 100 ml Ethanol. Nach Zugabe von 0.4 g 10 prozentigem Pd-C Katalysator wurde das Gemisch während 1 St bei RT unter 1 Atm hydriert. Man filtrierte, engte ein und chromatographierte über Kieselgel mit Ethanol. Das Produkt kristallisierte aus Ether. Ausbeute: 1.56 g (69%) rac-cis-4b-(2-Cyclobutylmethylamino-ethyl)-4b,5,6,7,8,8a,9,10-octahydro-phenanthren-3,8a-diol als weisse Kristalle; Schmp. 140-142°C.

**20.4.** rac-cis-4b-(2-Cyclobutylmethylamino-ethyl)-4b,5,6,7,8,8a,9,10-octahydro-phenanthren-3,8a-diol (1.52 g, 0.0046 Mol) wurde in 50 ml Methanol gelöst. Man tropfte eine 4.89 N ethanolische HCl-Lösung (0.94 ml, 0.0046 Mol) zu und engte ein. Der Rückstand kristallisierte aus Ethanol/Ether. Ausbeute: 1.62 g (96%) rac-cis-4b-(2-Cyclobutylmethylamino-ethyl)-4b,5,6,7,8,8a,9,10-octahydro-phenanthren-3,8a-diol hydrochlorid als weisse Kristalle; Schmp. 210-212°C.

**Beispiel 21**

**21.1** Man löste 2.0 g rac-cis-4b-(2-Aminoethyl)-3-benzyloxy-4b,5,6,7,8,8a,9,10-octahydro-phenanthren-8a-ol (0.0057 Mol) in 50 ml THF und tropfte bei 25°C unter Argon eine Lösung von 1.5 ml Phenylessigsäurechlorid (0.0113 Mol) in 5 ml THF zu. Das Gemisch wurde $1^1/_2$ St. unter Rückfluss gekocht. Man engte ein und chromatographierte den Rückstand über Kieselgel mit $CH_2Cl_2$/Essigester 1:1. Das Produkt wurde in Ether suspendiert, abfiltriert,und i. Vak. getrocknet. Ausbeute: 1.5 g (56%) rac-cis-N-[2-(6-Benzyloxy-10a-hydroxy-1,2,3,4,4a,9,10,10a-octahydro-phenanthren-4a-yl)-ethyl]-phenylacetamid als weisse Kristalle; Schmp. 114-116°C.

**21.2.** 0,5 g ( 0,013 Mol) Lithiumaluminiumhydrid wurden in 40 ml THF unter Argon suspendiert. Man tropfte eine Lösung aus 1.55 g rac-cis-N-[2-(6-Benzyloxy-10a-hydroxy-1,2,3,4,4a,9,10,10a-octahydro-phenanthren-4a-yl)-ethyl]-phenylacetamid (0.0033 Mol) in 15 ml THF zu und kochte $1^1/_2$ St. unter Rückfluss. Vorsichtig tropfte man bei RT 1.8 ml eines 2:1 Gemisches NaOH 28%/Wasser zu, so dass ein völlig weisser Niederschlag entstand. Nach Filtrieren und Einengen wurde der Rückstand über Kieselgel mit Ethanol chromatographiert. Ausbeute: 0.66 g (44%) rac-cis-3-Benzyloxy-4b-(2-phenethylamino-ethyl)-4b,5,6,7,8,8a,9,10-octahydro-phenanthren-8a-ol als gelbliches Öl.

MS: m/e (% Basis peak) = 445 ($C_{31}H_{37}NO_2^+$, 1.75), 364 (26), 346 (48), 289 (11), 134 (13), 105 (24), 91 (100), 58 (19), 44 (20).

**21.3.** Man löste 0.66 g rac-cis-3-Benzyloxy-4b-(2-phenethylamino-ethyl)-4b,5,6,7,8,8a,9,10-octahydro-phenanthren-8a-ol (0.0014 Mol) in 60 ml Ethanol.

Nach Zugabe von 0.16 g 10 prozentigem Pd-C Katalysator wurde das Gemisch während $1^1/_2$ St bei RT unter 1 Atm hydriert. Man filtrierte, engte ein und chromatographierte über Kieselgel mit Ethanol. Das Produkt kristallisierte aus Hexan. Ausbeute: 0.29 g (56%) rac-cis-4b-(2-Phenethylaminoethyl)-4b,5,6,7,8,8a,9,10-octahydro-phenanthren-3,8a-diol als weisse Kristalle ; Schmp. 82-84°C.

**21.4.** rac-cis-4b-(2-Phenethylamino-ethyl)-4b,5,6,7,8,8a,9,10-octahydro-phenanthren-3,8a-diol (0.15 g, 0.0004 Mol) wurde in 10 ml Methanol gelöst. Man tropfte eine 4.89 N ethanolische HCl-Lösung (0.084 ml, 0.0004 Mol) zu und engte ein. Der Rückstand kristallisierte aus Ether. Ausbeute: 0.16 g (100%) rac-cis-4b-(2-Phenethylamino-ethyl)-4b, 5,6,7,8,8a,9,10-octahydro-phenanthren-3,8a-diol hydrochlorid als weisse Kristalle; Schmp. 160-162°C.

**Beispiel 22**

**22.1.** Man löste 5.5 g rac-cis-4b-(2-Aminoethyl)-3-methoxy-4b,5,6,7,8,8a,9,10-octahydro-phenanthren-8a-ol (0.020 Mol) in 40 ml $CH_2Cl_2$, fügte 3.35 ml Triethylamin (0.024 Mol) zu, und tropfte bei 0°C unter Argon 1.7 ml Essigsäurechlorid (0.024 Mol) zu. Das Gemisch wurde 1 St. unter Rückfluss gekocht, dann auf Wasser gegossen und mit Ether ausgeschüttelt. Man trocknete die organische Phase mit $MgSO_4$, filtrierte ab, engte ein und chromatographierte den Rückstand über Kieselgel mit $CH_2Cl_2$/Methanol 10:1. Das Produkt wurde aus Ether umkristallisiert. Ausbeute: 2.7 g (43%) rac-cis-N-[2-(10a-Hydroxy-6-methoxy-1,2,3,4,4a,9,10,10a-octahydro-phenanthren-4a-yl)-ethyl]-acetamid als weisse Kristalle.

MS: m/e (% Basis peak) = 317 ($C_{19}H_{27}NO_3^+$, 2.4), 299 (3.4), 240 (8), 213 (68), 171 (15), 121 (18), 87 (100).

**22.2.** 3.16 g (0.084 Mol) Lithiumaluminiumhydrid wurde in 60 ml Dioxan unter Argon suspendiert. Man tropfte eine Lösung aus 5.3 g rac-cis-N-[2-(10a-Hydroxy-6-methoxy-1,2,3,4,4a,9,10,10a-octahydro-phenanthren-4a-yl)-ethyl]-acetamid (0.017 Mol) in 60 ml THF zu und kochte 20 St. unter Rückfluss. Vorsichtig tropfte man nacheinander bei RT 3.2 ml Wasser, 3.5 ml einer 15 prozentigen wässrigen NaOH Lösung, und 3.2 ml Wasser zu, so dass ein völlig weisser Niederschlag entstand. Nach Filtrieren und Einengen wurde der Rückstand über Kieselgel mit $CH_2Cl_2$/Methanol 10:1 chromatographiert. Das Produkt kristallisierte aus heissem Hexan. Ausbeute: 5.0 g (98%) rac-cis-4b-(2-Ethylamino-ethyl)-3-methoxy-4b,5,6,7,8,8a,9,10-octahydro-phenanthren-8a-ol als weisse Kristalle.

MS: m/e (% Basis peak) = 303 ($C_{19}H_{29}NO_2^+$, 13), 232 (39), 214 (16), 171 (6), 121 (5), 58 (100).

**22.3.** rac-cis-4b-(2-Ethylamino-ethyl)-3-methoxy-4b,5,6,7,8,8a,9,10-octahydro-phenanthren-8a-ol (5.0 g, 0.016 Mol) wurde in 50 ml Ether gelöst. Man leitete HCl-Gas ein und engte ein. Der Rückstand kristallisierte aus Ether. Ausbeute: 5.0 g (88%) rac-cis-4b-(2-Ethylamino-ethyl)-3-methoxy-4b,5,6,7,8,8a,9,10-octahydro-phenanthren-8a-ol hydrochlorid als weisse Kristalle; Schmp. 217-219°C.

**Beispiel 23**

**23.1.** Man löste 8.2 g rac-cis-4b-(2-Aminoethyl)-3-methoxy-4b,5,6,7,8,8a,9,10-octahydro-phenanthren-8a-ol (0.0298 Mol) in 60 ml THF, fügte 5 ml Triethylamin (0.0357 Mol) zu, und tropfte bei 25°C unter Argon 3.1 ml Cyclopropancarbonsäurechlorid (0.0357 Mol) zu. Das Gemisch wurde 1 St. unter Rückfluss gekocht, dann auf Wasser gegossen, mit Ether ausgeschüttelt und mit einer 1N wässriger HCl Lösung gewaschen. Man trocknete die organische Phase mit $MgSO_4$, filtrierte ab und engte ein. Ausbeute: 10 g (97%) rac-cis-N-[2-(10a-Hydroxy-6-methoxy-1,2,3,4,4a,9,10,10a-octahydro-phenanthren-4a-yl)-ethyl]-cyclopropancarboxamid als rohes Material.

MS: m/e (% Basis peak) = 343 ($C_{21}H_{29}NO_3^+$, 2.6), 325 (4.3), 255 (16), 240 (14), 213 (100),171 (41), 121 (31), 113 (100), 98 (76), 69 (79).

**23.2.** 1,1 g (0,029 Mol) Lithiumaluminiumhydrid wurden in 20 ml THF unter Argon suspendiert. Man tropfte eine Lösung aus 2.0 g rac-cis-N-[2-(10a-Hydroxy-6-methoxy-1,2,3,4,4a,9,10,10a-octahydro-phenanthren-4a-yl)-ethyl]-cyclopropancarboxamid (0.006 Mol) in 20 ml THF zu und kochte 20 St. unter Rückfluss. Vorsichtig tropfte man nacheinander bei RT 1.1 ml Wasser, 1.5 ml einer 15 prozentigen wässrigen NaOH Lösung, und 1.1 ml Wasser zu, so dass ein völlig weisser Niederschlag entstand. Nach Filtrieren und Einengen wurde der Rückstand über Kieselgel mit Ethanol chromatographiert. Das Produkt kristallisierte aus heissem Hexan. Ausbeute: 1.4 g (73%) rac-cis-4b-(2-Cyclopropylmethylamino-ethyl)-3-methoxy-4b,5,6,7,8,8a,9,10-octahydro-phenanthren-8a-ol als weisse Kristalle.

MS: m/e (% Basis peak) = 329 ($C_{21}H_{31}NO_2^+$, 24), 232 (65), 214 (37), 171 (12), 121 (13), 99 (17), 84 (100), 55 (58).

**23.3.** rac-cis-4b-(2-Cyclopropylmethylamino-ethyl)-3-methoxy-4b,5,6,7,8,8a,9,10-octahydro-phenanthren-8a-ol (6.4 g, 0.019 Mol) wurde in 70 ml Ether gelöst. Man leitete HCl-Gas ein und engte ein. Der Rückstand kristallisierte aus $CH_2Cl_2$/Ether. Ausbeute: 4.6 g (54%) rac-cis-4b-(2-Cyclopropylmethylaminoethyl)-3-methoxy-4b,5,6,7,8,8a,9,10-octahydro-phenanthren-8a-ol hydrochlorid als weisse Kristalle; Schmp. 180-182°C.

**Beispiel 24**

**24.1.** Man löste 4.0 g rac-cis-4b-(2-Aminoethyl)-3-methoxy-4b,5,6,7,8,8a,9,10-octahydro-phenanthren-8a-ol (0.0145 Mol) in 30 ml $CH_2Cl_2$, fügte 2.4 ml Triethylamin (0.0174 Mol) zu, und tropfte bei 0°C unter Argon 1.8 ml Cyclobutancarbonsäurechlorid (0.0174 Mol) zu. Das Gemisch wurde 20 St. bei RT gerührt, dann auf Wasser gegossen, mit Ether ausgeschüttelt und mit einer 1N wässriger HCl Lösung gewaschen. Man trocknete die organische Phase mit $MgSO_4$, filtrierte ab, engte ein und chromatographierte den Rückstand über Kieselgel mit $CH_2Cl_2$/Methanol 10:1. Ausbeute: 3.7 g (70%) rac-cis-N-[2-(10a-Hydroxy-6-methoxy-1,2,3,4,4a,9,10,10a-octahydro-phenanthren-4a-yl)-ethyl]-cyclobutancarboxamid als Öl.

MS: m/e (% Basis peak) = 357 ($C_{22}H_{31}NO_3^+$, 4.9), 339 (2.6), 240 (14), 214 (30), 171 (11), 159 (9), 127 (100), 72 (48), 56 (61).

**24.2** 3,45 g (0,091 Mol) Lithiumaluminiumhydrid wurden in 70 ml Dioxan unter Argon suspendiert. Man tropfte eine

Lösung aus 6.5 g rac-cis-N-[2-(10a-Hydroxy-6-methoxy-1,2,3,4,4a,9,10,10a-octahydro-phenanthren-4a-yl)-ethyl]-cyclobutancarboxamid (0.018 Mol) in 80 ml THF zu und kochte 20 St. unter Rückfluss. Vorsichtig tropfte man nacheinander bei RT 3.5 ml Wasser, 4.5 ml einer 15 prozentigen wässrigen NaOH Lösung, und 3.5 ml Wasser zu, so dass ein völlig weisser Niederschlag entstand. Man filtriete und engte ein. Ausbeute: 6.2 g (98%) rac-cis-4b-(2-Cyclobutylmethylamino-ethyl)-3-methoxy-4b,5,6,7,8,8a,9,10-octahydro-phenanthren-8a-ol als rohes farbloses Öl.

MS: m/e (% Basis peak) = 343 ($C_{22}H_{33}NO_2^+$, 33), 270 (17), 232 (81), 214 (40), 171 (16), 159 (12), 147 (9), 121 (17), 98 (100).

**24.3.** rac-cis-4b-(2-Cyclobutylmethylamino-ethyl)-3-methoxy-4b,5,6,7,8,8a,9,10-octahydro-phenanthren-8a-ol (6.2 g, 0.018 Mol) wurde in 100 ml Ether gelöst. Man leitete HCl-Gas ein und engte ein. Der Rückstand kristallisierte aus $CH_2Cl_2$/Ether. Ausbeute: 5.7 g (83%) rac-cis-4b-(2-Cyclobutylmethylaminoethyl)-3-methoxy-4b,5,6,7,8,8a,9,10-octahydro-phenanthren-8a-ol hydrochlorid als weisse Kristalle; Schmp. 156-158°C.

**Beispiel 25**

**25.1.** 2.2 g rac-cis-4b-(2-Aminoethyl)-4b,5,6,7,8,8a,9,10-octahydro-phenanthren-8a-ol (0.009 Mol) wurden in 10 ml einer 85 prozentigen wässrigen Ameisensäurelösung gelöst. Man gab 3 ml einer 37 prozentigen wässrigen Formaldehydlösung zu und kochte das Gemisch 16 St unter Rückfluss. Man kühlte auf RT ab, goss auf 50 ml Wasser, stellte mit 50 ml 4N NaOH basisch, schüttelte mit $CH_2Cl_2$ aus, wusch die organische Phase mit einer gesättigten wässrigen Kochsalzlösung, trocknete über $MgSO_4$, filtrierte ab, engte ein und chromatographierte über Kieselgel mit Essigester/ Methanol 1:4. Ausbeute: 1.23 g (50 %) rac-cis-4b-(2-Dimethylamino-ethyl)-4b,5,6,7,8,8a,9,10-octahydro-phenanthren-8a-ol als gelbliches zähes Öl.

MS: m/e (% Basis peak) = 273 ($C_{18}H_{27}NO^+$, 5.0), 217 (2.3), 184 (2.3), 129 (2.9), 128 (2.9), 91 (2.3), 72 (4), 58 (100).

**25.2.** rac-cis-4b-(2-Dimethylamino-ethyl)-4b,5,6,7,8,8a,9,10-octahydro-phenanthren-8a-ol (1.2 g, 0.00439 Mol) wurde in 30 ml Essigester gelöst. Man tropfte eine 4.8 N ethanolische HCl-Lösung (1.05 ml, 4.83 Mol) zu, wobei das Produkt auskristallisierte. Ausbeute: 1.17 g (86%) rac-cis-4b-(2-Dimethylaminoethyl)-4b,5,6,7,8,8a,9,10-octahydro-phenanthren-8a-ol hydrochlorid als weisse Kristalle; Schmp. 161-164°C.

**Beispiel 26**

**26.1.** 10 g rac-cis-4b-(2-Aminoethyl)-3-fluoro-4b,5,6,7,8,8a,9,10-octahydro-phenanthren-8a-ol (0.038 Mol) wurden in 28 ml einer 85 prozentigen wässrigen Ameisensäurelösung gelöst. Man gab 5.6 ml einer 37 prozentigen wässrigen Formaldehydlösung zu und kochte das Gemisch 16 St unter Rückfluss. Man kühlte auf RT ab, goss auf 50 ml Wasser, stellte mit 2N NaOH basisch, schüttelte mit $CH_2Cl_2$ aus, wusch die organische Phase mit einer gesättigten wässrigen Kochsalzlösung, trocknete über $MgSO_4$, filtrierte ab, engte ein und chromatographierte über Kieselgel mit $CH_2Cl_2$/Methanol/Ammoniak 90:9:1. Ausbeute: 2.34 g (49%) rac-cis-4b-(2-Dimethylamino-ethyl)-3-fluoro-4b,5,6,7,8,8a,9,10-octahydro-phenanthren-8a-ol als gelbliches Öl.

MS: m/e (% Basis peak) = 291 ($C_{18}H_{26}FNO^+$, 2.3), 235 (0.4), 159 (1.9), 146 (1.4), 133 (1.55), 72 (4), 58 (100).

**26.2.** rac-cis-4b-(2-Dimethylamino-ethyl)-3-fluoro-4b,5,6,7,8,8a,9,10-octahydro-phenanthren-8a-ol (2.81 g, 0.0096 Mol) wurde in 50 ml Ethanol gelöst. Man tropfte eine 4.89 N ethanolische HCl-Lösung (1.96 ml, 0.0096 Mol) zu und engte ein. Der Rückstand kristallisierte aus Ether. Ausbeute: 2.73 g (86%) rac-cis-4b-(2-Dimethylamino-ethyl)-3-fluoro-4b,5,6,7,8,8a,9,10-octahydro-phenanthren-8a-ol hydrochlorid als weisse Kristalle; Schmp. 194-196°C.

**Beispiel 27**

**27.1.** 6.9 g rac-cis-4b-(2-Aminoethyl)-3-chloro-4b,5,6,7,8,8a,9,10-octahydro-phenanthren-8a-ol (0.024 Mol) wurden in 18.1 ml einer 85 prozentigen wässrigen Ameisensäurelösung gelöst. Man gab 3.63 ml einer 37 prozentigen wässrigen Formaldehydlösung zu und kochte das Gemisch 16 St unter Rückfluss. Man kühlte auf RT ab, goss auf 50 ml Wasser, stellte mit 2N NaOH basisch, schüttelte mit $CH_2Cl_2$ aus, wusch die organische Phase mit einer gesättigten wässrigen Kochsalzlösung, trocknete über $MgSO_4$, filtrierte ab, engte ein und chromatographierte über Kieselgel mit $CH_2Cl_2$/Methanol 10:1. Das Produkt wurde im Kugelrohr destilliert. Ausbeute: 4.41 g (58%) rac-cis-3-Chloro-4b-(2-dimethylamino-ethyl)-4b,5,6,7,8,8a,9,10-octahydro-phenanthren-8a-ol als farbloses Öl (58%); Sdp. 220-230°C/0.01 Torr.

MS: m/e (% Basis peak) = 307 ($C_{18}H_{26}ClNO^+$, 2.3), 251 (0.75), 128 (1.1), 115 (1), 72 (4.5), 58 (100).

**27.2.** rac-cis-3-Chloro-4b-(2-dimethylamino-ethyl)-4b,5,6,7,8,8a,9,10-octahydro-phenanthren-8a-ol (3.56 g, 0.011 Mol) wurde in 60 ml Methanol gelöst. Man tropfte eine 4.89 N ethanolische HCl-Lösung (2.36 ml, 0.011 Mol) zu und engte ein. Der Rückstand kristallisierte aus Ethanol/Ether. Ausbeute: 3.43 g (86%) rac-cis-3-Chloro-4b-(2-dimethylamino-ethyl)-4b,5,6,7,8,8a,9,10-octahydro-phenanthren-8a-ol hydrochlorid als weisse Kristalle; Schmp. 240-242°C.

**Beispiel 28**

**28.1.** Man löste 2.0 g rac-cis-4b-(2-Amino-ethyl)-3-benzyloxy-4b,5,6,7,8,8a,9,10-octahydro-phenanthren-8a-ol (0.0057 Mol) in 40 ml Ethanol. Nach Zugabe von 0.4 g 10 prozentigem Pd-C Katalysator wurde das Gemisch während 1 St bei RT unter 1 Atm hydriert. Man filtrierte und engte ein. Das Produkt kristallisierte aus Acetonitril. Ausbeute: 1.31 g (88,5%) rac-cis-4b-(2-Amino-ethyl)-4b,5,6,7,8,8a,9,10-octahydro-phenanthren-3,8a-diol als weisse Kristalle; Schmp. 141-143°C (Zers.).

**28.2.** rac-cis-4b-(2-Amino-ethyl)-4b,5,6,7,8,8a,9,10-octahydro-phenanthren-3,8a-diol (1.76 g, 0.0067 Mol) wurde in 30 ml THF gelöst. Man tropfte eine 4.89 N ethanolische HCl-Lösung (1.38 ml, 0.0067 Mol) zu und engte ein. Der Rückstand kristallisierte aus THF/Ether. Ausbeute: 1.74 g (87%) rac-cis-4b-(2-Amino-ethyl)-4b,5,6,7,8,8a,9,10-octahydro-phenanthren-3,8a-diol hydrochlorid als weisse Kristalle; Schmp. 130-132°C.

**Beispiel 29**

**29.1.** Ein Gemisch von 1.39 g rac-cis-4b-(2-Amino-ethyl)-4b,5,6,7,8,8a,9,10-octahydro-phenanthren-3,8a-diol (0.00531 Mol), 1.22 ml 2-Brompyridin (1.98 g, 0.0125 Mol), 1.05 g Kaliumcarbonat (0.0076 Mol) und 125 mg Kupferpulver (0.00197 Mol) wurde 48 Stunden in 100 ml Pyridin unter Rückfluss gekocht. Nach Einengen chromatographierte man über Kieselgel mit Methanol/Ammoniak 19:1. Das Produkt wurde in Acetonitril umkristallisiert. Ausbeute: 0.77 g (43%) rac-cis-4b-(2-Amino-ethyl)-3-(pyridin-2-yloxy)-4b,5,6,7,8,8a,9,10-octahydro-phenanthren-8a-ol als beige Kristalle; Schmp. 157-159°C

**29.2.** rac-cis-4b-(2-Amino-ethyl)-3-(pyridin-2-yloxy)-4b,5,6,7,8,8a,9,10-octahydro-phenanthren-8a-ol (1.09 g, 0.00322 Mol) wurde in 20 ml Methanol gelöst. Man tropfte eine 4.89 N ethanolische HCl-Lösung (0.66 ml, 0.00323 Mol) zu und engte ein. Der Rückstand kristallisierte aus Ethanol/Ether. Ausbeute: 1.04 g (86%) rac-cis-4b-(2-Amino-ethyl)-3-(pyridin-2-yloxy)-4b,5,6,7,8,8a,9,10-octahydro-phenanthren-8a-ol hydrochlorid als weisse Kristalle; Schmp. 170-172°C.

**Beispiel 30**

**30.1.** 1.8 g rac-cis-4b-(2-Amino-ethyl)-3-(pyridin-2-yloxy)-4b,5,6,7,8,8a,9,10-octahydro-phenanthren-8a-ol (0.00532 Mol) wurden in 6 ml einer 85 prozentigen wässrigen Ameisensäurelösung gelöst. Man gab 1.8 ml einer 37 prozentigen wässrigen Formaldehydlösung zu und kochte das Gemisch $2^1/_2$ St unter Rückfluss. Man kühlte auf RT ab, goss auf 50 ml Wasser, stellte mit 27 ml 4N NaOH basisch, schüttelte mit $CH_2Cl_2$ aus, wusch die organische Phase mit einer gesättigten wässrigen Kochsalzlösung, trocknete über $MgSO_4$, filtrierte ab, engte ein und chromatographierte über Kieselgel mit Methanol. Ausbeute: 1.6 g (82%) rac-cis-4b-(2-Dimethylamino-ethyl)-3-(pyridin-2-yloxy)-4b,5,6,7,8,8a,9,10-octahydro-phenanthren-8a-ol als gelbliches Öl.

MS: m/e (% Basis peak) = 366 ($C_{23}H_{30}N_2O_2^+$, 3.9), 295 (3.7), 278 (7.2), 141 (1.0), 128 (1.7), 115 (2.2), 72 (6.5), 58 (100).

**30.2.** rac-cis-4b-(2-Dimethylamino-ethyl)-3-(pyridin-2-yloxy)-4b,5,6,7,8,8a,9,10-octahydro-phenanthren-8a-ol (1.6 g, 0.00437 Mol) wurde in 30 ml Essigester gelöst. Man tropfte eine 4.78 N ethanolische HCl-Lösung (1.0 ml, 0.00478 Mol) zu, wobei das Produkt auskristallisierte. Ausbeute: 1.62 g (92%) rac-cis-4b-(2-Dimethylamino-ethyl)-3-(pyridin-2-yloxy)-4b,5,6,7,8,8a,9,10-octahydro-phenanthren-8a-ol hydrochlorid als weisse Kristalle; Schmp. 209-210°C.

**Beispiel 31**

**31.1.** 1 g rac-cis-4b-(2-Aminoethyl)-3-benzyloxy-4b,5,6,7,8,8a,9,10-octahydro-phenanthren-8a-ol (0.00284 Mol) wurden in 2 ml einer 85 prozentigen wässrigen Ameisensäurelösung gelöst. Man gab 0.4 ml einer 37 prozentigen wässrigen Formaldehydlösung zu und kochte das Gemisch 4 St unter Rückfluss. Man kühlte auf RT ab, goss auf 20 ml Wasser, stellte mit 2N NaOH basisch, schüttelte mit Ether aus, wusch die organische Phase mit Wasser, trocknete über $Na_2SO_4$, filtrierte ab, engte ein,und chromatographierte über Kieselgel mit Methanol und Methanol/Ammoniak 9: 1. Ausbeute: 0.74 g (69%) rac-cis-3-Benzyloxy-4b-(2-dimethylamino-ethyl)-4b,5,6,7,8,8a,9,10-octahydro-phenanthren-8a-ol als hellgelbes Öl.

MS: m/e (% Basis peak) = 379 ($C_{25}H_{33}NO_2^+$, 7.4), 308 (5.8), 288 (4.5), 217 (9), 91 (25), 58 (100)

**31.2.** Man löste 2.75 g rac-cis-3-Benzyloxy-4b-(2-dimethylamino-ethyl)-4b,5,6,7,8,8a,9,10-octahydro-phenanthren-8a-ol (0.0072 Mol) in 80 ml THF, fügte 2.2 ml Triethylamin (0.015 Mol) zu, und tropfte bei 25°C unter Argon eine Lösung von 1.1 ml Essigsäurechlorid (0.015 Mol) in 5 ml THF zu. Das Gemisch wurde 2 St. unter Rückfluss gekocht, dann auf Wasser gegossen und mit $CH_2Cl_2$ ausgeschüttelt. Man trocknete die organische Phase mit $MgSO_4$, filtrierte ab, engte ein und chromatographierte den Rückstand über Kieselgel mit Methanol. Ausbeute: 2 g (66%) Essigsäure

rac-cis-3-benzyloxy-4b-(2-dimethylamino-ethyl)-4b,5,6,7,8,8a,9,10-octahydro-phenanthren-8a-yl ester als gelbliches Öl.

MS: m/e (% Basis peak) = 421 ($C_{27}H_{35}NO_3^+$, 0.9), 362 (1.4), 290 (3), 270 (2.8), 199 (7.1), 91 (26), 58 (100).

**31.3.** Man löste 2.34 g Essigsäure rac-cis-3-benzyloxy-4b-(2-dimethylamino-ethyl)-4b,5,6,7,8,8a,9,10-octahydro-phenanthren-8a-yl ester (0.0055 Mol) in 20 ml Methanol. Nach Zugabe von 0.23 g 10 prozentigem Pd-C Katalysator wurde das Gemisch während 2 St bei RT unter 1 Atm hydriert. Man filtrierte, engte ein und chromatographierte über Kieselgel mit Methanol/Ammoniak 19:1. Das Produkt kristallisierte aus Ether. Ausbeute: 1.84 g (90%) Essigsäure rac-cis-4b-(2-dimethylamino-ethyl)-3-hydroxy-4b,5,6,7,8,8a,9,10-octahydro-phenanthren-8a-yl ester als weisse Kristalle; Schmp. 229-230°C.

**31.4.** Essigsäure rac-cis-4b-(2-dimethylamino-ethyl)-3-hydroxy-4b,5,6,7,8,8a,9,10-octahydro-phenanthren-8a-yl ester (0.95 g, 0.0029 Mol) wurde in 50 ml Methanol gelöst. Man tropfte eine 4.89 N ethanolische HCl-Lösung (0.6 ml, 0.0029 Mol) zu und engt ein. Der Rückstand kristallisierte aus Ether. Ausbeute: 0.97 g (91%) Essigsäure rac-cis-4b-(2-dimethylamino-ethyl)-3-hydroxy-4b,5,6,7,8,8a,9,10-octahydro-phenanthren-8a-yl ester hydrochlorid als weisse Kristalle; Schmp. 222-224°C.

**Beispiel 32**

**32.1.** Man löste 0.6 g rac-cis-3-Benzyloxy-4b-(2-dimethylamino-ethyl)-4b,5,6,7,8,8a,9,10-octahydro-phenanthren-8a-ol (0.00158 Mol) in 20 ml Methanol. Nach Zugabe von 0.1 g 10 prozentigem Pd-C Katalysator wurde das Gemisch während 1 St bei RT unter 1 Atm hydriert. Man filtrierte, engte ein und chromatographierte über Kieselgel mit Methanol . Das Produkt kristallisierte aus Ether. Ausbeute: 0.33 g (72%) rac-cis-4b-(2-Dimethylaminoethyl)-4b,5,6,7,8,8a,9,10-octahydro-phenanthren-3,8a-diol als weisse Kristalle; Schmp. 189-190°C.

**32.2.** rac-cis-4b-(2-Dimethylamino-ethyl)-4b,5,6,7,8,8a,9,10-octahydro-phenanthren-3,8a-diol (2.2 g, 0.0076 Mol) wurde in 100 ml Methanol gelöst. Man tropfte eine 4.89 N ethanolische HCl-Lösung (1.55 ml, 0.076 Mol) zu und engte ein. Der Rückstand kristallisierte aus Methanol/Ether. Ausbeute: 2.4 g (97%) rac-cis-4b-(2-Dimethylamino-ethyl)-4b, 5,6,7,8,8a,9,10-octahydro-phenanthren-3,8a-diol als weisse Kristalle; Schmp. 222-224°C°C.

**Beispiel 33**

**33.1.** Man löste 1.4 g rac-cis-3-Benzyloxy-4b-(2-dimethylamino-ethyl)-4b,5,6,7,8,8a,9,10-octahydro-phenanthren-8a-ol (0.0037 Mol) in 20 ml THF, fügte 1.11 ml Triethylamin (0.0079 Mol) zu, und tropfte bei 25°C unter Argon eine Lösung von 0.86 ml Buttersäurechlorid (0.0082 Mol) in 5 ml THF zu. Das Gemisch wurde 3 St. unter Rückfluss gekocht, dann auf Wasser gegossen und mit $CH_2Cl_2$ ausgeschüttelt. Man trocknete die organische Phase mit $MgSO_4$, filtrierte ab und engte ein. Ausbeute: 1.59 g (96%) Buttersäure rac-cis-3-benzyloxy-4b-(2-dimethylamino-ethyl)-4b,5,6,7,8,8a, 9,10-octahydro-phenanthren-8a-yl ester als braunes Öl.

MS: m/e (% Basis peak) = 449 ($C_{20}H_{39}NO_3^+$, 1.2), 362 (3.1), 334 (1.5), 290 (6.6), 270 (4.9), 199 (9.5), 149 (3), 141 (4.5), 91 (39), 73 (13), 58 (100).

**33.2.** Man löste 1.59 g Buttersäure rac-cis-3-Benzyloxy-4b-(2-dimethylaminoethyl)-4b,5,6,7,8,8a,9,10-octahydro-phenanthren-8a-yl ester (0.0035 Mol) in 20 ml Methanol. Nach Zugabe von 0.16 g 10 prozentigem Pd-C Katalysator wurde das Gemisch während 1.5 St bei RT unter 1 Atm hydriert. Man filtrierte, engte ein und chromatographierte über Kieselgel mit MeOH. Das Produkt kristallisierte aus Ether. Ausbeute: 0.87 g (70%) Buttersäure rac-cis-4b-(2-dimethylaminoethyl)-3-hydroxy-4b,5,6,7,8,8a,9,10-octahydro-phenanthren-8a-yl ester als weisse Kristalle; Schmp. 158-160°C.

**33.3.** Buttersäure rac-cis-4b-(2-dimethylamino-ethyl)-3-hydroxy-4b,5,6,7,8,8a,9,10-octahydro-phenanthren-8a-yl ester (0.9 g, 0.0025 Mol) wurde in 30 ml Methanol gelöst. Man tropfte eine 4.89 N ethanolische HCl-Lösung (0.51 ml, 0.0025 Mol) zu und engte ein. Der Rückstand kristallisierte aus Acetonitril/Ether. Ausbeute: 0.92 g (95%) Buttersäure rac-cis-4b-(2-dimethylamino-ethyl)-3-hydroxy-4b,5,6,7,8,8a,9,10-octahydro-phenanthren-8a-yl ester hydrochlorid als weisse Kristalle; Schmp. 210-212°C.

**Beispiel 34**

**34.1.** 2.0 g rac-cis-4b-(2-Aminoethyl)-3-methoxy-4b,5,6,7,8,8a,9,10-octahydro-phenanthren-8a-ol (0.0072 Mol) wurden in 5.1 ml einer 85 prozentigen wässrigen Ameisensäurelösung gelöst. Man gab 1 ml einer 37 prozentigen wässrigen Formaldehydlösung zu und kochte das Gemisch 16 St unter Rückfluss. Man kühlte auf RT ab, goss auf 5 ml Wasser, stellte mit 8 ml 2N NaOH basisch, schüttelte mit Essigester aus, wusch die organische Phase mit einer gesättigten wässrigen Kochsalzlösung, trocknete über $MgSO_4$, filtrierte ab, engte ein und chromatographierte über Kieselgel mit Essigester/Methanol/Ammoniak 90:9:1. Ausbeute: 0.99 g (45%) rac-cis-4b-(2-Dimethylamino-ethyl)-3-methoxy-4b,5,6,7,8,8a,9,10-octahydro-phenanthren-8a-ol als gelbliches Öl.

MS: m/e (% Basis peak) = 303 ($C_{19}H_{29}NO_2^+$, 4), 232 (9), 214 (3.5), 58 (100).

**34.2.** rac-cis-4b-(2-Dimethylamino-ethyl)-3-methoxy-4b,5,6,7,8,8a,9,10-octahydro-phenanthren-8a-ol (0.99 g, 0.0032 Mol) wurde in 10 ml Ethanol gelöst. Man tropfte eine 4.89 N ethanolische HCl-Lösung (0.67 ml, 0.0032 Mol) zu und engte ein. Der Rückstand kristallisierte aus Acetonitril/Ether. Ausbeute: 0.9 g (82%) rac-cis-4b-(2-Dimethylamino-ethyl)-3-methoxy-4b,5,6,7,8,8a,9,10-octahydro-phenanthren-8a-ol hydrochlorid als weisse Kristalle; Schmp. 159-161°C.

**Beispiel 35**

**35.1.** 4.5 g rac-cis-4b-(2-Aminoethyl)-2,3-dichloro-4b,5,6,7,8,8a,9,10-octahydro-phenanthren-8a-ol (0.0143 Mol) wurden in 16 ml einer 85 prozentigen wässrigen Ameisensäurelösung gelöst. Man gab 3.0 ml einer 37 prozentigen wässrigen Formaldehydlösung zu und kochte das Gemisch 19 St unter Rückfluss. Man kühlte auf RT ab, goss auf 100 ml Wasser, stellte mit 120 ml 4N NaOH basisch, schüttelte mit $CH_2Cl_2$ aus, wusch die organische Phase mit einer gesättigten wässrigen Kochsalzlösung, trocknete über $MgSO_4$, filtrierte ab, engte ein und chromatographierte über Kieselgel mit Isopropylalkohol/Essigester 3:1. Ausbeute: 1.5 g (31%) rac-cis-2,3-Dichloro-4b-(2-dimethylamino-ethyl)-4b,5,6,7,8,8a,9,10-octahydro-phenanthren-8a-ol als gelbliches Öl.

MS: m/e (% Basis peak) = 341 ($C_{18}H_{25}Cl_2NO^+$, 1.0), 117 (5.3), 72 (4), 58 (100).

**35.2.** rac-cis-2,3-Dichloro-4b-(2-dimethylamino-ethyl)-4b,5,6,7,8,8a,9,10-octahydro-phenanthren-8a-ol (1.5 g, 0.00438 Mol) wurde in 30 ml Ethanol gelöst. Man tropfte eine 5 N ethanolische HCl-Lösung (1.0 ml, 0.005 Mol) zu und engte ein. Der Rückstand kristallisierte aus Isopropylalkohol/Ether. Ausbeute: 1.22 g (74%) rac-cis-2,3-Dichloro-4b-(2-dimethylamino-ethyl)-4b,5,6,7,8,8a,9,10-octahydro-phenanthren-8a-ol hydrochlorid als weisse Kristalle; Schmp. 211-215°C.

**Beispiel 36**

**36.1.** Man löste 2.34 g rac-cis-4b-(2-Dimethylamino-ethyl)-3-fluoro-4b,5,6,7,8,8a,9,10-octahydro-phenanthren-8a-ol (0.008 Mol) in 50 ml THF, fügte 5 ml Triethylamin (0.036 Mol) zu, und tropfte bei 25°C unter Argon eine Lösung von 2.5 ml Essigsäurechlorid (0.035 Mol) in 5 ml THF zu. Das Gemisch wurde 4 St. unter Rückfluss gekocht, dann auf Wasser gegossen und mit $CH_2Cl_2$ ausgeschüttelt. Man trocknete die organische Phase mit $MgSO_4$, filtrierte ab, engte ein und chromatographierte den Rückstand über Kieselgel mit Methanol. Ausbeute: 1.69 g (63%) Essigsäure rac-cis-4b-(2-dimethylaminoethyl)-3-fluoro-4b,5,6,7,8,8a,9,10-octahydro-phenanthren-8a-yl ester als gelbliches Öl.

MS: m/e (% Basis peak) = 333 ($C_{20}H_{28}FNO_2^+$, 0.25), 274 (2.3), 202 (1.4), 159 (3.2), 146 (1.6), 133 (1.65), 109 (1.05), 73 (5), 71 (4) 58 (100).

**36.2.** Essigsäure rac-cis-4b-(2-dimethylamino-ethyl)-3-fluoro-4b,5,6,7,8,8a,9,10-octahydro-phenanthren-8a-yl ester (1.69 g, 0.005 Mol) wurde in 40 ml Ethanol gelöst. Man tropfte eine 4.89 N ethanolische HCl-Lösung (1.03 ml, 0.005 Mol) zu und engte ein. Der Rückstand kristallisierte aus Ether. Ausbeute: 1.25 g (67%) Essigsäure rac-cis-4b-(2-dimethylamino-ethyl)-3-fluoro-4b,5,6,7,8,8a,9,10-octahydro-phenanthren-8a-yl ester hydrochlorid als weisse Kristalle; Schmp. 203-205°C.

**Beispiel 37**

**37.1.** Man löste 2.5 g rac-cis-3-Chloro-4b-(2-dimethylamino-ethyl)-4b,5,6,7,8,8a,9,10-octahydro-phenanthren-8a-ol (0.008 Mol) in 50 ml THF, fügte 5 ml Triethylamin (0.035 Mol) zu, und tropfte bei 25°C unter Argon eine Lösung von 2.5 ml Essigsäurechlorid (0.035 Mol) in 10 ml THF zu. Das Gemisch wurde 2 St. unter Rückfluss gekocht, dann auf Wasser gegossen und mit Essigester ausgeschüttelt. Man trocknete die organische Phase mit $MgSO_4$, filtrierte ab, engte ein und chromatographierte den Rückstand über Kieselgel mit Methanol. Ausbeute: 1.62 g (57%) Essigsäure rac-cis-3-chloro-4b-(2-dimethylamino-ethyl)-4b,5,6,7,8,8a,9,10-octahydro-phenanthren-8a-yl-ester als gelbliches Öl.

MS: m/e (% Basis peak) = 349 ($C_{20}H_{28}ClNO_2^+$, 0.35), 290 (2.8), 218 (2.1), 175 (2.2), 149 (3.5), 97 (6), 85 (7), 83 (8), 71 (16), 58 (100).

**37.2.** Essigsäure rac-cis-3-chloro-4b-(2-dimethylamino-ethyl)-4b,5,6,7,8,8a,9,10-octahydro-phenanthren-8a-yl ester (1.76 g, 0.005 Mol) wurde in 30 ml Ethanol gelöst. Man tropfte eine 4.89 N ethanolische HCl-Lösung (1.02 ml, 0.005 Mol) zu und engte ein. Der Rückstand kristallisierte aus Ether. Ausbeute: 1.57 g (81%) Essigsäure rac-cis-3-chloro-4b-(2-dimethylamino-ethyl)-4b,5,6,7,8,8a,9,10-octahydro-phenanthren-8a-yl ester hydrochlorid als weisse Kristalle; Schmp. 192-194°C.

**Beispiel 38**

**38.1.** Man löste 2.0 g rac-cis-3-Chloro-4b-(2-dimethylamino-ethyl)-4b,5,6,7,8,8a,9,10-octahydro-phenanthren-8a-ol (0.0065 Mol) in 50 ml THF, fügte 2 ml Triethylamin (0.014 Mol) zu, und tropfte bei 25°C unter Argon eine Lösung

von 1.5 ml Buttersäurechlorid (0.014 Mol) in 5 ml THF zu. Das Gemisch wurde 3 St. unter Rückfluss gekocht, dann auf Wasser gegossen und mit Essigester ausgeschüttelt. Man trocknete die organische Phase mit $MgSO_4$, filtrierte ab, engte ein und chromatographierte den Rückstand über Kieselgel mit Methanol. Ausbeute: 2.02 g (82%) Buttersäure rac-cis-3-chloro-4b-(2-dimethylamino-ethyl)-4b,5,6,7,8,8a,9,10-octahydro-phenanthren-8a-yl ester als gelbliches Öl.

MS: m/e (% Basis peak) = 377 ($C_{22}H_{32}ClNO_2^+$, 0.3), 290 (3.7), 218 (1.5), 183 (1.4), 73 (7.5), 71 (6.5), 58 (100).

**38.2.** Buttersäure rac-cis-3-chloro-4b-(2-dimethylamino-ethyl)-4b,5,6,7,8,8a,9,10-octahydro-phenanthren-8a-yl ester (2.47 g, 0.0065 Mol) wurde in 40 ml Ethanol gelöst. Man tropfte eine 4.89 N ethanolische HCl-Lösung (1.33 ml, 0.0065 Mol) zu und engte ein. Der Rückstand kristallisierte aus Ether. Ausbeute: 2.2 g (82%) Buttersäure rac-cis-3-chloro-4b-(2-dimethylamino-ethyl)-4b,5,6,7,8,8a,9,10-octahydro-phenanthren-8a-yl ester hydrochlorid als weisse Kristalle; Schmp. 164-166°C.

**Beispiel 39**

**39.1.** Zu einer Suspension von 7.14 g (-)-2,2'-(1,1'-Binaphthyl)-phosphorsäure (0.0205 Mol) in 900 ml Aceton gab man 12 g rac-cis-4b-(2-Amino-ethyl)-3-benzyloxy-4b,5,6,7,8,8a,9,10-octahydro-phenanthren-8a-ol (0.0341 Mol) in 500 ml Aceton zu. Nach Erwärmen am Rückfluss erhielt man eine farblose Lösung, die bei Raumtemperatur kristallisierte. Nach drei weiteren Umkristallisierungen aus Methanol/Isopropylalkohol 1:1 erhielt man 4.74 g weisse Kristalle; Schmp. 293-295°C.

Man löste diese Kristalle in 200 ml Wasser, stellte mit einer wässrigen 28 prozentigen Natriumhydroxidlösung basisch, filtrierte, schüttelte mit $CH_2Cl_2$ aus, wusch die organische Phase mit Wasser, trocknete über $Na_2SO_4$, filtrierte ab und engte ein. Der Rückstand kristallisierte aus Acetonitril/Ether. Ausbeute: 1.67 g (14%) (+)-cis-4b-(2-Amino-ethyl)-3-benzyloxy-4b,5,6,7,8,8a,9,10-octahydro-phenanthren-8a-ol als weisse Kristalle; Schmp. 124-125°C und $[\alpha]_D^{20}$=+13.5° ($CHCl_3$, c=1%).

**39.2.** Man löste 1.57 g (+)-cis-4b-(2-Amino-ethyl)-3-benzyloxy-4b,5,6,7,8,8a,9,10-octahydro-phenanthren-8a-ol (0.00446 Mol) in 60 ml Ethanol. Nach Zugabe von 0.35 g 10 prozentigem Pd-C Katalysator wurde das Gemisch während 1 St bei RT unter 1 Atm hydriert. Man filtrierte und engte ein. Das Produkt kristallisierte aus Acetonitril. Ausbeute: 1.0 g (86%) (+)-cis-4b-(2-Amino-ethyl)-4b,5,6,7,8,8a,9,10-octahydro-phenanthren-3,8a-diol als weisse Kristalle; Schmp. 186-188°C und $[\alpha]_D^{20}$=+38.4° (MeOH, c=1%).

**39.3.** (+)-cis-4b-(2-Amino-ethyl)-4b,5,6,7,8,8a,9,10-octahydro-phenanthren-3,8a-diol (0.96 g, 0.0037 Mol) wurde in 30 ml Methanol gelöst. Man tropfte eine 4.89 N ethanolische HCl-Lösung (0.75 ml, 0.0037 Mol) zu und engte ein. Der Rückstand kristallisierte aus Ether. Ausbeute: 1.07 g (98%) (+)-cis-4b-(2-Amino-ethyl)-4b,5,6,7,8,8a,9,10-octahydro-phenanthren-3,8a-diol hydrochlorid als weisse Kristalle; Schmp. 109-111°C. $[\alpha]_D^{20}$=+16.7° (MeOH, c=1%).

**Beispiel 40**

**40.1.** Zu einer Suspension von 3.68 g (+)-2,2'-(1,1'-Binaphthyl)-phosphorsäure (0.0106 Mol) in 460 ml Aceton gab man 6.18 g rac-cis-4b-(2-Amino-ethyl)-3-benzyloxy-4b,5,6,7,8,8a,9,10-octahydro-phenanthren-8a-ol (0.0176 Mol) in 250 ml Aceton zu. Nach Erwärmen am Rückfluss erhielt man eine farblose Lösung, die bei Raumtemperatur kristallisierte. Nach drei weiteren Umkristallisierungen aus Methanol/Isopropylalkohol 1:1 erhielt man 5.29 g weisse Kristalle; Schmp. 291-293°C.

Man löste diese Kristalle in 300 ml Wasser, stellte mit einer wässrigen 28 prozentigen Natriumhydroxidlösung basisch, filtrierte, schüttelte mit $CH_2Cl_2$ aus, wusch die organische Phase mit Wasser, trocknete über $Na_2SO_4$, filtrierte ab und engte ein. Man chromatographierte über Kieselgel mit Methanol und kristallisierte den Rückstand aus Acetonitril/Ether. Ausbeute: 2.63 g (42%) (-)-cis-4b-(2-Amino-ethyl)-3-benzyloxy-4b,5,6,7,8,8a,9,10-octahydro-phenanthren-8a-ol als weisse Kristalle ; Schmp. 124-125°C und $[\alpha]_D^{20}$=-11.9° ($CHCl_3$, c=1%).

**40.2.** Man löste 2.94 g (-)-cis-4b-(2-Amino-ethyl)-3-benzyloxy-4b,5,6,7,8,8a,9,10-octahydro-phenanthren-8a-ol (0.00758 Mol) in 100 ml Methanol. Nach Zugabe von 0.7 g 10 prozentigem Pd-C Katalysator wurde das Gemisch während 1 St bei RT unter 1 Atm hydriert. Man filtrierte und engte ein. Das Produkt kristallisierte aus Acetonitril. Ausbeute: 1.67 g (84%) (-)-cis-4b-(2-Amino-ethyl)-4b,5,6,7,8,8a,9,10-octahydro-phenanthren-3,8a-diol als weisse Kristalle (84%); Schmp. 185-187°C und $[\alpha]_D^{20}$=-37.6° (MeOH, c=1%).

**40.3.** (-)-cis-4b-(2-Amino-ethyl)-4b,5,6,7,8,8a,9,10-octahydro-phenanthren-3,8a-diol (1.84 g, 0.007 Mol) wurde in 20 ml Methanol gelöst. Man tropfte eine 4.89 N ethanolische HCl-Lösung (1.44 ml, 0.007 Mol) zu und engte ein. Der Rückstand kristallisierte aus Ether. Ausbeute: 1.93 g (92%) (-)-cis-4b-(2-Aminoethyl)-4b,5,6,7,8,8a,9,10-octahydro-phenanthren-3,8a-diol hydrochlorid als weisse Kristalle; Schmp. 115-117°C. $[\alpha]_D^{20}$=-17.6° (MeOH, c=1%).

**Beispiel 41**

**41.1.** Zu einer Lösung von 20 g 3'4'-Dihydro-spiro[cyclopentan-1,2'(1'H)-naphthalen]-1'-on in 60 ml Schwefelsäure

tropfte man langsam 14 ml eines Gemisches Schwefelsäure/Salpetersäure 65% 1:1 zu. Nach 2 St. Rühren bei RT goss man das Gemisch auf 200 g Eis, schüttelte mit Ether aus, wusch mit einer gesättigten wässrigen $NaHCO_3$ Lösung und mit Wasser, trocknete auf $Na_2SO_4$, filtrierte, engte ein und chromatographierte über Kieselgel mit Cyclohexan / Essigester 9:1. Ausbeute: 12.7 g (52%). Eine Probe wurde aus heissem Isopropylether umkristallisiert und ergab 7'-Nitro-3'4'-dihydro-spiro[cyclopentan-1,2'(1'H)-naphthalen]-1'-on als weisse Kristalle mit Schmp. 69-70°C.

**41.2.** Man löste 7.04 g 7'-Nitro-3'4'-dihydro-spiro[cyclopentan-1,2'(1'H)-naphthalen]-1'-on (0.0287 Mol) in 100 ml THF. Nach Zugabe von 1 g 10 prozentigem Pd-C Katalysator wurde das Gemisch während $^3/_4$ St bei RT unter 1 Atm hydriert. Man filtrierte, engte ein und chromatographierte über Kieselgel mit $CH_2Cl_2$. Das Produkt kristallisierte aus n-Hexan. Ausbeute: 4.95 g (80%) 7'-Amino-3'4'-dihydro-spiro[cyclopentan-1,2'(1'H)-naphthalen]-1'-on als beige Kristalle; Schmp. 78-80°C.

**41.3.** Zu einer Lösung von 8.28 g 7'-Amino-3'4'-dihydro-spiro[cyclopentan-1,2'(1'H)-naphthalen]-1'-on (0.0385 Mol) in 100 ml trocknem Ether gab man bei 0°C 100 ml einer 1.6N Lösung von Methyllithium in Hexan (0.16 Mol) und rührte 2 St. bei RT. Man goss die Suspension auf 200 g Eis, schüttelte mit Ether aus, wusch mit Wasser, trocknete mit $Na_2SO_4$, filtrierte, engte ein und chromatographierte über Kieselgel mit Ether. Das Produkt wurde aus Isopropylether umkristallisiert. Ausbeute: 4.47 g (50%) (RS)-7'-Amino-1'-methyl-3'4'-dihydro-spiro[cyclopentan-1,2'(1'H)-naphthalen]-1'-ol als beige Kristalle; Schmp. 119-120°C.

**41.4.** Eine Lösung von 4.27 g (RS)-7'-Amino-1'-methyl-3'4'-dihydrospiro[cyclopentan-1,2'(1'H)-naphthalen]-1'-ol (0.0185 Mol) und 0.47 g Jod (0.00185 Mol) in 150 ml Toluol wurde $^1/_4$ St. unter Rückfluss gekocht. Man kühlte ab, wusch mit Wasser, trocknete mit $Na_2SO_4$, filtrierte, engte ein und chromatographierte über Kieselgel mit $CH_2Cl_2$. Das Produkt wurde durch eine 10 cm Vigreux Säule destilliert. Ausbeute: 2.51 g 1'-Methylen-3'4'-dihydrospiro[cyclopentan-1,2'(1'H)-naphthalen]-7'-amin als farbloses Öl; Sdp. 113-115°C/0.08 Torr.

**41.5.** Zu einer Lösung von 6.0 g 1'-Methylen-3'4'-dihydro-spiro[cyclopentan-1,2'(1'H)-naphthalen]-7'-amin (0.0281 Mol) und 4.2 ml Triethylamin (3.13 g, 0.0309 Mol) in 150 ml Toluol in 100 ml THF gab man unter Eiskühlung 3.6 ml Benzoylchlorid (4.35 g, 0.0309 Mol) und kochte dann 2 St. unter Rückfluss. Zur abgekühlten Lösung goss man 100 ml Wasser. Man schüttelte mit Ether aus, wusch mit Wasser, trocknete mit $Na_2SO_4$, filtrierte, engte ein und chromatographierte über Kieselgel mit $CH_2Cl_2$. Der erhaltene Festkörper wurde 2 St. in Ether kräftig gerührt, abgenutscht und i. Vak. getrocknet. Ausbeute: 6.98 g (78%) N-[1'-Methylen-3'4'-dihydro-spiro[cyclopentan-1,2'(1'H)-naphthalen]-7'-yl]-benzamid als weisser Festkörper; Schmp. 160-161°C.

**41.6.** Zu einer Lösung von 6.98 g N-[1'-Methylen-3'4'-dihydrospiro[cyclopentan-1,2'(1'H)-naphthalen]-7'-yl]-benzamid (0.022 Mol) in 70 ml THF bei -78°C unter Argonbegasung tropfte man eine Lösung von 2.3 ml Chlorsulfonylisocyanat (3.73 g, 0.0264 Mol) in 5 ml THF zu. Man rührte noch 15 min bei -78°C und liess innerhalb 1 St zur RT erwärmen. Nach 36 St. wurde die Lösung eingeengt. Ausbeute: 17.9 g rac-cis-8-Benzamido-1,2,4,5-tetrahydro-3a,9b-butano-naphtho[2,1-b]furan-2-ylidene-sulfamoylchlorid [(E) oder (Z) oder (E/Z)-Gemisch] als gelbes Öl, das direkt in die nächste Stufe eingesetzt wurde. Eine Probe wurde aus Essigester/Hexan umkristallisiert und ergab weisse Kristalle; Schmp. 185-187°C.

**41.7.** 6,8 g (0.18 Mol) Lithiumaluminiumhydrid wurden in 250 ml Dioxan unter Argon suspendiert. Man tropfte eine Lösung aus 17.9 g rohem rac-cis-8-Benzamido-1,2,4,5-tetrahydro-3a,9b-butano-naphtho[2,1-b]furan-2-ylidene-sulfamoylchlorid [(E) oder (Z) oder (E/Z)-Gemisch] (0.022 Mol) in 250 ml THF zu und kochte 24 St. unter Rückfluss. Vorsichtig tropfte man nacheinander bei ~40°C 70 ml Essigester, 7 ml Wasser, 7 ml einer 15 prozentigen wässrigen NaOH Lösung und 21 ml Wasser zu, so dass ein völlig weisser Niederschlag entstand. Nach Filtrieren und Einengen wurde der Rückstand über Kieselgel mit Methanol und Methanol/Ammoniak 9:1 chromatographiert. Das Produkt wurde in Ether suspendiert, abgenutscht, mit Ether gewaschen und i. Vak. getrocknet. Ausbeute: 2.58 g (33%) rac-cis-4b-(2-Amino-ethyl)-3-benzylamino-4b,5,6,7,8,8a,9,10-octahydrophenanthren-8a-ol als weisse Kristalle; Schmp. 152-154°C.

**41.8.** Man löste 3.0 g rac-cis-4b-(2-Amino-ethyl)-3-benzylamino-4b,5,6,7,8,8a,9,10-octahydro-phenanthren-8a-ol (0.00856 Mol) in 300 ml Ethanol. Nach Zugabe von 1.5 g 10 prozentigem Pd-C Katalysator wurde das Gemisch während 20 St bei RT unter 50 Bar hydriert. Man filtrierte, engte ein und chromatographierte über Kieselgel mit Methanol/Ammoniak 9:1. Das Produkt wurde aus Ethanol/Ether umkristallisiert. Ausbeute: 1.5 g rac-cis-3-Amino-4b-(2-amino-ethyl)-4b,5,6,7,8,8a,9,10-octahydro-phenanthren-8a-ol als weisse Kristalle; Schmp. 160-162°C.

**41.9.** rac-cis-3-Amino-4b-(2-amino-ethyl)-4b,5,6,7,8,8a,9,10-octahydro-phenanthren-8a-ol (1.5 g, 0.00576 Mol) wurde in 30 ml Ethanol gelöst. Man tropfte eine 4.89 N ethanolische HCl-Lösung (1.18 ml, 5.77 Mol) zu, filtrierte über Norit und engte ein. Der Rückstand wurde 48 St. in Ether kräftig geschüttelt, abgenutscht und i. Vak. getrocknet. Ausbeute: 1.32 g (77%) rac-cis-3-Amino-4b-(2-amino-ethyl)-4b,5,6,7,8,8a,9,10-octahydro-phenanthren-8a-ol hydrochlorid als beiger, amorpher Festkörper; Schmp. 105-107°C.

**Beispiel 42**

**42.1.** Zu einer Suspension von 7.0 g (+)-2,2'-(1,1'-Binaphthyl)-phosphorsäure (0.0201 Mol) in 500 ml Aceton gab man eine Lösung von 9.37 g rac-cis-4b-(2-Amino-ethyl)-3-chloro-4b,5,6,7,8,8a,9,10-octahydro-phenanthren-8a-ol

(0.0304 Mol) in 100 ml Aceton zu. Nach leichtem Erwärmen erhielt man eine hellgelbe Lösung, die eingeengt wurde.

Der amorphe Rückstand wurde in 1 l heissem Essigester gelöst, dekantiert und bei Raumtemperatur kristallisiert. Nach drei weiteren Umkristallisierungen aus Ethanol erhielt man 4.01 g weisse Kristalle; Schmp. 166-168°C.

Man löste diese Kristalle in 150 ml Wasser, stellte mit einer wässrigen 2N Natriumhydroxidlösung basisch, filtrierte, schüttelte mit $CH_2Cl_2$ aus, wusch die organische Phase mit Wasser, trocknete über $Na_2SO_4$, filtrierte ab und engte ein. Ausbeute: 1.86 g (20%) (-)-cis-3-Chloro-4b-[2-(dimethylamino-ethyl)-4b,5,6,7,8,8a,9,10-octahydro-phenanthren-8a-ol als hellgelbes Öl mit $[\alpha]_D^{20}$=-15.2° (MeOH, c=1%).

**42.2.** (-)-cis-3-Chloro-4b-(2-dimethylamino-ethyl)-4b,5,6,7,8,8a,9,10-octahydro-phenanthren-8a-ol (1.78 g, 0.0058 Mol) wurde in 50 ml Ethanol gelöst. Man tropfte eine 4.89 N ethanolische HCl-Lösung (1.18 ml, 0.0058 Mol) zu und engte ein. Der Rückstand kristallisierte aus Ethanol/Ether. Ausbeute: 1.67 g (84%) (+)-cis-3-Chloro-4b-(2-dimethylamino-ethyl)-4b,5,6,7,8,8a,9,10-octahydro-phenanthren-8a-ol hydrochlorid als weisse Kristalle; Schmp. 225-227°C. $[\alpha]_D^{20}$=+2.8° (MeOH, c=1%).

**Beispiel 43**

**43.1.** Zu einer Suspension von 7.74 g (-)-2,2'-(1,1'-Binaphthyl)-phosphorsäure (0.0222 Mol) in 550 ml Aceton gab man 10.4 g rac-cis-4b-(2-Amino-ethyl)-3-chloro-4b,5,6,7,8,8a,9,10-octahydro-phenanthren-8a-ol (0.0337 Mol) in 100 ml Aceton zu. Nach leichtem Erwärmen erhielt man eine hellgelbe Lösung, die eingeengt wurde.

Der amorphe Rückstand (18.8 g) wurde in 560 ml heissem Essigester gelöst, dekantiert und bei Raumtemperatur kristallisiert. Nach drei weiteren Umkristallisierungen aus Ethanol erhielt man 5.01 g weisse Kristalle; Schmp. 166-168°C.Man löste diese Kristalle in 200 ml Wasser, stellte mit einer wässrigen 2N Natriumhydroxidlösung basisch, filtrierte, schüttelte mit $CH_2Cl_2$ aus, wusch die organische Phase mit Wasser, trocknete über $Na_2SO_4$, filtrierte ab und engte ein. Ausbeute: 2.3 g (22%) (+)-cis-3-Chloro-4b-(2-dimethylaminoethyl)-4b,5,6,7,8,8a,9,10-octahydro-phenanthren-8a-ol als hellgelbes Öl mit $[\alpha]_D^{20}$=+16.2° (MeOH, c=1%).

**43.2.** (+)-cis-3-Chloro-4b-(2-dimethylamino-ethyl)-4b,5,6,7,8,8a,9,10-octahydro-phenanthren-8a-ol (2.22 g, 0.007 Mol) wurde in 50 ml Ethanol gelöst. Man tropfte eine 4.89 N ethanolische HCl-Lösung (1.47 ml, 0.007 Mol) zu und engte ein. Der Rückstand kristallisierte aus Ethanol/Ether. Ausbeute: 2.11 g (85%) (-)-cis-3-Chloro-4b-(2-dimethylamino-ethyl)-4b,5,6,7,8,8a,9,10-octahydro-phenanthren-8a-ol hydrochlorid als weisse Kristalle; Schmp. 225-226°C. $[\alpha]_D^{20}$= -2.8° (MeOH, c=1%).

**Beispiel 44**

**44.1** Zu einer Lösung von 50 g 1,2,3,4-Tetrahydronaphthalen-1-on (0.34 Mol) in 700 ml THF unter Argonbegasung gab man portionsweise bei -78°C 76.7 g Kalium tert-Butylat (0.68 Mol) zu. Man rührte 2 St bei -78°C nach und tropfte dann eine Lösung von 70 ml 1,4-Dibrombutan (128 g, 0.59 Mol) in 150 ml THF zu. Das Kühlbad wurde entfernt und man rührte 16 St bei RT nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Hexan/Toluol 2:1. Das Produkt wurde durch eine 20 cm Vigreux Säule destilliert. Ausbeute: 29.6 g (76%) 3'4'-Dihydro-spiro[cyclopentan-1,2'(1'H)-naphthalen]-1'-on als farblose Flüssigkeit; Sdp. 92-94°C/ 0.08 Torr.

**44.2.** 134 g Methyltriphenylphosphoniumbromid/Natriumamid-Gemisch (0.32 Mol) wurden während $^3/_4$ St in 700 ml THF bei RT unter Argon gerührt. Zu der gelben Suspension tropfte man eine Lösung von 29.5 g 3'4'-Dihydrospiro [cyclopentan-1,2'(1'H)-naphthalen]-1'-on (0.147 Mol) in 200 ml THF zu und rührte dann 18 St nach. Man fügte Wasser zu, schüttelte mit Ether aus, wusch die organische Phase mit einer gesättigten wässrigen Kochsalzlösung, trocknete über $MgSO_4$, filtrierte ab, engte ein und chromatographierte den Rückstand über Kieselgel mit Toluol. Das Produkt wurde durch eine 20 cm Vigreux Säule destilliert. Ausbeute: 26.6 g (91%) 1'-Methylen-3'4'-dihydrospiro[cyclopentan-1,2'(1'H)-naphthalen] als farblose Flüssigkeit ; Sdp. 78-79°C/0.09 Torr.

**44.3.** Zu einer Lösung von 22 g 1'-Methylen-3'4'-dihydro-spiro[cyclopentan-1,2'(1'H)-naphthalen] (0.111 Mol) in 200 ml Ether bei -78°C unter Argonbegasung tropfte man eine Lösung von 11.6 ml Chlorsulfonylisocyanat (0.133 Mol) in 30 ml Ether zu. Man rührte 15 min bei -78°C und liess innerhalb 1 St zur RT erwärmen. Das entstandene Kristallisat wurde abgenutscht, mit Ether gewaschen und i. Vak. getrocknet. Ausbeute: 31.7 g (84%) rac-cis-1,2,4,5-Tetrahydro-3a,9b-butano-naphtho[2,1-b]furan-2-yliden-sulfamoylchlorid [(E) oder (Z) oder (E/Z)-Gemisch] als gelbliche Kristalle; Schmp. 141-144°C.

**44.4** 18.4 g (0.48 Mol) Lithiumaluminiumhydrid wurden in 500 ml Dioxan unter Argon suspendiert. Man tropfte eine Lösung aus 32.9 g rac-cis-1,2,4,5-Tetrahydro-3a,9b-butano-naphtho[2,1-b]furan-2-yliden-sulfamoylchlorid [(E) oder (Z) oder (E/Z)-Gemisch] (0.097 Mol) in 300 ml THF zu und kochte $1^1/_2$ St unter Rückfluss. Vorsichtig tropfte man bei RT 60 ml eines 2:1 Gemisches NaOH 28%/Wasser zu, so dass ein völlig weisser Niederschlag entstand. Nach Filtrieren und Einengen wurde der Rückstand über Kieselgel mit Methanol chromatographiert. Das Produkt kristallisierte aus

Acetonitril. Ausbeute: 9.9 g (42%) rac-cis-4b-(2-Amino-ethyl)-4b,5,6,7,8,8a,9,10-octahydro-phenanthren-8a-ol als weisse Kristalle; Schmp. 157-158°C.

**44.5.** 5 g (0.024 Mol) rac-cis-4b-(2-Amino-ethyl)-4b,5,6,7,8,8a,9,10-octahydro-phenanthren-8a-ol wurden in 100 ml Methanol gelöst. Dann tropfte man 4.9 ml (0.024 Mol) einer 4.89N ethanolischen HCl-Lösung zu und engte ein. Der Rückstand kristallisierte aus Ether. Ausbeute: 5.71 g (84%) rac-cis-4b-(2-Amino-ethyl)-4b,5,6,7,8,8a,9,10-octahydro-phenanthren-8a-ol hydrochlorid als weisse Kristalle; Schmp. 222-224°C.

## Patentansprüche

1. Verbindungen der allgemeinen Formel

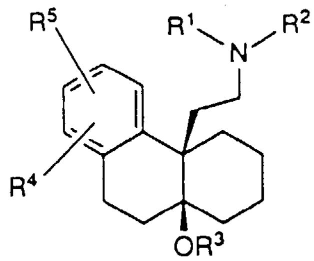

I

worin

| | |
|---|---|
| $R^1$ und $R^2$ | unabhängig voneinander Wasserstoff oder gegebenenfalls durch Aryl oder $C_{3-6}$-Cycloalkyl substituiertes $C_1$-$C_7$-Alkyl; |
| $R^4$ und $R^5$ | entweder beide Wasserstoff oder beide Halogen, oder das eine Wasserstoff und das andere Halogen, Hydroxy, $C_1$-$C_7$-Alkoxy, Aryloxy oder Amino und |
| $R^3$ | Wasserstoff oder - falls keine primäre(n) oder sekundäre(n) Aminogruppe(n) vorhanden ist (sind) - Alkanoyl bedeuten, |

sowie pharmazeutisch verwendbare Salze von Verbindungen der Formel I mit Säuren; mit Ausnahme von rac-cis-4b-(2-Amino-ethyl)- 4b,5,6,7,8,8a,9,10-octahydro-phenanthren-8a-ol.

2. Verbindungen gemäss Anspruch 1, worin $R^1$ und $R^2$ unabhängig voneinander Wasserstoff oder gegebenenfalls durch $C_{3-6}$-Cycloalkyl substituiertes $C_1$-$C_7$-Alkyl, $R^3$ Wasserstoff oder Alkanoyl und $R^4$ und $R^5$ jeweils Halogen oder das eine Wasserstoff und das andere Halogen, Heteroaryloxy, $C_1$-$C_7$-Alkoxy oder Hydroxy bedeuten.

3. Verbindungen gemäss Anspruch 1, worin $R^1$ und $R^2$ $C_1$-$C_7$-Alkyl, $R^3$ Wasserstoff und $R^4$ und $R^5$ jeweils Wasserstoff oder das eine Wasserstoff und das andere Halogen oder Hydroxy bedeuten.

4. Eine Verbindung gemäß Anspruch 1, nämlich rac-cis-4b-(2-Dimethylamino-ethyl)-4b,5,6,7,8,8a,9,10-octahydro-phenanthren-8a-ol.

5. Eine Verbindung gemäß Anspruch 1, nämlich rac-cis-3-Chloro-4b-(2-dimethylamino-ethyl)-4b,5,6,7,8,8a,9,10-octahydro-phenanthren-8a-ol.

6. Eine Verbindung gemäß Anspruch 1, nämlich (-)-cis-3-Chloro-4b-(2-dimethylamino-ethyl)-4b,5,6,7,8,8a,9,10-octahydro-phenanthren-8a-ol.

7. Eine Verbindung gemäß Anspruch 1, nämlich (+)-cis-3-Chloro-4b-(2-dimethylamino-ethyl)-4b,5,6,7,8,8a,9,10-octahydro-phenanthren-8a-ol.

8. Eine Verbindung gemäß Anspruch 1, nämlich rac-cis-4b-(2-Dimethylamino-ethyl)-4b,5,6,7,8,8a,9,10-octahydro-phenanthren-3,8a-diol.

9. Verbindungen gemäß Anspruch 1, nämlich

rac-cis-4-(2-Amino-ethyl)-3-fluoro-4b,5,6,7,8,8a,9,10-octahydro-phenanthren-8a-ol;
rac-cis-4b-(2-Amino-ethyl)-3-chloro-4b,5,6,7,8,8a,9,10-octahydro-phenanthren-8a-ol;
rac-cis-4b-(2-Amino-ethyl)-3-bromo-4b,5,6,7,8,8a,9,10-octahydro-phenanthren-8a-ol;
rac-cis-4b-(2-Amino-ethyl)-4b,5,6,7,8,8a,9,10-octahydro-phenanthren-3,8a-diol;
(+)-cis-4b-(2-Amino-ethyl)-4b,5,6,7,8,8a,9,10-octahydro-phenanthren-3,8a-diol;
(-)-cis-4b-(2-Amino-ethyl)-4b,5,6,7,8,8a,9,10-octahydro-phenanthren-3,8a-diol;
rac-cis-4b-(2-Dimethylamino-ethyl)-3-fluoro-4b,5,6,7,8,8a,9,10-octahydro-phenanthren-8a-ol;
rac-cis-4b-(2-Dimethylamino-ethyl)-3-methoxy-4b,5,6,7,8,8a,9,10-octahydro-phenanthren-8a-ol;
Essigsäure rac-cis-4b-(2-dimethylamino-ethyl)-3-fluoro-4b,5,6,7,8,8a,9,10-octahydro-phenanthren-8a-yl ester;
Essigsäure rac-cis-3-chloro-4b-(2-dimethylamino-ethyl)-4b,5,6,7,8,8a,9,10-octahydro-phenanthren-8a-yl ester;
rac-cis-4b-(2-Amino-ethyl)-3-chloro-1-fluoro-4b,5,6,7,8,8a,9,10-octahydro-phenanthren-8a-ol.

**10.** Verbindungen der allgemeinen Formeln

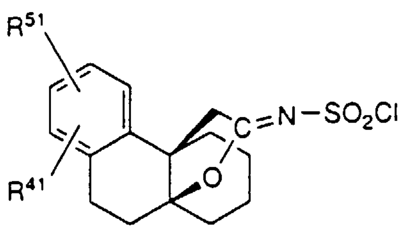

II

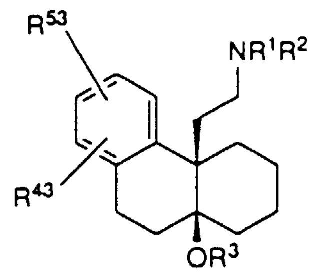

III

und

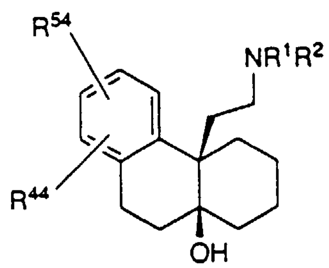

IV

worin $R^1$, $R^2$ und $R^3$ die in Anspruch 1 angegebene Bedeutung besitzen, $R^{41}$ und $R^{51}$ beide Halogen bedeuten oder das eine Wasserstoff und das andere Halogen, $C_1$-$C_7$-Alkoxy oder Aryloxy bedeutet, eines von $R^{43}$ und $R^{53}$ Wasserstoff und das andere Arylmethoxy bedeutet und eines von $R^{44}$ und $R^{54}$ Wasserstoff und das andere Arylmethylamino bedeutet,
sowie Analoga von Verbindungen der Formel II, worin aber eines von $R^{41}$ und $R^{51}$ Wasserstoff und das andere Arylmethoxy oder Arylcarbonylamino bedeutet.

**11.** Verbindungen nach einem der Ansprüche 1-9 und rac-cis-4b-(2-Amino-ethyl)-4b,5,6,7,8,8a,9,10-octahydro-phenanthren-8a-ol sowie pharmazeutisch annehmbare Säureadditionssalze davon zur Anwendung als therapeutische Wirkstoffe.

**12.** Verbindungen nach einem der Ansprüche 1-9 und rac-cis-4b-(2-Amino-ethyl)-4b,5,6,7,8,8a,9,10-octahydro-phenanthren-8a-ol sowie pharmazeutisch annehmbare Säureadditionssalze davon zur Anwendung als neuroprotective Wirkstoffe zur Behandlung oder Verhütung von Ischämie, Hypoglykämie, Hypoxie, cerebralen Gefässpasmen, Spastizität, Trauma, Hemorrhagie, Infektionen (viral, bakteriell, amöbisch, prional), epileptischen Anfällen, autoimmunen Erkrankungen, Entzugssymptomen, Alzheimerscher Krankheit, Parkinsonscher Krankheit, amyotropher Lateralsclerose, Morbus Huntington, Vergiftungen, Olivoponto-cerebellarer Atrophie, Rückenmarkverletzung, Schizophrenie, Depressionen, Angstzuständen, Abhängigkeit, Schmerzen, Autismus und mentaler Retardierung.

**13.** Verfahren zur Herstellung von Verbindungen gemäss einem der Ansprüche 1-9, dadurch gekennzeichnet, dass man

a) zur Herstellung einer Verbindung der Formel I, worin $R^1$, $R^2$ und $R^3$ je Wasserstoff und $R^4$ und $R^5$ beide Halogen oder das eine Wasserstoff und das andere Halogen, $C_1$-$C_7$-Alkoxy oder Aryloxy bedeuten,
eine Verbindung der allgemeinen Formel

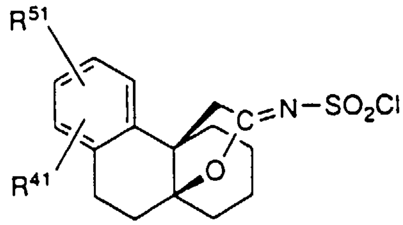

II

worin $R^{41}$ und $R^{51}$ beide Halogen oder das eine Wasserstoff und das andere Halogen, $C_1$-$C_7$-Alkoxy oder Aryloxy bedeuten,
reduziert, oder

b) zur Herstellung einer Verbindung der Formel I, worin eines von $R^1$ und $R^2$ Wasserstoff und das andere gegebenenfalls durch Aryl oder $C_{3-6}$-Cycloalkyl substituiertes $C_1$-$C_7$-Alkyl, oder $R^1$ und $R^2$ je gegebenenfalls durch Aryl oder $C_{3-6}$-Cycloalkyl substituiertes $C_1$-$C_7$-Alkyl, $R^3$ Wasserstoff und $R^4$ und $R^5$ beide Wasserstoff oder Halogen oder das eine Wasserstoff und das andere Halogen, $C_1$-$C_7$-Alkoxy oder Aryloxy bedeuten,
eine Verbindung der allgemeinen Formel

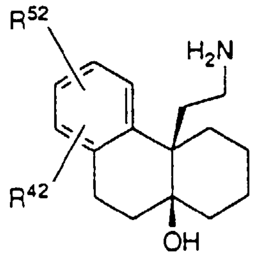

Ia

worin $R^{42}$ und $R^{52}$ beide Wasserstoff oder Halogen oder das eine Wasserstoff und das andere Halogen, $C_1$-$C_7$-Alkoxy oder Aryloxy bedeuten,
an der primären Aminogruppe entsprechend mono- oder disubstituiert; oder

c) zur Herstellung einer Verbindung der Formel I, worin $R^1$ und $R^2$ gegebenenfalls durch Aryl oder $C_{3-6}$-Cycloalkyl substituiertes $C_1$-$C_7$-Alkyl, $R^3$ Alkanoyl und $R^4$ und $R^5$ beide Wasserstoff oder Halogen oder das eine Wasserstoff und das andere Halogen, $C_1$-$C_7$-Alkoxy oder Aryloxy bedeuten, eine Verbindung der allgemeinen Formel

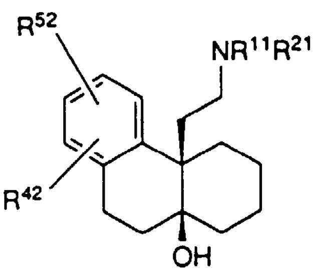

Ib

worin $R^{11}$ und $R^{21}$ gegebenenfalls durch Aryl oder $C_{3-6}$-Cycloalkyl substituiertes $C_1$-$C_7$-Alkyl bedeuten und $R^{42}$ und $R^{52}$ obige Bedeutung besitzen,
mit einer eine Alkanoyl-Gruppe liefernden Verbindung behandelt, oder

d) zur Herstellung einer Verbindung der Formel I, worin eines von $R^4$ und $R^5$ Wasserstoff und das andere Hydroxy bedeutet,
eine Verbindung der allgemeinen Formel

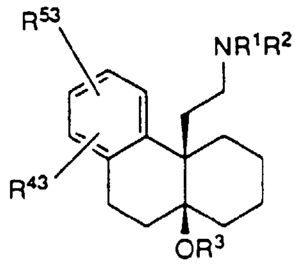

III

worin $R^1$, $R^2$ und $R^3$ die in Anspruch 1 angegebene Bedeutung besitzen, und eines von $R^{43}$ und $R^{53}$ Wasserstoff und das andere Arylmethoxy bedeutet,
in Gegenwart eines Katalysators hydriert, oder

e) zur Herstellung einer Verbindung der Formel I, worin $R^1$ und $R^2$ die in Anspruch 1 angegebene Bedeutung besitzen, $R^3$ Wasserstoff und eines von $R^4$ und $R^5$ Wasserstoff und das andere Amino bedeuten,
eine Verbindung der allgemeinen Formel

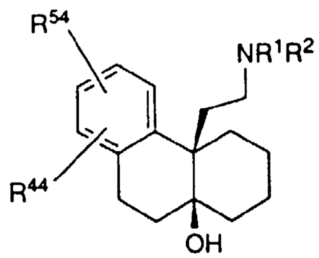

IV

worin $R^1$ und $R^2$ die in Anspruch 1 angegebene Bedeutung besitzen und eines von $R^{44}$ und $R^{54}$ Wasserstoff und das andere Arylmethylamino bedeutet,
in Gegenwart eines Katalysators hydriert, oder

f) zur Herstellung einer Verbindung der Formel I, worin $R^1$, $R^2$ und $R^3$ die in Anspruch 1 angegebene Bedeutung besitzen und eines von $R^4$ und $R^5$ Wasserstoff und das andere Heteroaryloxy bedeutet,
eine Verbindung der allgemeinen Formel

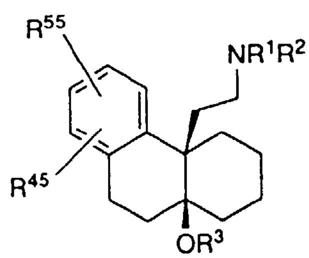

Ic

worin $R^1$, $R^2$ und $R^3$ die in Anspruch 1 angegebene Bedeutung besitzen und eines von $R^{45}$ und $R^{55}$ Wasserstoff und das andere Hydroxy bedeuten,
mit einer Verbindung der Formel X-Z, worin Z eine Abgangsgruppe und X Heteroaryl bedeuten, behandelt,

g) erwünschtenfalls ein erhaltenes Diastereomerengemisch und/oder Racemat auftrennt, und/oder

h) erwünschtenfalls eine erhaltene Verbindung der Formel I in ein pharmazeutisch annehmbares Salz überführt.

**14.** Arzneimittel, insbesondere zur Behandlung oder Verhütung von Ischämie, Hypoglykämie, Hypoxie, cerebralen Gefässpasmen, Spastizität, Trauma, Hemorrhagie, Infektionen (viral, bakteriell, amöbisch, prional), epileptischen Anfällen, autoimmunen Erkrankungen, Entzugssymptomen, Alzheimerscher Krankheit, Parkinsonscher Krankheit, amyotropher Lateralsclerose, Morbus Huntington, Vergiftungen, Olivoponto-cerebellarer Atrophie, Rückenmarkverletzung, Schizophrenie, Depressionen, Angstzuständen, Abhängigkeit, Schmerzen, Autismus und mentaler Retardierung, enthaltend eine Verbindung nach einem der Ansprüche 1-9 oder rac-cis-4b-(2-Amino-ethyl)-4b, 5,6,7,8,8a,9,10-octahydro-phenanthren-8a-ol oder ein pharmazeutisch annehmbares Säureadditionssalz davon und ein therapeutisch inertes Trägermaterial.

**15.** Verwendung von Verbindungen nach einem der Ansprüche 1-9 sowie von rac-cis-4b-(2-Amino-ethyl)-4b, 5,6,7,8,8a,9,10-octahydrophen-anthren-8a-ol oder von pharmazeutisch anwendbaren Säureadditionssalzen davon zur Herstellung von neuroprotectiven Mitteln zur Behandlung oder Verhütung von Ischämie, Hypoglykämie, Hypoxie, cerebralen Gefässpasmen, Spastizität, Trauma, Hemorrhagie, Infektionen (viral, bakteriell, amöbisch, prional), epileptischen Anfällen, autoimmunen Erkrankungen, Entzugssymptomen, Alzheimerscher Krankheit, Parkinsonscher Krankheit, amyotropher Lateralsclerose, Morbus Huntington, Vergiftungen, Olivoponto-cerebellarer Atrophie, Rückenmarkverletzung, Schizophrenie, Depressionen, Angstzuständen, Abhängigkeit, Schmerzen, Autismus und mentaler Retardierung.

**Claims**

**1.** Compounds of the general formula

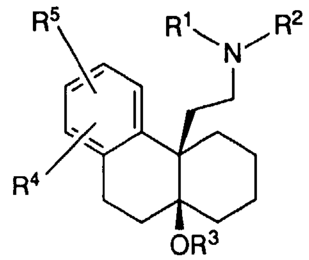

I

wherein

| | |
|---|---|
| $R^1$ and $R^2$ | each individually signify hydrogen or $C_1$-$C_7$-alkyl optionally substituted by aryl or $C_{3-6}$-cycloalkyl; |
| $R^4$ and $R^5$ | either both signify hydrogen or both signify halogen or one signifies hydrogen and the other signifies halogen, hydroxy, $C_1$-$C_7$-alkoxy, aryloxy or amino and |
| $R^3$ | signifies hydrogen or, where no primary or secondary amino group(s) is/are present, alkanoyl, |

as well as pharmaceutically usable salts of compounds of formula I with acids; with the exception of rac-cis-4b-(2-amino-ethyl)-4b,5,6,7,8,8a,9,10-octahydro-phenanthren-8a-ol.

**2.** Compounds according to claim 1, wherein $R^1$ and $R^2$ each independently signify hydrogen or $C_1$-$C_7$-alkyl optionally substituted by $C_{3-6}$-cycloalkyl, $R^3$ signifies hydrogen or alkanoyl and $R^4$ and $R^5$ each signify halogen or one signifies hydrogen and the other signifies halogen, heteroaryloxy, $C_1$-$C_7$-alkoxy or hydroxy.

**3.** Compounds according to claim 1, wherein $R^1$ and $R^2$ signify $C_1$-$C_7$-alkyl, $R^3$ signifies hydrogen and $R^4$ and $R^5$ each signify hydrogen or one signifies hydrogen and the other signifies halogen or hydroxy.

**4.** A compound in accordance with claim 1, namely rac-cis-4b-(2-Dimethylamino-ethyl)-4b,5,6,7,8,8a,9, 10-octahydro-phenanthren-8a-ol.

**5.** A compound in accordance with claim 1, namely rac-cis-3-Chloro-4b-(2-dimethylamino-ethyl)-4b,5, 6,7,8,8a, 9,10-octahydro-phenanthren-8a-ol.

6. A compound in accordance with claim 1, namely (-)-cis-3-Chloro-4b-(2-dimethylamino-ethyl)-4b,5,6, 7,8,8a, 9,10-octahydro-phenanthren-8a-ol.

7. A compound in accordance with claim 1, namely (+)-cis-3-Chloro-4b-(2-dimethylamino-ethyl)-4b,5,6, 7,8,8a, 9,10-octahydro-phenanthren-8a-ol.

8. A compound in accordance with claim 1, namely rac-cis-4b-(2-Dimethylamino-ethyl)-4b,5,6,7,8,8a,9, 10-octahydro-phenanthrene-3,8a-diol.

9. Compounds in accordance with claim 1, namely

rac-cis-4b-(2-Amino-ethyl)-3-fluoro-4b,5,6,7,8,8a, 9,10-octahydro-phenanthren-8a-ol;
rac-cis-4b-(2-amino-ethyl)-3-chloro-4b,5,6,7,8,8a,9,10-octahydro-phenanthren-8a-ol;
rac-cis-4b-(2-amino-ethyl)-3-bromo-4b,5,6,7,8,8a,9,10-octahydro-phenanthren-8a-ol;
rac-cis-4b-(2-amino-ethyl)-4b,5,6,7,8,8a,9,10-octahydro-phenanthrene-3,8a-diol;
(+)-cis-4b-(2-amino-ethyl)-4b,5,6,7,8,8a,9,10-octahydro-phenanthrene-3,8a-diol;
(-)-cis-4b-(2-amino-ethyl)-4b,5,6,7,8,8a,9,10-octahydro-phenanthrene-3,8a-diol;
rac-cis-4b-(2-dimethylamino-ethyl)-3-fluoro-4b,5,6,7,8, 8a,9,10-octahydro-phenanthren-8a-ol;
rac-cis-4b-(2-dimethylamino-ethyl)-3-methoxy-4b,5,6,7, 8,8a,9,10-octahydro-phenanthren-8a-ol;
acetic acid rac-cis-4b-(2-dimethylamino-ethyl)-3-fluoro-4b,5,6,7,8,8a,9,10-octahydro-phenanthren-8a-yl ester;
acetic acid rac-cis-3-chloro-4b-(2-dimethylamino-ethyl)-4b,5,6,7,8,8a,9,10-octahydro-phenanthren-8a-yl ester;
rac-cis-4b-(2-amino-ethyl)-3-chloro-1-fluoro-4b,5,6,7,8, 8a,9,10-octahydro-phenanthren-8a-ol.

10. Compounds of the general formulae

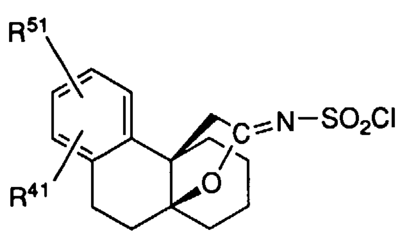

II

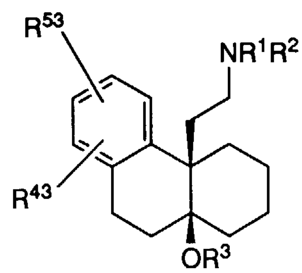

III

and

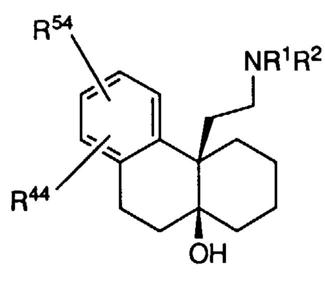

IV

wherein $R^1$, $R^2$ and $R^3$ have the significance given in claim 1, $R^{41}$ and $R^{51}$ both signify halogen or one signifies hydrogen and the other signifies halogen, $C_1$-$C_7$-alkoxy or aryloxy, one of $R^{43}$ and $R^{53}$ signifies hydrogen and the other signifies arylmethoxy and one of $R^{44}$ and $R^{54}$ signifies hydrogen and the other signifies arylmethylamino, as well as analogues of compounds of formula II in which one of $R^{41}$ and $R^{51}$ signifies hydrogen and the other signifies arylmethoxy or arylcarbonylamino.

11. Compounds according to any one of claims 1-9 and rac-cis-4b-(2-amino-ethyl)-4b,5,6,7,8,8a,9,10-octahydro-phenanthren-8a-ol as well as pharmaceutically acceptable acid addition salts thereof for use as therapeutically active substances.

**12.** Compounds according to any one of claims 1-9 and rac-cis-4b-(2-amino-ethyl)-4b,5,6,7,8,8a,9,10-octahydro-phenanthren-8a-ol as well as pharmaceutically acceptable acid addition salts thereof for use as neuroprotective active substances for the treatment or prevention of ischemia, hypoglycaemia, hypoxia, cerebral vasospasms, spasticity, trauma, hemorrhage, infections (viral, bacterial, amoebic, prional), epileptic seizures, autoimmune disorders, withdrawal symptoms, Alzheimer's disease, Parkinson's disease, amyotrophic lateral sclerosis, Huntington's chorea, intoxications, Olivoponto-cerebellar atrophy, spinal injury, schizophrenia, depressions, anxiety states, dependence, pains, autism and mental retardation.

**13.** A process for the manufacture of compounds according to any one of claims 1-9, which process comprises

a) for the manufacture of a compound of formula I in which $R^1$, $R^2$ and $R^3$ each signify hydrogen and $R^4$ and $R^5$ both signify halogen or one signifies hydrogen and the other signifies halogen, $C_1$-$C_7$-alkoxy or aryloxy, reducing a compound of the general formula

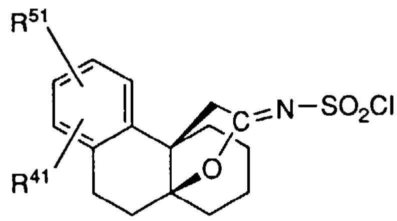

II

wherein $R^{41}$ and $R^{51}$ both signify halogen or one signifies hydrogen and the other signifies halogen, lower alkoxy or aryloxy,
or

b) for the manufacture of a compound of formula I in which one of $R^1$ and $R^2$ signifies hydrogen and the other signifies $C_1$-$C_7$-alkyl optionally substituted by aryl or $C_{3-6}$-cycloalkyl or $R^1$ and $R^2$ each signify $C_1$-$C_7$-alkyl optionally substituted by aryl or $C_{3-6}$-cycloalkyl, $R^3$ signifies hydrogen and $R^4$ and $R^5$ both signify hydrogen or halogen or one signifies hydrogen and the other signifies halogen, $C_1$-$C_7$-alkoxy or aryloxy, correspondingly mono- or disubstituting a compound of the general formula

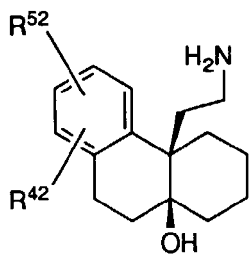

Ia

wherein $R^{42}$ and $R^{52}$ both signify hydrogen or halogen or one signifies hydrogen and the other signifies halogen, $C_1$-$C_7$-alkoxy or aryloxy,
at the primary amino group; or

c) for the manufacture of a compound of formula I in which $R^1$ and $R^2$ signify $C_1$-$C_7$-alkyl optionally substituted by aryl or $C_{3-6}$-cycloalkyl, $R^3$ signifies alkanoyl and $R^4$ and $R^5$ both signify hydrogen or halogen or one signifies hydrogen and the other signifies halogen, $C_1$-$C_7$-alkoxy or aryloxy, treating a compound of the general formula

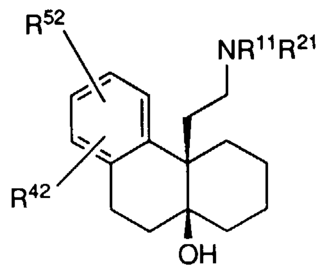

Ib

wherein $R^{11}$ and $R^{21}$ signify $C_1$-$C_7$-alkyl optionally substituted by aryl or $C_{3-6}$-cycloalkyl and $R^{42}$ and $R^{52}$ have the significance given earlier in this claim, with a compound yielding an alkanoyl group, or

d) for the manufacture of a compound of formula I in which one of $R^4$ and $R^5$ signifies hydrogen and the other signifies hydroxy, hydrogenating a compound of the general formula

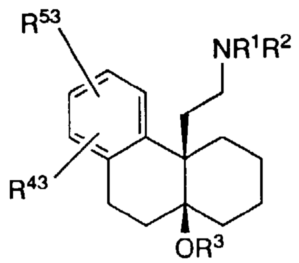

III

wherein $R^1$, $R^2$ and $R^3$ have the significance given in claim 1 and one of $R^{43}$ and $R^{53}$ signifies hydrogen and the other signifies arylmethoxy, in the presence of a catalyst, or

e) for the manufacture of a compound of formula I in which $R^1$ and $R^2$ have the significance given in claim 1, $R^3$ signifies hydrogen and one of $R^4$ and $R^5$ signifies hydrogen and the other signifies amino, hydrogenating a compound of the general formula

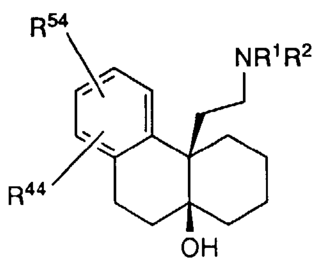

IV

wherein $R^1$ and $R^2$ have the significance given in claim 1 and one of $R^{44}$ and $R^{54}$ signifies hydrogen and the other signifies arylmethylamino, in the presence of a catalyst, or

f) for the manufacture of a compound of formula I in which $R^1$, $R^2$ and $R^3$ have the significance given in claim 1 and one of $R^4$ and $R^5$ signifies hydrogen and the other signifies heteroaryloxy, treating a compound of the general formula

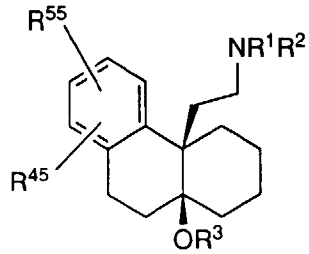

Ic

wherein $R^1$, $R^2$ and $R^3$ have the significance given in claim 1 and one of $R^{45}$ and $R^{55}$ signifies hydrogen and the other signifies hydroxy,
with a compound of the formula X-Z, wherein Z signifies a leaving group and X signifies heteroaryl,

g) if desired, separating a diastereomeric mixture obtained and/or resolving a racemate obtained, and/or h) if desired, converting a compound of formula I obtained into a pharmaceutically acceptable salt.

**14.** A medicament, especially for the treatment or prevention of ischemia, hypoglycaemia, hypoxia, cerebral vasospasms, spasticity, trauma, hemorrhage, infections (viral, bacterial, amoebic, prional), epileptic seizures, autoimmune disorders, withdrawal symptoms, Alzheimer's disease, Parkinson's disease, amyotrophic lateral sclerosis, Huntington's chorea, intoxications, Olivoponto-cerebellar atrophy, spinal injury, schizophrenia, depressions, anxiety states, dependence, pains, autism and mental retardation, containing a compound according to any one claims 1-9 or rac-cis-4b-(2-amino-ethyl)-4b,5,6,7,8,8a,9,10-octahydro-phenanthren-8a-ol or a pharmaceutically acceptable acid addition salt thereof and a therapeutically inert carrier material.

**15.** The use of compound according to any one of claims 1-9 as well as of rac-cis-4b-(2-amino-ethyl)-4b,5,6,7,8,8a, 9, 10-octahydro-phenanthren-8a-ol or of pharmaceutically usable acid addition salts thereof for the manufacture of neuroprotective medicaments for the treatment or prevention of ischemia, hypoglycaemia, hypoxia, cerebral vasospasms, spasticity, trauma, hemorrhage, infections (viral, bacterial, amoebic, prional), epileptic seizures, autoimmune disorders, withdrawal symptoms, Alzheimer's disease, Parkinson's disease, amyotrophic lateral sclerosis, Huntington's chorea, intoxications, Olivoponto-cerebellar atrophy, spinal injury, schizophrenia, depressions, anxiety states, dependence, pains, autism and mental retardation.

**Revendications**

**1.** Composés de formule générale

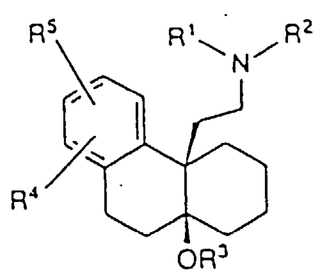

I

dans laquelle

$R^1$ et $R^2$, indépendamment l'un de l'autre, sont chacun un hydrogène ou un groupe alkyle en $C_1$-$C_7$, éventuellement substitué par des groupes aryle ou cycloalkyle en $C_{3-6}$ ;
$R^4$ et $R^5$ sont tous les deux des hydrogènes ou tous les deux des halogènes, ou encore l'un est un hydrogène et l'autre est un halogène ou un groupe hydroxy, alcoxy en $C_1$-$C_7$, aryloxy ou amino, et
$R^3$ est un hydrogène, ou encore, en l'absence de groupe(s) primaire(s) ou secondaire(s), un groupe alcanoyle,

ainsi que les sels utilisables d'un point de vue pharmaceutique de composés de formule I avec des acides ;

à l'exception du rac-cis-4b-(2-amino-éthyl)-4b,5,6,7,8, 8a,9,10-octahydro-phénanthrène-8a-ol.

**2.** Composés selon la revendication 1, dans lesquels $R^1$ et $R^2$, indépendamment l'un de l'autre, sont chacun un hydrogène ou un groupe alkyle en $C_1$-$C_7$ éventuellement substitué par des groupes cycloalkyle en $C_{3-6}$, $R^3$ est un hydrogène ou un groupe alcanoyle, et $R^4$ et $R^5$ sont chacun un halogène, ou encore l'un est un hydrogène et l'autre est un halogène, ou un groupe hétéroaryloxy, alcoxy en $C_1$-$C_7$ ou hydroxy.

**3.** Composés selon la revendication 1, dans lesquels $R^1$ et $R^2$ sont des groupes alkyle en $C_1$-$C_7$, $R^3$ est un hydrogène, et $R^4$ et $R^5$ sont chacun un hydrogène, ou l'un est un hydrogène et l'autre un halogène ou le groupe hydroxy.

**4.** Composés selon la revendication 1, à savoir le rac-cis-4b-(2-diméthylamino-éthyl)-4b,5,6,7,8,8a,9,10-octahydro-phénanthrène-8a-ol.

**5.** Composés selon la revendication 1, à savoir le rac-cis-3-chloro-4b-(2-diméthylamino-éthyl)-4b,5,6,7,8, 8a,9,10-octahydro-phénanthrène-8a-ol.

**6.** Composés selon la revendication 1, à savoir le (-)-cis-3-chloro-4b-(2-diméthylamino-éthyl)-4b,5,6,7,8, 8a,9,10-octahydro-phénanthrène-8a-ol.

**7.** Composés selon la revendication 1, à savoir le (+)-cis-3-chloro-4b-(2-diméthylamino-éthyl)-4b,5,6,7,8, 8a,9,10-octahydro-phénanthrène-8a-ol.

**8.** Composés selon la revendication 1, à savoir le rac-cis-4b-(2-diméthylamino-éthyl)-4b,5,6,7,8,8a,9,10-octahydro-phénanthrène-3,8a-ol.

**9.** Composés selon la revendication 1, à savoir les composés suivants :

rac-cis-4b-(2-amino-éthyl)-3-fluoro-4b,5,6,7,8,8a, 9,10-octahydro-phénanthrène-8a-ol ;
rac-cis-4b-(2-amino-éthyl)-3-chloro-4b,5,6,7,8,8a, 9,10-octahydro-phénanthrène-8a-ol ;
rac-cis-4b-(2-amino-éthyl)-3-bromo-4b,5,6,7,8,8a,9, 10-octahydro-phénanthrène-8a-ol;
rac-cis-4b-(2-amino-éthyl)-4b,5,6,7,8,8a,9,10-octahydro-phénanthrène-3,8a-ol ;
(+)-cis-4b-(2-amino-éthyl)-4b,6,7,8,8a,9,10-octahydro-phénanthrène-3,8a-diol ;
(-)-cis-4b-(2-amino-éthyl)-4b,6,7,8,8a,9,10-octahydro-phénanthrène-3,8a-diol ;
rac-cis-4b-(2-diméthylamino-éthyl)-3-fluoro-4b,5,6, 7,8,8a,9,10-octahydro-phénanthrène-8a-ol ;
rac-cis-4b-(2-diméthylamino-éthyl)-3-méthoxy-4b,5, 6,7,8,8a,9,10-octahydro-phénanthrène-8a-ol;
acétate de rac-cis-4b-(2-diméthylamino-éthyl)-3-fluoro-4b,5,6,7,8,8a,9,10-octahydrophénanthrène-8a-yle ;
acétate de rac-cis-3-chloro-4b-(2-diméthylaminoéthyl)-4b,5,6,7,8,8a,9,10-octahydrophénanthrène-8a-yle ;
rac-cis-4b-(2-amino-éthyl)-3-fluoro-1-fluoro-4b,5, 6,7,8,8a,9,10-octahydro-phénanthrène-8a-ol.

**10.** Composés de formules générales

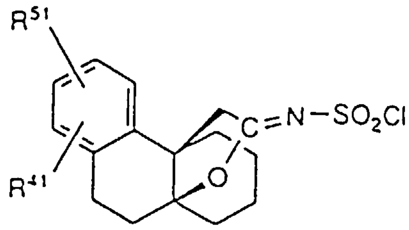 II 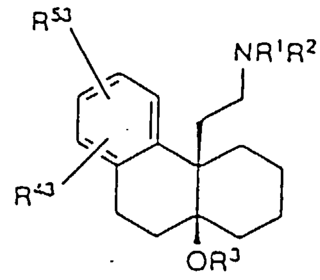 III

et

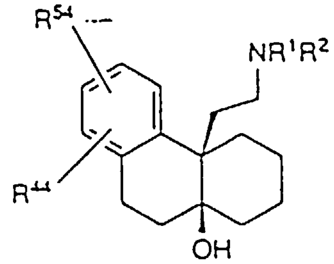 IV

où $R^1$, $R^2$ et $R^3$ ont les significations données dans la revendication 1, $R^{41}$ et $R^{51}$ sont tous les deux des halogènes, ou l'un est un hydrogène et l'autre est un halogène ou un groupe alcoxy en $C_1$-$C_7$ ou aryloxy, l'un des radicaux $R^{43}$ et $R^{53}$ est un hydrogène et l'autre est un groupe arylméthoxy, et l'un des radicaux $R^{44}$ et $R^{54}$ est un hydrogène et l'autre est un groupe arylméthylamino, ainsi que les analogues de composés de formule II, mais où l'un des radicaux $R^{41}$ et $R^{51}$ est un hydrogène et l'autre est un groupe arylméthoxy ou arylcarbonylamino.

**11.** Composés selon l'une des revendications 1 à 9, et rac-cis-4b-(2-amino-éthyl)-4b,5,6,7,8,8a,9,10-octahydro-phénanthrène-8a-ol, ainsi que leurs sels d'addition avec un acide, acceptables d'un point de vue pharmaceutique, pour utilisation en tant que principes actifs thérapeutiques.

**12.** Composés selon l'une des revendications 1 à 9, et rac-cis-4b-(2-amino-éthyl)-4b,5,6,7,8,8a,9,10-octahydro-phénanthrène-8a-ol, ainsi que leurs sels d'addition avec un acide, acceptables d'un point de vue pharmaceutique, pour utilisation en tant que principes actifs neuroprotecteurs pour le traitement ou la prophylaxie de l'ischémie, de l'hypoglycémie, de l'hypoxie, des spasmes des vaisseaux cérébraux, de la spasticité, des traumatismes, de l'hémorragie, des infections (virales, bactériennes, amibiennes, prionales), des accès épileptiques, des maladies auto-immunes, des symptômes de sevrage, de la maladie d'Alzheimer, de la maladie de Parkinson, de la sclérose latérale amyotrophique, de la maladie de Huntington, des empoisonnements, de l'atrophie olivoponto-cérébelleuse, des lésions de la moelle épinière, de la schizophrénie, des dépressions, des états d'angoisse, de la dépendance, des douleurs, de l'autisme et du retard mental.

**13.** Procédé pour préparer des composés selon l'une des revendications 1 à 9, caractérisé en ce que

a) pour préparer un composé de formule I dans laquelle $R^1$, $R^2$ et $R^3$ sont chacun des hydrogènes, et $R^4$ et $R^5$ sont tous les deux des hydrogènes, ou l'un est un hydrogène et l'autre est un halogène ou un groupe alcoxy en $C_1$-$C_7$ ou aryloxy, on réduit un composé de formule générale

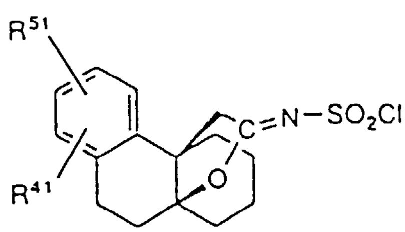 II

dans laquelle $R^{41}$ et $R^{51}$ sont tous les deux des hydrogènes, ou encore l'un est un hydrogène et l'autre est

un halogène ou un groupe alcoxy en $C_1$-$C_7$ ou aryloxy, ou bien
b) pour préparer un composé de formule I dans laquelle l'un des radicaux $R^1$ et $R^2$ est un hydrogène et l'autre est un groupe alkyle en $C_1$-$C_7$ éventuellement substitué par des groupes aryle ou cycloalkyle en $C_{3-6}$, ou bien $R^1$ et $R^2$ sont chacun un groupe alkyle en $C_1$-$C_7$ éventuellement substitué par des substituants aryle ou cycloalkyle en $C_{3-6}$, $R^3$ est un hydrogène, et $R^4$ et $R^5$ sont tous les deux des hydrogènes ou des halogènes, ou l'un est un hydrogène et l'autre est un halogène ou un groupe alkyle en $C_1$-$C_7$ ou aryloxy, on procède à une mono- ou à une disubstitution appropriée, sur le groupe amino primaire, d'un composé de formule générale

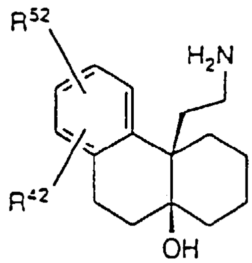

Ia

dans laquelle $R^{42}$ et $R^{52}$ sont tous les deux des hydrogènes ou des halogènes, ou encore l'un est un hydrogène et l'autre est un halogène ou un groupe alcoxy en $C_1$-$C_7$ ou aryloxy ; ou bien
c) pour préparer un composé de formule I dans laquelle $R^1$ et $R^2$ sont des groupes alkyle en $C_1$-$C_7$ éventuellement substitués par des groupes aryle ou cycloalkyle en $C_{3-6}$, $R^3$ est un groupe alcanoyle et $R^4$ et $R^5$ sont tous les deux des hydrogènes ou des halogènes, ou encore l'un est un hydrogène et l'autre est un halogène ou un groupe alcoxy en $C_1$-$C_7$ ou aryloxy, on traite avec un composé qui cède un groupe alcanoyle un composé de formule générale

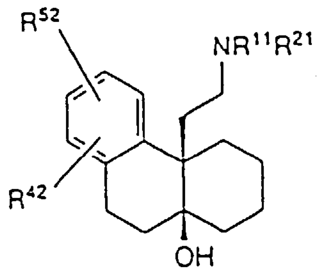

Ib

dans laquelle $R^{11}$ et $R^{21}$ sont des groupes alkyle en $C_1$-$C_7$ éventuellement substitués par des groupes aryle ou cycloalkyle en $C_{3-6}$, et $R^{42}$ et $R^{52}$ ont les significations données ci-dessus ; ou bien
d) pour préparer un composé de formule I dans laquelle l'un des radicaux $R^4$ et $R^5$ est un hydrogène et l'autre est un groupe hydroxy, on hydrogène en présence d'un catalyseur un composé de formule générale

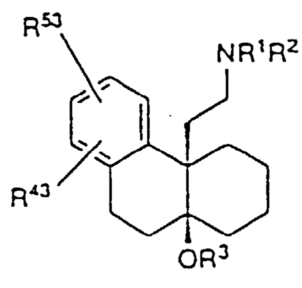

III

dans laquelle $R^1$, $R^2$ et $R^3$ ont les significations données dans la revendication 1, et l'un des radicaux $R^{43}$ et $R^{53}$ est un hydrogène, et l'autre est un groupe arylméthoxy ; ou bien
e) pour préparer un composé de formule I dans laquelle $R^1$ et $R^2$ ont les significations données dans la revendication 1, $R^3$ est un hydrogène et l'un des radicaux $R^4$ et $R^5$ est un hydrogène et l'autre est le groupe amino, on hydrogène en présence d'un catalyseur un composé de formule générale

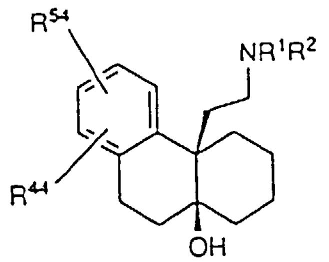

IV

dans laquelle $R^1$ et $R^2$ ont les significations données dans la revendication 1, et l'un des radicaux $R^{44}$ et $R^{54}$ est un hydrogène, et l'autre est un groupe arylméthylamino ; ou bien

f) pour préparer un composé de formule I dans laquelle $R^1$, $R^2$ et $R^3$ ont les significations données dans la revendication 1, et l'un des radicaux $R^4$ et $R^5$ est un hydrogène et l'autre est un groupe hétéroaryloxy, on traite avec un composé de formule X-Z dans laquelle Z est un groupe éliminable et X est un groupe hétéroaryle, un composé de formule générale

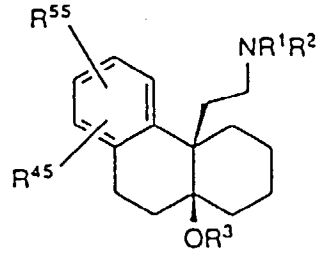

Ic

dans laquelle $R^1$, $R^2$ et $R^3$ sont les significations données dans la revendication 1, et l'un des radicaux $R^{45}$ et $R^{55}$ est un hydrogène et l'autre est un groupe hydroxy,

g) si on le souhaite, on sépare le mélange de diastéréoisomères et/ou le racémate obtenus, et/ou

h) si on le souhaite, on convertit un composé de formule I tel qu'obtenu en un sel acceptable d'un point de vue pharmaceutique.

**14.** Médicament, en particulier pour le traitement ou la prophylaxie de l'ischémie, de l'hypoglycémie, de l'hypoxie, des spasmes des vaisseaux cérébraux, de la spasticité, des traumatismes, de l'hémorragie, des infections (virales, bactériennes, amibiennes, prionales), des accès épileptiques, des maladies auto-immunes, des symptômes de sevrage, de la maladie d'Alzheimer, de la maladie de Parkinson, de la sclérose latérale amyotrophique, de la maladie de Huntington, des empoisonnements, de l'atrophie olivoponto-cérébelleuse, des lésions de la moelle épinière, de la schizophrénie, des dépressions, des états d'angoisse, de la dépendance, des douleurs, de l'autisme et du retard mental, contenant un composé selon l'une des revendications 1 à 9 ou le rac-cis-4b-(2-aminoéthyl)-4b,5,6,7,8,8a,9,10-octahydro-phénanthrène-8a-ol, ou un de leurs sels d'addition avec un acide, acceptables d'un point de vue pharmaceutique, et un matériau support inerte d'un point de vue thérapeutique.

**15.** Utilisation de composés selon l'une des revendications 1 à 9, ainsi que de rac-cis-4b-(2-aminoéthyl)-4b,5,6,7,8,8a,9,10-octahydro-phénanthrène-8a-ol ou de leurs sels d'addition avec un acide, utilisables d'un point de vue pharmaceutique, pour la préparation de produits neuroprotecteurs pour le traitement ou la prophylaxie de l'ischémie, de l'hypoglycémie, de l'hypoxie, des spasmes des vaisseaux cérébraux, de la spasticité, des traumatismes, de l'hémorragie, des infections (virales, bactériennes, amibiennes, prionales), des accès épileptiques, des maladies auto-immunes, des symptômes de sevrage, de la maladie d'Alzheimer, de la maladie de Parkinson, de la sclérose latérale amyotrophique, de la maladie de Huntington, des empoisonnements, de l'atrophie olivoponto-cérébelleuse, des lésions de la moelle épinière, de la schizophrénie, des dépressions, des états d'angoisse, de la dépendance, des douleurs, de l'autisme et du retard mental.